# EUROPEAN PATENT SPECIFICATION

(11) **EP 1 254 176 B1**
(45) Date of publication and mention of the grant of the patent: **09.01.2008**
(21) Application number: 01909036.4
(22) Date of filing: 09.02.2001
(51) Int. Cl.: C07K 14/715, C12N 15/12, C12N 15/62, C07K 16/28, A61P 35/00, A61P 29/00

(54) **Use of the extracellular domain of TRADE molecules in medicaments for treating neoplasia**
Verwendung der extrazellulären Domäne von TRADE Molekülen in Medikamenten zur Behandlung von Neoplasie
Utilisation du domaine extracellulaire des molecules TRADE dans des médicaments pour le traitement du néoplasme

(30) Priority: 11.02.2000 US 181922 P; 14.02.2000 US 182148 P
(43) Date of publication of application: 06.11.2002
(73) Proprietor: Genetics Institute, LLC, Cambridge, MA 02140 (US)
(72) Inventor: WOOD, Clive, Boston, MA 02114 (US); CHAUDHARY, Divya, Andover, MA 01810 (US); LONG, Andrew, Chelmsford, MA 01824 (US)
(74) Representative: Dörries, Hans Ulrich
(86) International application number: PCT/US2001/004238
(87) International publication number: WO 2001/058954

(56) References cited:
- WO-A-98/30693
- WO-A-98/38304
- WO-A-98/41629
- WO-A-98/51793
- WO-A-99/11791
- WO-A-99/13078
- US-A- 5 447 851
- KOJIMA T ET AL: "TROY, a newly identified member of the tumor necrosis factor receptor superfamily, exhibits a homology with Edar and is expressed in embryonic skin and hair follicles" JOURNAL OF BIOLOGICAL CHEMISTRY, AMERICAN SOCIETY OF BIOLOGICAL CHEMISTS, BALTIMORE, MD, US, vol. 275, no. 27, 7 July 2000 (2000-07-07), pages 20742-20747, XP002163651 ISSN: 0021-9258
- CHAUDHARY DIVYA ET AL: "TRADE, a novel TNF receptor superfamily member, induces apoptosis and activates NFkappaB and Jnk." SCANDINAVIAN JOURNAL OF IMMUNOLOGY, vol. 51, no. Supplement 1, June 2000 (2000-06), page 33 XP001019629 8th International TNF Congress, Conference on Tumor Necrosis Factor and Related Molecules Scientific Advances and Medical Applications;Trondheim, Norway; May 14-18, 2000 ISSN: 0300-9475

## Description

### RELATED APPLICATIONS

This application claims priority to USSN 60/181,922, filed on February 11, 2000, and USSN 60/182,148 , filed on February 14, 2000.

### BACKGROUND OF THE INVENTION

The tumor necrosis factor receptor (TNF-R) family members play key roles in the regulation of cell survival and death decisions (Baker and Reddy, 1998, Oncogene 17:3261-3270). In particular, they have been widely studied for their roles in lymphocyte activation, inflammation and apoptosis (Gravestein and Borst, 1998, Seminars in Immunology 10:423-434). All known members of the TNF-R family have at least two copies of a characteristic cysteine-rich domain in their extracellular region. This pattern of cysteine residues has facilitated the discovery, using bioinformatics, of new family members in expressed sequence tag libraries. At least nineteen (possibly twenty two) members of the family have now been reported (WO 99/11791, WO 99/13078).

For the most part, trimeric forms of the TNF ligand family induce the oligomerization of TNF receptor family members (Natoli et al., 1998, Biochemical Pharmacology, 56:915-920), leading to juxtaposition of the intracellular signaling domains of the receptors and activation of the downstream signal. This downstream signal can lead to the activation of transcription factors NFkB and AP1, through signaling mediators that activate the IkB kinases and JNK kinase, respectively (Wallach et al., 1999, Ann Rev of Immunology, 17:331-367). Several of these receptors contain a well characterized death domain in their intracellular region, which interacts with adaptor molecules that lead to the caspase activation cascade resulting in programmed cell death (Orlinick and Chao, 1998, Cellular Signalling, 10:543-551). Some of the receptors function exclusively in either NFkB activation, *e.g.* TNF-RII (Ng et al., 1998, Biochem Biophys Res Comm, 244:756-762), or apoptosis induction *e.g.* DR4 (Muzio, 1998, Int Jof Clinical Laboratory Res, 28:141-147), while some can signal both NFkB activation and apoptosis *e.g.* DR3 (Chinnaiyan et al., 1996, Science, 274:990-992). Signaling programmed cell death is a significant role of the TNF receptor family, such that some members have been defined to exclusively function in this role with well defined decoy receptor members as regulators (Ashkenazi and Dixit, 1999, Curr Opinion Cell Biol,11:255-260). A common structural feature amongst these members is the well conserved death domain in the intracellular region (Bantel et al., 1998; Bothwell, 1996, European Cytokine Network, 9:681-684). Decoy receptors are speculated to compete for ligand binding with specific death domain containing receptors, and function as a decoy as they contain either non-functional partial death domain, *e.g.* DcR2 (Marsters et al, 1997, Current Biology, 7:1003-1006), or no death domain, *e.g.* DcR3 (Pitti et al., 1998, Nature, 396:669-703). Besides type I transmembrane proteins, this family includes a soluble secreted protein, *e.*g. OPG (Emery et al., 1998, J of Biol Chem, 273:14363-14367), and a gpi-linked protein, DcR1 (Degli-Esposti, 1999, J of Leukocyte Biology, 65:535-542).

### SUMMARY OF THE INVENTION

The present invention is based, at least in part, on the discovery that novel TRADE molecules are useful as modulating agents in regulating a variety of cellular processes, including modulation of cell proliferation (e.g., by either inducing proliferation or apoptosis) by signaling via the NFkB and JNK signaling pathways, particularly in epithelial cells.

Accordingly, in one aspect, the present invention provides the use of an agent in the manufacture of a medicament for treating neoplasia, wherein the agent is a soluble form of a TRADE polypeptide comprising the extra-cellular domain of a protein according to SEQ ID NO:2 or SEQ ID NO:4.

Also described is a method for modulating cell proliferation comprising contacting a cell with an agent that modulates the activity of a TRADEα polypeptide or a TRADEβ polypeptide, such that cell proliferation is modulated.

In one embodiment, the neoplasia is neoplasia of a cell selected from the group consisting of: an epithelial cell, a ductal epithelial cell, or a bronchial epithelial cell. In another embodiment, the cell is a carcinoma or an adenocarcinoma In another embodiment, the cell is selected from the group consisting of: a lung cell, a liver cell, a brain cell, and a prostate cell.

In yet another embodiment, the agent is a soluble form of a TRADE polypeptide comprising a TRADE polypeptide extracellular domain. In another embodiment, the soluble form of the TRADE polypeptide is a TRADE-Fc fusion protein. In one embodiment, the agent consists essentially of a TRADE polypeptide extracellular domain.

In one embodiment, the agent is a nucleic acid molecule that modulates expression of a TRADEα polypeptide or a TRADEβ polypeptide. In one embodiment, the agent is a nucleic acid molecule encoding a TRADEα polypeptide or TRADEβ polypeptide or portion thereof. In another embodiment, the agent is a nucleic acid molecule which is antisense to a nucleic acid molecule encoding a TRADEα polypeptide or TRADEβ polypeptide or portion thereof. In another embodiment, the agent is an antibody that recognizes a TRADE family member polypeptide. In still another embodiment, the activity is selected from the group consisting of: activation of a JNK signaling pathway, activation of an NFkB signaling pathway, and activation of apoptosis.

Also described herein is a method of modulating the proliferation of a cell comprising contacting a prostate, liver, or lung cell with an agent that modulates the activity of a polypeptide selected from the group consisting of: a TRADEα polypeptide, a TRAIN polypeptide, a αOAF065 polypeptide, and a TRADEβ polypeptide.

Furthermore described herein is a method of modulating the proliferation of a cell comprising contacting the cell with an agent that modulates the expression of a TRADE family member polypeptide, wherein the cell is selected from the group consisting of an epithelial cell, a ductal epithelial cell, a carcinoma cell, and an adenocarcinoma cell, such that the proliferation of the cell is modulated.

Also described is a method of modulating the proliferation of a cell comprising contacting the cell with an agent that modulates the activity of a TRADE family member polypeptide, wherein the cell is selected from the group consisting of: an epithelial cell, a ductal epithelial cell, a carcinoma cell, and an adenocarcinoma cell such that the proliferation of the cell is modulated.

In one embodiment, the TRADE family polypeptide is selected from the group consisting of: TRADEα, TRADEβ, Apo4, TRAIN, αOAF065, and βOAF065. In one embodiment, the agent is a soluble form of a TRADE family polypeptide comprising a TRADE extracellular domain. In one embodiment, the soluble form of a TRADE family polypeptide is a TRADE-Fc fusion protein. In another embodiment, the agent consists essentially of a TRADE family extracellular domain. In one embodiment, the agent is a nucleic acid molecule that modulates expression of a TRADE family polypeptide. In one embodiment, the agent is a nucleic acid molecule encoding a TRADE family polypeptide or portion thereof. In one embodiment, the agent is a nucleic acid molecule which is antisense to a nucleic acid molecule encoding a TRADE family polypeptide or portion thereof. In one embodiment, the agent is an antibody that recognizes a TRADE family polypeptide.

In one embodiment, the activity is selected from the group of activities consisting of: activation of a JNK signaling pathway, activation of an NFkB signaling pathway, and activation of apoptosis.

Also described herein is a method for modulating the proliferation of a cell comprising contacting the cell with an agent that modulates the expression of a TRADE family member polypeptide, wherein the cell is selected from the group consisting of: a brain cell, a liver cell, a prostate cell, an intestinal cell, or a lung cell, such that the proliferation of the cell is modulated.

Also described herein is a method of modulating the proliferation of a cell comprising contacting the cell with an agent that modulates the activity of a TRADE family member polypeptide, wherein the cell is selected from the group consisting of: a brain cell, a liver cell, a prostate cell, an intestinal cell, or a lung cell, such that the proliferation of the cell is modulated

In one embodiment, the TRADE family member polypeptide is selected from the group consisting of: a TRADEα polypeptide, a TRAIN polypeptide, a αOAF065 polypeptide, and a TRADEβ polypeptide.

Also described is a method for treating a subject having a disorder that would benefit from modulation of expression of a TRADEα polypeptide or TRADEβ polypeptide comprising administering to the subject an agent that modulates expression of TRADEα polypeptide or TRADEβ polypeptide such that a disorder that treatment occurs.

Also described is a method for treating a subject having a disorder that would benefit from modulation of activity of a TRADEα polypeptide or TRADEβ polypeptide comprising administering to the subject an agent that modulates activity of TRADEα polypeptide or TRADEβ polypeptide such that treatment occurs.

In one embodiment, the disorder is a proliferative disease or disorder selected from the group consisting of: inflammation and neoplasia. In one embodiment, the neoplasia is a carcinoma. In one embodiment, the neoplasia is present in lung or prostate tissue. In one embodiment, the neoplasia is an adenocarcinoma

Also described is a method for treating a subject having a carcinoma or an adenocarcinoma comprising administering to the subject an agent that modulates activity of a TRADE family polypeptide such that the carcinoma or an adenocarcinoma is treated.

Also described is a method for treating a subject having a carcinoma or an adenocarcinoma comprising administering to the subject an agent that modulates expression of a TRADE family polypeptide such that a carcinoma or an adenocarcinoma is treated.

Furthermore described is a method for treating a subject having a carcinoma or an adenocarcinoma of a tissue selected from the group consisting of: lung, liver, brain, and intestine, comprising administering to the subject an agent that modulates activity of a TRADE family polypeptide such that the carcinoma or an adenocarcinoma is treated.

Also described is a method of detecting a TRADE associated disorder comprising: obtaining a biological sample from a subject and testing for the presence of a TRADE polypeptide in the sample in order to detect a TRADE associated disorder, wherein the sample comprises a cell type selected from the group consisting of: lung cells, liver cells, brain cells, or intestinal cells.

### BRIEF DESCRIPTION OF THE DRAWINGS

*Figure 1* depicts an amino acid sequence comparison between the two human TRADE proteins of the invention (α and β) and the related TNF receptor family proteins TRAIN and Apo4.
*Figure 2* depicts a comparison of the C-termini of human TRADE α and TRADE β
*Figure 3* depicts an alignment of the two full cysteine-rich domains of human TRADE α and β with those of related proteins human p75^{NGFR}, human OX40, and human CD40.
*Figure 4* depicts a sequence comparison between the human TRADEα amino acid sequence and the murine TRADE amino acid sequence of the invention.
*Figure 5* depicts an immunochemical analysis of TRADE using flow cytometry.
*Figure 6* depicts the results of transfection experiments which indicate that ectopic expression of human TRADEα can activate the NFκB and JNK signal pathways.
*Figure 7* depicts apoptosis induced by expression of TRADE.
*Figure 8* depicts a SDS-PAGE analysis of soluble TRADE-Fc protein which was purified from the conditioned media of transfected human COS cells under both reducing and non-reducing conditions.
*Figure 9* depicts a schematic diagram of the deletion constructs used in the TRADE biochemical analysis.
*Figure 10* depicts kinase activity associated with TRADEα and TRADEβ.
*Figure 11A* demonstrates deletion analysis of TRADE and the effects on kinase activity. *Figure 11B* is a Western blot of the immunoprecipitates used in Figure 11A showing equivalent expression of all constructs.
*Figures 12A and B* depict the binding of TRAF6, but not TRAF2, to TRADEα and TRADEβ.
*Figure* 13 depicts the binding of TRAF3 to TRADEα and TRADEβ.
*Figures 14A and 14B* show deletion analysis of TRADEα and TRADEβ and the effect NFkB activity.

### DETAILED DESCRIPTION OF THE INVENTION

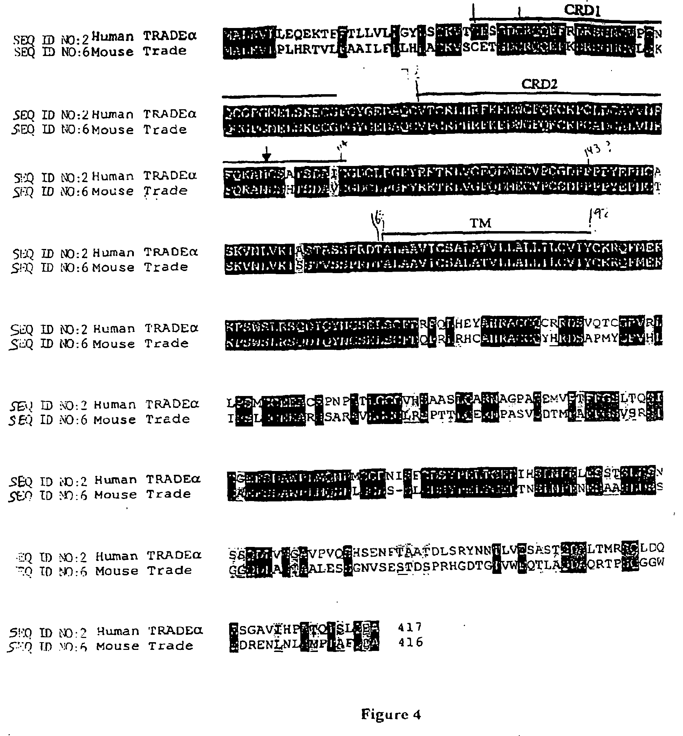

The present invention is based, at least in part, on the finding that TRADE molecules, are useful as modulating agents in regulating a variety of cellular processes, including proliferation (e.g., by modulating growth or apoptosis) and that these molecules signal via the NFKβ and JNK signaling pathways, particularly in epithelial cells. The invention also identifies novel TRADE molecules, TRADEα (shown in SEQ ID Nos: 1 and 2) and TRADEβ (shown in SEQ ID Nos:3 and 4). Mouse TRADE is shown in SEQ IDNos: 5 and 6. The invention places TRADEα and TRADEβ in a family of related TRADE molecules and identifies novel uses for all the members of the family. The invention further identifies certain TRADE domains, e.g., an intracellular domain comprising amino acid residues corresponding to residues 193-417 of SEQ ID NO:2 or 193-423 of SEQ ID NO:4, an extracellular domain comprising amino acid residues corresponding to residues 1-168 of SEQ ID NO:2 or 4, a transmembrane domain comprising amino acid residues corresponding to residues 169-192 of SEQ ID NO:2 or 4, a first cysteine-rich domain comprising amino acid residues corresponding to residues 29-63 of SEQ ID NO:2 or 4, a second cysteine-rich domain comprising amino acid residues corresponding to residues 72-114 of SEQ ID NO:2 or 4, a third cysteine-rich domain comprising amino acid residues corresponding to residues 114-139 of SEQ ID NO:2 or 4, a serine/threonine/proline-rich domain comprising amino acid residues corresponding to residues 137-168 of SEQ ID NO:2 or 4, and a TRADE-related death effector domain comprising amino acid residues corresponding to residues 218-417 of SEQ ID NO:2 or 218-423 of SEQ ID NO:4. TRADE α and β comprise an N-glycosylation site at residues corresponding to residues 105-108 of SEQ ID NO:2 or 4, a cAMP/cGMP-dependent protein kinase phosphorylation site at residues corresponding to residues 200 to 203 of SEQ ID NO: 2 or 4, a cAMP/cGMP-dependent protein kinase phosphorylation site at residues corresponding to residues 238 to 241 of SEQ ID NO: 2 or 4, a protein kinase C phosphorylation site at residues corresponding to residues 205 to 207 of SEQ ID NO: 2 or 4, a casein kinase II phosphorylation site at residues corresponding to residues 219 to 222 of SEQ ID NO: 2 or 4, and at residues corresponding to residues 325 to 328 of SEQ ID NO:2 or 4, a tyrosine kinase phosphorylation site at residues corresponding to residues 207-213 of SEQ ID NOs:2 or 4, and an N-myristoylation site at residues corresponding to residues 215-220 of SEQ ID NO:2 or 4. Furthermore, members of the TRADE family of proteins can be recognized by the absence of a TNF receptor death domain consensus sequence in the intracellular portion of the TRADE peptide. However, despite the lack of a TNF receptor death domain consensus sequence, TRADE peptides are able to induce apoptosis through the activity of a death effector domain, as demonstrated in the appended examples. Moreover, the invention identifies TRADE consensus domains which are conserved among human and mouse TRADE orthologs, as illustrated in Figure 4.

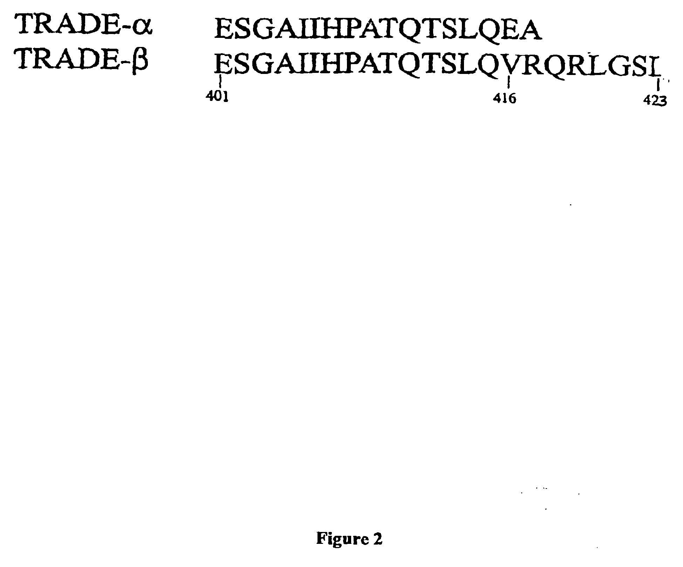

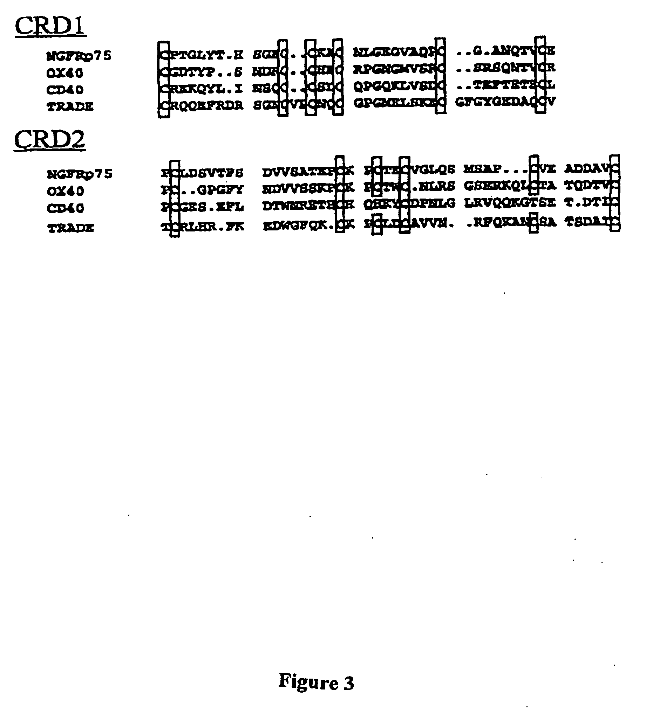

The nucleotide sequence of the TRADEα and TRADEβ cDNAs are identical at the 5' end, but diverge close to the region encoding the final C-terminal amino acids of the molecules. Both TRADEα and TRADEβ have identical putative N-terminal signal sequences of 25 amino acids, mature extracellular region of 143 amino acids and a single transmembrane domain. The extracellular region contains two domains homologous to the cysteine-rich domains of the TNF-R family (see Figure 3). The second domain is followed by a cysteine rich region that may be an incomplete match to the consensus cysteine rich domain. Such an incomplete match is found in some other family members such as TNFRI (Wyllie, 1997, Eur J Cell Biol, 73:189-197) and HVEM (Harrop et al, 1998, J Biol Chem, 273:27548-27556). Additionally, there is a serine/threonine/proline-rich stretch in the extracellular juxtamembrane region, as found in some other family members such as 4-1BB and CD27 (Gravestein et al, 1993, Eur J Immunol, 23:943-950). The intracellular region of TRADEα consists of 234 amino acids, with no apparent homologies (including the lack of a death domain, Kitson et al, 1996, Nature, 384:372-375), and a component that is included in other family members, (*e.g*. TNF-RI). The intracellular region of TRADEβ shares this sequence with TRADEα, but differs from TRADEα by 2 amino acids and comprises 6 additional amino acids at its C-terminus (see Figure 2).

Northern analysis described in more detail in the appended examples, has shown human TRADEα and TRADEβ expression in various tissues and organs with the highest levels in adult prostate, lung, ovary, and fetal lung and liver. More importantly, immunohistochemistry was used to demonstrate TRADEα and TRADEβ are primarily localized in the prostate, parotid gland and testis to ductal epithelial tissues. Expression of TRADE in adenocarcinomas has also been detected.

These and other aspects of the invention are described in further detail in the following subsections:

### I. Definitions

As used herein the term "TRADE" refers to **T**NF **R**eceptor family member **A**ssociated with **DE**ath protein. Two novel TRADE molecules are described herein. The nucleotide sequence of the "TRADEα" molecule is set forth in SEQ ID NO:1 and the amino acid sequence of TRADEα is set forth in SEQ ID NO:2. The nucleotide sequence of "TRADEβ" is set forth in SEQ ID NO:3 and the amino acid sequence of TRADEβ is set forth in SEQ ID NO:4. The nucleotide sequence of the TRADEα and TRADEβ cDNAs are identical at the 5' end, but diverge close to the region encoding the final C-terminal amino acids of the molecules, with TRADEβ having a longer cytoplasmic domain. As used herein, the term "TRADE", unless specifically used to refer to the TRADEα or TRADEβ molecule (or their specific SEQ ID NO), will be understood to refer to a TRADE family polypeptide as defined below.

"TRADE family polypeptide" is intended to include proteins or nucleic acid molecules having a TRADE structural domain or motif and having sufficient amino acid or nucleotide sequence identity with a TRADEα or TRADEβ molecule as defined herein. Such family members can be naturally or non-naturally occurring and can be from the same or different species. For example, a family can contain a first protein of human origin, as well as other, distinct proteins of human origin or, alternatively, can contain homologues of non-human origin. Preferred members of a family may also have common functional characteristics. Exemplary TRADE family molecules include: TRADEα and TRADEβ (described herein), Apo4 (WO99/11791), TRAIN (WO99/13078), AX92_3 (WO98/01554, WO99/20644), αOAF065 and βOAF065 (WO98/38304).

Preferred TRADE polypeptides comprise one or more of the following TRADE domains: an intracellular domain (*e.g.* comprising residues 193-417 of SEQ ID NO:2 or 193-423 of SEQ ID NO:4), an extracellular domain (*e.g.* comprising residues 1-168 of SEQ ID NO:2 or 4), a transmembrane domain (*e.g.* comprising residues 169-192 of SEQ ID NO:2 or 4), a first cysteine-rich domain (*e.g.,* comprising residues 29-63 of SEQ ID NO:2 or 4), a second cysteine-rich domain (*e.g.,* comprising residues 72-114 of SEQ ID NO:2 or 4), a third, partial cysteine-rich domain (*e.g.,* comprising residues 114-139 of SEQ ID NO:2 or 4), a serine/threonine/proline-rich domain (*e.g.,* comprising residues 137-168 of SEQ ID NO:2 or 4), a TRADE-related death effector domain (*e.g.,* comprising residues 218-417 of SEQ ID NO:2 or 218-42.3 of SEQ ID NO:4), an N-glycosylation site (*e.g.,* comprising residues 105-108 of SEQ ID NO:2 or 4), a cAMP/cGMP-dependent protein kinase phosphorylation site (*e.g*., comprising residues 200 to 203 of SEQ ID NO:2 or 4), a cAMP/cGMP-dependent protein kinase phosphorylation site (*e.g.,* comprising residues 238 to 241 of SEQ ID NO: 2 or 4), at least one protein kinase C phosphorylation site (*e.g.*, comprising residues 205 to 207 of SEQ ID NO:2 or 4), a first casein kinase II phosphorylation site (*e.g*., comprising residues 219 to 222 of SEQ ID NO:2 or 4), a second casein kinase II phosphorylation site (*e.g.,* comprising residues 325 to 328 of SEQ ID NO:2 or 4), a tyrosine kinase phosphorylation site (*e.g.*, comprising residues 207-213 of SEQ ID NO:2 or 4), an N-myristoylation site (*e.g.*, comprising residues 215-220 of SEQ ID NO:2 or 4), a TRAF binding domain (*e.g*. comprising residues 1-328 of SEQ ID NO:2 or 4; preferably comprising residues 218-328), a kinase associating domain (*e.g.* comprising residues 1-368 of SEQ ID NO:2 or 4; preferably comprising residues 328-368), or an NFkB activation signaling domain (e.g. comprising residues comprising residues 1-368 of SEQ ID NO:2 or 4; preferably in the intracellular domain of TRADE). TRADE molecules also lack a TNF receptor death domain consensus sequence in the intracellular portion of the TRADE peptide. The TNF receptor death domain consensus sequence, as defined for HMM searches, is illustrated by the consensus sequence listed under the PFAM Accession Number PF00531 (http://pfam.wustl.edu).

As used herein, the term "TRADE activity" or "activity of a TRADE polypeptide" includes the ability to modulate cell proliferation (e.g., by enhancing proliferation or apoptosis), and/or the ability to modulate an NFkB signaling pathway, and/or the ability to modulate a JNK signaling pathway in a cell, such as an epithelial cell. As used herein, the term "modulate" includes alteration, e.g., by increasing or decreasing the particular parameter being described, e.g., TRADE activity. As described in the appended Examples, when TRADE is overexpressed, it results in activation of the NFkB and JNK pathways. Activation of these pathways is associated with cellular proliferative responses. The examples also demonstrate that overexpression of TRADE can result in cell death signaling, leading to apoptosis. In one embodiment, a TRADE activity is a direct activity, such as an association with a TRADE-target molecule or binding partner. As used herein, a "target molecule" or "binding partner" is a molecule with which a TRADE protein binds or interacts in nature, such that TRADE-mediated function is achieved.

As used herein the term "apoptosis" includes programmed cell death which can be characterized using techniques which are known in the art. Apoptotic cell death can be characterized, e.g., by cell shrinkage, membrane blebbing and chromatin condensation culminating in cell fragmentation. Cells undergoing apoptosis also display a characteristic pattern of internucleosomal DNA cleavage. As used herein, the term "modulating apoptosis" includes modulating programmed cell death in a cell, such as a epithelial cell. As used herein, the term "modulates apoptosis" includes either up regulation or down regulation of apoptosis in a cell. Modulation of apoptosis is discussed in more detail below and can be useful in ameliorating various disorders, e.g., neurological disorders.

As used herein, the term "NFkB signaling pathway" refers to any one of the signaling pathways known in the art which involve activation or deactivation of the transcription factor NFkB, and which are at least partially mediated by the NFkB factor (Karin, 1998, Cancer J from Scientific American, 4:92-99; Wallach et al, 1999, Ann Rev of Immunology, 17:331-367). Generally, such NFkB signaling pathway are responsive to a number of extracellular influences *e.g.* mitogens, cytokines, stress, and the like. The NFkB signaling pathways involve a range of cellular processes, including, but not limited to, modulation of apoptosis. These signaling pathways often comprise, but are by no means limited to, mechanisms which involve the activation or deactivation via phosphorylation state of an inhibitor peptide of NFkB (IkB), thus indirectly activating or deactivating NFkB.

As used herein, the term "JNK signaling pathway" refers to any one of the signaling pathways known in the art which involve the Jun amino terminal kinase (JNK) (Karin, 1998, Cancer J from Scientific American, 4:92-99; Wallach et al, 1999, Ann Rev of Immunology, 17:331-367). This kinase is generally responsive to a number of extracellular signals *e.g.* mitogens, cytokines, stress, and the like. The JNK signaling pathways mediate a range of cellular processes, including, but not limited to, modulation of apoptosis. In a preferred embodiment, these signaling pathways comprise mechanisms which involve the JNK-mediated modulation of the activity of transcription factor c-Jun via its phosphorylation state. In a further embodiment, JNK activation occurs through the activity of one or more members of the TRAF protein family (**T**NF **R**eceptor **A**ssociated **F**actor; see, *e.g*., Wajant et al, 1999, Cytokine Growth Factor Rev 10:15-26).

The "TRAF" family includes a family of cytoplasmic adapter proteins that mediate signal transduction from many members of the TNF-receptor superfamily and the interleukin-1 receptor (see *e.g.*, Arch, R.H. et al., 1998, Genes Dev. 12:2821-2830). To date, there are six distinct TRAF molecules in mammalian species (termed TRAF1 through TRAF6). The carboxy-terminal region of these proteins is required for self-association and interaction with receptors. The domain contains a predicted coiled-coil region that is followed by a highly conserved TRAF-C domain. TRAF1, TRAF2, and TRAF3 share this conserved C-terminal TRAF domain. TRAF proteins also share a number of additional predicted structural features such as an amino-terminal RING finger domain and a stretch of predicted zinc fingers located downstream of the RING finger domain. These proteins have been shown to promote cell survival or initiate programmed cell death (see *e.g.* Arch, R.H. et al., 1998, Genes Dev. 12:2821-2830.)

An "isolated" nucleic acid molecule is one which is separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid molecule is free of sequences which naturally flank the nucleic acid molecule (i.e., sequences located at the 5' and 3' ends of the nucleic acid molecule) in the genomic DNA of the organism from which the nucleic acid molecule is derived. For example, in various embodiments, the isolated TRADE nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized. An "isolated" TRADE nucleic acid molecule may, however, be linked to other nucleotide sequences that do not normally flank the TRADE sequences in genomic DNA (e.g., the TRADE nucleotide sequences may be linked to vector sequences). In certain preferred embodiments, an "isolated" nucleic acid molecule, such as a cDNA molecule, also may be free of other cellular material. However, it is not necessary for the TRADE nucleic acid molecule to be free of other cellular material to be considered "isolated" (e.g., a TRADE DNA molecule separated from other mammalian DNA and inserted into a bacterial cell would still be considered to be "isolated").

As used herein, an "isolated protein" or "isolated polypeptide" refers to a protein or polypeptide that is substantially free of other proteins, polypeptides, cellular material and culture medium when isolated from cells or produced by recombinant DNA techniques, or chemical precursors or other chemicals when chemically synthesized. An "isolated" or "purified" protein or biologically active portion thereof is substantially free of cellular material or other contaminating proteins from the cell or tissue source from which the TRADE protein is derived, or substantially free from chemical precursors or other chemicals when chemically synthesized. The language "substantially free of cellular material" includes preparations of TRADE protein in which the protein is separated from cellular components of the cells from which it is isolated or recombinantly produced. In one embodiment, the language "substantially free of cellular material" includes preparations of TRADE protein having less than about 30% (by dry weight) of non- TRADE protein (also referred to herein as a "contaminating protein"), more preferably less than about 20% of non- TRADE protein, still more preferably less than about 10% of non- TRADE protein, and most preferably less than about 5% non- TRADE protein. When the TRADE protein or biologically active portion thereof is recombinantly produced, it is also preferably substantially free of culture medium, i.e., culture medium represents less than about 20%, more preferably less than about 10%, and most preferably less than about 5% of the volume of the protein preparation.

The language "substantially free of chemical precursors or other chemicals" includes preparations of TRADE protein in which the protein is separated from chemical precursors or other chemicals which are involved in the synthesis of the protein. In one embodiment, the language "substantially free of chemical precursors or other chemicals" includes preparations of TRADE protein having less than about 30% (by dry weight) of chemical precursors or non- TRADE chemicals, more preferably less than about 20% chemical precursors or non- TRADE chemicals, still more preferably less than about 10% chemical precursors or non- TRADE chemicals, and most preferably less than about 5% chemical precursors or non- TRADE chemicals.

As used herein the term "epithelial cell" includes all cells which are part of the epithelium, the covering of internal and external surfaces of the body, including the lining of vessels and other small cavities. Cells of the epithelium are generally joined together tightly with a cementing substance and can be classified based on the shape of the superficial layers and number of layers deep. All classifications of such cells of the epithelium are understood to fall under the term epithelial cells, as used herein. Furthermore, the term epithelial cell, as used herein, may be further understood to encompass cells of the epithelium from any organ or tissue of the body. In a preferred embodiment, the term "epithelial cell" refers to the cells of the epithelium of the lung, the prostate, or of the parotid gland. In a preferred embodiment, the term "epithelial" includes the ductal epithelial cells of the prostate.

As used herein, the term "neoplasia" refers to a proliferative disease or disorder resulting from uncontrolled or abberant cell division. The term neoplasia includes malignant and non-malignant disorders. As used herein, the term "adenocarcinoma" refers generally to cancers of glandular epithelial cells and "carcinoma" refers to malignant epithelial tumors.

As used herein, the term "modulate TRADE activity or expression " includes up regulation and down regulation of a TRADE activity or TRADE expression (e.g., at the level of transcription or translation) in a cell.

The term "interact" as used herein is meant to include detectable interactions between molecules, such as can be detected using, for example, a yeast two hybrid assay. The term interact is also meant to include "binding" interactions between molecules. Interactions may be protein-protein or protein-nucleic acid in nature.

As used herein, a "naturally-occurring" nucleic acid molecule refers to an RNA or DNA molecule having a nucleotide sequence that occurs in nature (*e.g.,* encodes a natural protein).

As used herein, an "antisense" nucleic acid comprises a nucleotide sequence which is complementary to a "sense" nucleic acid encoding a protein, *e.g.,* complementary to the coding strand of a double-stranded cDNA molecule, complementary to an mRNA sequence or complementary to the coding strand of a gene. Accordingly, an antisense nucleic acid can hydrogen bond to a sense nucleic acid.

As used herein, the term "coding region" refers to regions of a nucleotide sequence comprising codons which are translated into amino acid residues, whereas the term "noncoding region" refers to regions of a nucleotide sequence that are not translated into amino acids (*e.g*., 5' and 3' untranslated regions).

As used herein, the term "vector" refers to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" or simply "expression vectors". In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g.*, replication defective retroviruses, adenoviruses and adeno-associated viruses), which serve equivalent functions.

As used herein, the term "host cell" is intended to refer to a cell into which a nucleic acid molecule of the invention, such as a recombinant expression vector of the invention, has been introduced. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. Preferably a host cell is a mammalian cell, e.g., a human cell. In particularly preferred embodiments, it is a epithelial cell.

As used herein, "heterologous DNA" or "heterologous nucleic acid" includes DNA that does not occur naturally as part of the genome in which it is present or which is found in a location or locations in the genome that differs from that in which it occurs in nature or which is operatively linked to DNA to which it is not normally linked in nature (i.e., a gene that has been operatively linked to a heterologous promoter). Heterologous DNA is not naturally occurring in that position or is not endogenous to the cell into which it is introduced, but has been obtained from another cell. Heterologous DNA can be from the same species or from a different species. In one embodiment, it is mammalian, e.g., human. DNA that one of skill in the art would recognize or consider as heterologous or foreign to the cell in which is expressed is herein encompassed by the term heterologous DNA.

The terms "heterologous protein", "recombinant protein", and "exogenous protein" are used interchangeably throughout the specification and refer to a polypeptide which is produced by recombinant DNA techniques, wherein generally, DNA encoding the polypeptide is inserted into a suitable expression vector which is in turn used to transform a host cell to produce the heterologous protein. That is, the polypeptide is expressed from a heterologous nucleic acid.

As used herein, a "transgenic animal" refers to a non-human animal, preferably a mammal, more preferably a mouse, in which one or more of the cells of the animal includes a "transgene". The term "transgene" refers to exogenous DNA which is integrated into the genome of a cell from which a transgenic animal develops and which remains in the genome of the mature animal, for example directing the expression of an encoded gene product in one or more cell types or tissues of the transgenic animal.

As used herein, a "homologous recombinant animal" refers to a type of transgenic non-human animal, preferably a mammal, more preferably a mouse, in which an endogenous gene has been altered by homologous recombination between the endogenous gene and an exogenous DNA molecule introduced into a cell of the animal, *e.g.*, an embryonic cell of the animal, prior to development of the animal.

As used herein, the term "antibody" is intended to include immunoglobulin molecules and immunologically active portions of immunoglobulin molecules, *i.e.,* molecules that contain an antigen binding site which binds (immunoreacts with) an antigen, such as Fab and F(ab')₂ fragments, single chain antibodies, intracellular antibodies, scFv, Fd, or other fragments. Preferably, antibodies of the invention bind specifically or substantially specifically to TRADE molecules (i.e., have little to no cross reactivity with non-TRADE molecules). The terms "monoclonal antibodies" and "monoclonal antibody composition", as used herein, refer to a population of antibody molecules that contain only one species of an antigen binding site capable of immunoreacting with a particular epitope of an antigen, whereas the term "polyclonal antibodies" and "polyclonal antibody composition" refer to a population of antibody molecules that contain multiple species of antigen binding sites capable of interacting with a particular antigen. A monoclonal antibody compositions thus typically display a single binding affinity for a particular antigen with which it immunoreacts.

As used herein, the term "disorders that would benefit from the modulation of TRADE activity or expression" or "TRADE associated disorder" includes disorders in which TRADE activity is aberrant or in which a non-TRADE activity that would benefit from modulation of a TRADE activity is aberrant. Preferably, TRADE associated disorders involve aberrant proliferation of cells, e.g., excessive or unwanted proliferation of cells or deficient proliferation of cells. In one embodiment, TRADE associated disorders include such as neoplasia or inflammation. Examples of TRADE associated disorders include: disorders involving aberrant or unwanted proliferation of cells, e.g., inflammation, neoplasia, apoptosis, or necrosis. Preferably, the cells undergoing unwanted proliferation in a TRADE-associated disorder are epithelial cells, *e.g.*, of the lung, liver, brain, intestine, or prostate. Further examples of TRADE associated disorders include carcinomas, adenocarcinomas, and other neoplasias. TRADE-associated disorders may also include disorders that have been linked generally to aberrant TNF receptor activity or function, including Crohn's Disease (Baert and Rutgeerts, 1999, Int J Colorectal Dis, 14:47-51) and certain cardiovascular diseases (Ferrari, 1999, Pharmacol Res, 40:97-105). They may also include disorders characterized by uncontrolled or aberrant levels of apoptosis, for example myelokathexis (Aprikyan et al., 2000, Blood, 95:320-327), and autoimmune lymphoproliferative syndrome (Jackson and Puck, 1999, Curr Op Pediatr, 11:521-527; Straus et al., 1999, Ann Intern Med, 130:591-601).

### II. Methods of Use

The nucleic acid molecules, proteins, protein homologues, and antibodies described herein can be used in one or more of the following methods: a) methods of modulating proliferation of a cell, b) methods of treating disorders, e.g., up- or down-modulating proliferation in a subject; b) screening assays; c) predictive medicine (e.g., diagnostic assays, prognostic assays, or monitoring clinical trials). In one embodiment, the subject can be preselected based on the fact that they have a TRADE associated disorder.

The methods of the invention can be practiced e.g., using agents that modulate the expression and/or activity of a TRADE polypeptide. Such agents include, nucleic acid molecules, used, for example, to express TRADE proteins (e.g., via a recombinant expression vector in a host cell in gene therapy applications), to detect TRADE mRNA (e.g., in a biological sample) or a genetic alteration in a TRADE gene, and to modulate TRADE activity, as described further below. Agents also include TRADE proteins, used, e.g., to treat disorders characterized by insufficient or excessive production of TRADE inhibitors. In addition, the TRADE proteins can be used to screen for naturally occurring TRADE binding proteins, to screen for drugs or compounds which modulate TRADE activity, as well as to treat disorders that would benefit from modulation of TRADE, e.g., characterized by insufficient or excessive production of TRADE protein or production of TRADE protein forms which have decreased or aberrant activity compared to TRADE wild type protein. Moreover, anti-TRADE antibodies can be used to detect and isolate TRADE proteins, regulate the bioavailability of TRADE proteins, and modulate TRADE activity e.g., modulate proliferation. In preferred embodiments the methods of the invention, e.g., detection, modulation of TRADE, etc. are performed in epithelial cells. In one embodiment the epithelial cells are ductal epithelial cells. In another embodiment, the epithelial cells are derived from a tissue in which trade is expressed. Preferably, the tissue is selected from the group consisting of: the liver, the brain, the prostate, the lung, or the intestine. In one embodiment, the detection method is performed to determine whether a neoplastic condition exists, e.g., a carcinoma or an adenocarcinoma. In one embodiment of the invention, the subject methods are used (e.g., to modulate or detect) a TRADE family member molecule. In another embodiment, such methods are specific for one or more members of the TRADE family, but do not act on all members of the TRADE family. For example, in a preferred embodiment, modulation of the expression or activity of a TRADE family polypeptide does not modulate one or more of : Apo4, TRAIN, AX92_3, αOAF065, or βOAF065. For example, in one embodiment modulation of a TRADEα or TRADEβ polypeptide does not modulate one or more of: Apo4, TRAIN, AX92_3, αOAF065, or βOAF065.

### A. Methods of Modulating TRADE

The present invention provides for methods of modulating TRADE activity, e.g., in a cell or *in vitro* for the purpose of identifying agents that modulate TRADE expression and/or activity, as well as both prophylactic and therapeutic methods of treating a subject at risk of (or susceptible to) a disorder or having a disorder associated with aberrant TRADE expression or activity or a disorder that would benefit from modulation of TRADE expression and/or activity.

Yet another aspect of the invention pertains to methods of modulating TRADE expression and/or activity in a cell. The modulatory methods of the invention involve contacting the cell with an agent that modulates TRADE expression and/or activity such that TRADE expression and/or activity in the cell is modulated. The agent may act by modulating the activity of TRADE protein in the cell or by modulating transcription of the TRADE gene or translation of the TRADE mRNA.

Accordingly, in one embodiment, the agent inhibits TRADE activity. An inhibitory agent may function, for example, by directly inhibiting TRADE pro-proliferation or pro-apoptotic activity or by modulating a signaling pathway which regulates TRADE activity. In another embodiment, the agent stimulates TRADE activity. A stimulatory agent may function, for example, by directly stimulating TRADE pro-proliferation or pro-apoptotic activity, or by modulating a signaling pathway that regulates TRADE activity.

Exemplary inhibitory agents include antisense TRADE nucleic acid molecules (*e.g*., to inhibit translation of TRADE mRNA), intracellular anti- TRADE antibodies (*e.g.,* to inhibit the activity of TRADE protein), and dominant negative mutants of the TRADE protein. Other inhibitory agents that can be used to inhibit the activity of a TRADE protein are chemical compounds that inhibit TRADE - pro-proliferation or pro-apoptotic activity. Such compounds can be identified using screening assays that select for such compounds, as described herein. Additionally or alternatively, compounds that inhibit TRADE pro- proliferation or pro-apoptotic activity can be designed using approaches known in the art.

According to another modulatory method for the invention, TRADE activity is stimulated in a cell by contacting the cell with a stimulatory agent. Examples of such stimulatory agents include active TRADE protein and nucleic acid molecules encoding TRADE that are introduced into the cell to increase TRADE activity in the cell. A preferred stimulatory agent is a nucleic acid molecule encoding a TRADE protein, wherein the nucleic acid molecule is introduced into the cell in a form suitable for expression of the active TRADE protein in the cell. To express a TRADE protein in a cell, typically a TRADE cDNA is first introduced into a recombinant expression vector using standard molecular biology techniques, as described herein. A TRADE cDNA can be obtained, for example, by amplification using the polymerase chain reaction (PCR) or by screening an appropriate cDNA library as described herein. Following isolation or amplification of TRADE cDNA, the DNA fragment is introduced into an expression vector and transfected into target cells by standard methods, as described herein. Other stimulatory agents that can be used to stimulate the activity and/or expression of a TRADE protein are chemical compounds that stimulate TRADE activity and/or expression in cells, such as compounds that enhance TRADE pro-apoptotic activity. Such compounds can be identified using screening assays that select for such compounds, as described in detail herein.

The modulatory methods of the invention can be performed *in vitro* (*e.g.,* by culturing the cell with the agent or by introducing the agent into cells in culture) or, alternatively, *in vivo* (*e.g.,* by administering the agent to a subject or by introducing the agent into cells of a subject, such as by gene therapy). For practicing the modulatory method *in vitro,* cells can be obtained from a subject by standard methods and incubated (*i.e.,* cultured) *in vitro* with a modulatory agent of the invention to modulate TRADE activity in the cells.

For stimulatory or inhibitory agents that comprise nucleic acids (including recombinant expression vectors encoding TRADE protein, antisense RNA, intracellular antibodies or dominant negative inhibitors), the agents can be introduced into cells of the subject using methods known in the art for introducing nucleic acid (*e.g*., DNA) into cells *in vivo.* Examples of such methods encompass both non-viral and viral methods, including:
*Direct Injection*: Naked DNA can be introduced into cells *in vivo* by directly injecting the DNA into the cells (see *e.g.*, Acsadi et al., 1991, Nature 332:815-818; Wolff et al., 1990, Science 247:1465-1468). For example, a delivery apparatus (*e.g.,* a "gene gun") for injecting DNA into cells *in vivo* can be used. Such an apparatus is commercially available (*e.g.*, from BioRad).
*Cationic Lipids*: Naked DNA can be introduced into cells *in vivo* by complexing the DNA with cationic lipids or encapsulating the DNA in cationic liposomes. Examples of suitable cationic lipid formulations include N-[-1-(2,3-dioleoyloxy)propyl]N,N,N-triethylammonium chloride (DOTMA) and a 1:1 molar ratio of 1,2-dimyristyloxy-propyl-3-dimethylhydroxyethylammonium bromide (DMRIE) and dioleoyl phosphatidylethanolamine (DOPE) (see *e.g.*, Logan, J.J. et al., 1995, Gene Therapy 2:38-49; San, H. et al., 1993, Human Gene Therapy 4:781-788).
*Receptor-Mediated DNA Uptake*: Naked DNA can also be introduced into cells *in vivo* by complexing the DNA to a cation, such as poly-lysine, which is coupled to a ligand for a cell-surface receptor (see for example Wu, G. and Wu, C.H., 1988, J. Biol. Chem. 263:14621; Wilson et al., 1992, J. Biol. Chem. 267:963-967; and U.S. Patent No. 5,166,320). Binding of the DNA-ligand complex to the receptor facilitates uptake of the DNA by receptor-mediated endocytosis. A DNA-ligand complex linked to adenovirus capsids which naturally disrupt endosomes, thereby releasing material into the cytoplasm can be used to avoid degradation of the complex by intracellular lysosomes (see for example Curiel et al., 1991, Proc. Natl. Acad. Sci. USA 88:8850; Cristiano et al., 1993, Proc. Natl. Acad. Sci. USA 90:2122-2126).
*Retroviruses*: Defective retroviruses are well characterized for use in gene transfer for gene therapy purposes (for a review see Miller, A.D., 1990, *Blood* 76:271). A recombinant retrovirus can be constructed having a nucleotide sequences of interest incorporated into the retroviral genome. Additionally, portions of the retroviral genome can be removed to render the retrovirus replication defective. The replication defective retrovirus is then packaged into virions which can be used to infect a target cell through the use of a helper virus by standard techniques. Protocols for producing recombinant retroviruses and for infecting cells *in vitro* or *in vivo* with such viruses can be found in Current Protocols in Molecular Biology, Ausubel, F.M. et al. (eds.) Greene Publishing Associates, 1989, Sections 9.10-9.14 and other standard laboratory manuals. Examples of suitable retroviruses include pLJ, pZIP, pWE and pEM which are well known to those skilled in the art. Examples of suitable packaging virus lines include ψCrip, ψCre, ψ2 and ψAm. Retroviruses have been used to introduce a variety of genes into many different cell types, including epithelial cells, endothelial cells, lymphocytes, myoblasts, hepatocytes, bone marrow cells, *in vitro* and/or *in vivo* (see for example Eglitis, et al., 1985, Science 230:1395-1398; Danos and Mulligan, 1988, Proc. Natl. Acad. Sci. USA 85:6460-6464; Wilson et al., 1988, Proc. Natl. Acad Sci. USA 85:3014-3018; Armentano et al., 1990, Proc. Natl. Acad. Sci. USA 87:6141-6145; Huber et al., 1991, Proc. Natl. Acad Sci. USA 88:8039-8043; Ferry et al., 1991, Proc. Natl. Acad. Sci. USA 88:8377-8381; Chowdhury et al., 1991, Science 254:1802-1805; van Beusechem et al., 1992, Proc. Natl. Acad. Sci. USA 89:7640-7644; Kay et al., 1992, Human Gene Therapy 3:641-647; Dai et al., 1992, Proc. Natl. Acad Sci. USA 89:10892-10895; Hwu et al., 1993, J. Immunol. 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573). Retroviral vectors require target cell division in order for the retroviral genome (and foreign nucleic acid inserted into it) to be integrated into the host genome to stably introduce nucleic acid into the cell. Thus, it may be necessary to stimulate replication of the target cell.
*Adenoviruses*: The genome of an adenovirus can be manipulated such that it encodes and expresses a gene product of interest but is inactivated in terms of its ability to replicate in a normal lytic viral life cycle. See for example Berkner et al., 1988, BioTechniques 6:616; Rosenfeld et al., 1991, Science 252:431-434; and Rosenfeld et al., 1992, Cell 68:143-155. Suitable adenoviral vectors derived from the adenovirus strain Ad type 5 d1324 or other strains of adenovirus (*e.g.*, Ad2, Ad3, Ad7 *etc.*) are well known to those skilled in the art. Recombinant adenoviruses are advantageous in that they do not require dividing cells to be effective gene delivery vehicles and can be used to infect a wide variety of cell types, including airway epithelium (Rosenfeld *et al.,* 1992, cited *supra*), endothelial cells (Lemarchand et al., 1992, Proc. Natl. Acad Sci. USA 89:6482-6486), hepatocytes (Herz and Gerard, 1993, Proc. Natl. Acad. Sci. USA 90:2812-2816) and muscle cells (Quantin et al., 1992, Proc. Natl. Acad Sci. USA 89:2581-2584). Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (*e.g.*, retroviral DNA). Moreover, the carrying capacity of the adenoviral genome for foreign DNA is large (up to 8 kilobases) relative to other gene delivery vectors (Berkner *et al.* cited *supra;* Haj-Ahmand and Graham, 1986, J. Virol. 57:267). Most replication-defective adenoviral vectors currently in use are deleted for all or parts of the viral E1 and E3 genes but retain as much as 80 % of the adenoviral genetic material.
*Adeno-Associated Viruses*: Adeno-associated virus (AAV) is a naturally occurring defective virus that requires another virus, such as an adenovirus or a herpes virus, as a helper virus for efficient replication and a productive life cycle. (For a review see Muzyczka et al. Curr. Topics in Micro. and Immunol., 1992, 158:97-129). It is also one of the few viruses that may integrate its DNA into non-dividing cells, and exhibits a high frequency of stable integration (see for example Flotte et al., 1992, Am. J. Respir. Cell. Mol. Biol. 7:349-356; Samulski et al., 1989, J. Virol. 63:3822-3828; and McLaughlin et al., 1989, J. Virol. 62:1963-1973). Vectors containing as little as 300 base pairs of AAV can be packaged and can integrate. Space for exogenous DNA is limited to about 4.5 kb. An AAV vector such as that described in Tratschin et al., 1985, Mol. Cell. Biol. 5:3251-3260 can be used to introduce DNA into cells. A variety of nucleic acids have been introduced into different cell types using AAV vectors (see for example Hermonat et al., 1984, Proc. Natl. Acad Sci. USA 81:6466-6470; Tratschin et al., 1985, Mol. Cell. Biol. 4:2072-2081; Wondisford et al., 1988, Mol. Endocrinol. 2:32-39; Tratschin et al., 1984, J. Virol. 51:611-619; and Flotte et al., 1993, J. Biol. Chem. 268:3781-3790).

The efficacy of a particular expression vector system and method for introducing nucleic acid into a cell can be assessed by standard approaches routinely used in the art. For example, DNA introduced into a cell can be detected by a filter hybridization technique (*e.g.*, Southern blotting) and RNA produced by transcription of introduced DNA can be detected, for example, by Northern blotting, RNase protection or reverse transcriptase-polymerase chain reaction (RT-PCR). The gene product can be detected by an appropriate assay, for example by immunological detection of a produced protein, such as with a specific antibody, or by a functional assay to detect a functional activity of the gene product.

There are a wide variety of pathological conditions for which TRADE modulating agents of the present invention can be used in treatment. In one embodiment, such agents can down-modulate proliferation or up-modulate apoptosis in a cell. In a further embodiment this method can be used to treat a subject suffering from a disorder which would benefit from the up-modidation of apoptosis. In a preferred embodiment, TRADE is modulated to enhance apoptosis of a epithelial cell, such as to promote the apoptosis in cancer cells, e.g., in the lung, liver, brain, intestine or prostate.

In another embodiment, TRADE is modulated to up-modulate proliferation or down-modulate apoptosis in a cell, for example, in the promotion of epithelial cell survival in Alzheimer's or amyotrophic lateral sclerosis (ALS) patients (Lee, M., 1999, J. of Neuropath & Exper. Neurology 58:459; Desjardins, P. and Ledoux, S, 1998, Neurosci. Letters. 244:69; Yoshihisa et al., 1998, Brain Research. 780:260). TRADE molecules are expressed in the brain. Other exemplary disorders for which modulation of TRADE can be used in treatment include other nervous system disorders. The term disorder is meant to include both normal conditions that would benefit from an alteration in TRADE activity and/or expression and various disease states.

### 1. Prophylactic Methods

In one aspect, the invention provides a method for preventing in a subject, a disease or condition that would benefit from modulation of TRADE activity and/or expression, e.g., a disorder associated with an aberrant TRADE expression or activity, by administering to the subject a TRADE polypeptide or an agent which modulates TRADE polypeptide expression or at least one TRADE activity. Subjects at risk for a disease which is caused or contributed to by aberrant TRADE expression or activity can be identified by, for example, any or a combination of diagnostic or prognostic assays as described herein. Administration of a prophylactic agent can occur prior to the manifestation of symptoms characteristic of TRADE aberrance, such that a disease or disorder is prevented or, alternatively, delayed in its progression. Depending on the type of TRADE aberrance or condition, for example, a TRADE polypeptide, TRADE agonist or TRADE antagonist agent can be used for treating the subject. The appropriate agent can be determined based on screening assays described herein.

### 2. Therapeutic Methods

Another aspect of the invention pertains to methods of modulating TRADE expression or activity for therapeutic purposes. Accordingly, in an exemplary embodiment, the modulatory method for the invention involves contacting a cell with a TRADE polypeptide or agent that modulates one or more of the activities of TRADE protein associated with the cell. An agent that modulates TRADE protein activity can be an agent as described herein, such as a nucleic acid or a protein, a naturally-occurring target molecule of a TRADE protein (e.g., a TRADE binding protein), a TRADE antibody, a TRADE agonist or antagonist, a peptidomimetic of a TRADE agonist or antagonist, or other small molecule. In one embodiment, the agent stimulates one or more TRADE activities. Examples of such stimulatory agents include active TRADE protein and a nucleic acid molecules encoding TRADE polypeptide that has been introduced into the cell. In another embodiment, the agent inhibits one or more TRADE activities. Examples of such inhibitory agents include, e.g., antisense TRADE nucleic acid molecules, anti-TRADE antibodies, and TRADE inhibitors. These modulatory methods can be performed *in vitro* (e.g., by culturing the cell with the agent) or, alternatively, *in vivo* (e.g., by administering the agent to a subject). As such, the present invention provides methods of treating an individual afflicted with a disease or disorder that would benefit from modulation of a TRADE protein, e.g., a disorder which would benefit from up- or down-modulation of the immune response, or which is characterized by aberrant expression or activity of a TRADE protein or nucleic acid molecule. In one embodiment, the method involves administering an agent (e.g., an agent identified by a screening assay described herein), or combination of agents that modulates (e.g., upregulates or downregulates) TRADE expression or activity. In another embodiment, the method involves administering a TRADE protein or nucleic acid molecule as therapy to compensate for reduced or aberrant TRADE expression or activity.

Stimulation of TRADE activity is desirable in situations in which TRADE is abnormally downregulated and/or in which increased TRADE activity is likely to have a beneficial effect, e.g., when it is desirable to increase proliferation or increase apoptosis in a cell. Likewise, inhibition of TRADE activity is desirable in situations in which TRADE is abnormally upregulated and/or in which decreased TRADE activity is likely to have a beneficial effect, e.g., when it is desirable to decrease proliferation or decrease apoptosis in a cell. Exemplary situations in which TRADE modulation will be desirable are in the treatment of TRADE-associated disorders, including disorders involving aberrant or unwanted proliferation of cells, e.g., inflammation or cancer. Preferably, the cells undergoing unwanted proliferation are epithelial cells, *e.g.*, of the lung or prostate. Further examples of TRADE associated disorders include carcinomas, adenocarcinomas, and other neoplasias. TRADE disorders may also include disorders that have been linked generally to aberrant TNF receptor activity or function, including Crohn's Disease (Baert and Rutgeerts, 1999, Int J Colorectal Dis, 14:47-51) and certain cardiovascular diseases (Ferrari, 1999, Pharmacol Res, 40:97-105). They may also include disorders characterized by uncontrolled or aberrant levels of apoptosis, for example myelokathexis (Aprikyan et al., 2000, Blood, 95:320-327), and autoimmune lymphoproliferative syndrome (Jackson and Puck, 1999, Curr Op Pediatr, 11:521-527; Straus et al., 1999, Ann Intern Med, 130:591-601).

Like other members of the TNF receptor family, modulation of TRADE molecules can have different downstream consequences depending upon other factors. TRADE is a novel orphan receptor that has the potential to generate a mitogenic signal or an apoptotic signal, depending upon the required physiological context. The dual capacity to induce activation and apoptosis is a common property of ligands of the TNF receptor superfamily (Pimentel-Muinos and Seed, 1999, Immunity, 11:783). For example, CD95 aggregation triggers cell death (Itoh et al., 1991, Cell 66:233), but also proliferation and NF-kB activation (Alderson et al., 1993, J. Exp. Med 178:2231; Smith et al., 1993, Cell. 73:1349). CD40 also mediates both apoptosis as well as B cell differentiation and survival. (Banchereau et al., 1991 Science, 251:70; Hess and Engelmann, 1996, J. Exp. Med. 183:159). In one embodiment, the presence of an external agent can be used to influence the outcome of modulation of a TNF receptor superfamily receptor (Mackay et al., 1996, J. Biol. Chem., 272:24934). One of ordinary skill in the art will be able to determine what the consequences of TRADE modulation in a particular situation will be in a cell specific context by assaying the effect of TRADE up or down-modulation on the cell type in question. Such assays can be performed without undue experimentation using methods known in the art, such as those exemplified herein.

### III. TRADE Modulating Agents

### A. Isolated Nucleic Acid Molecules Encoding TRADE Or Portions Thereof

In practicing the methods of the invention, various agents can be used to modulate the activity and/or expression of TRADE in a cell. In one embodiment, an agent is a nucleic acid molecule encoding a TRADE polypeptide or a portion thereof. Such nucleic acid molecules are described in more detail below.

Analysis of the TRADE polypeptide has identified a region of the protein which mediates the interaction of TRADE with a polypeptide in the JNK or NFkB signaling pathway, *e.g.*, via amino acids in the intracellular domain of the TRADE polypeptide, *e.g.* a TRAF-interacting domain. Accordingly, in one aspect, the invention pertains to nucleic acid molecules that encode a portion of a TRADE polypeptide that interacts with a TRADE binding partner, *e.g.* a TRAF protein. TRAF proteins have at least one domain which may interact directly with TRADE peptides. As a family, TRAF proteins are defined by several distinct structural features, including a signature C-terminal TRAF domain of approximately 230 amino acids (Rothe et al., 1994, Cell, 78:681-692) which is involved in a variety of protein-protein interactions. This C-terminal TRAF domain can be further divided into the TRAF-N and TRAF-C subdomains (Cheng et al., 1995, Science, 267:1494-1498). It is known that some TRAF molecules can form homo- and heterodimers in order to interact with members of the TNF superfamily of receptors. The C-terminal TRAF domain is sufficient for both self association and receptor interaction (Rothe, 1995, Science, 269:1424-1427; Takeuchi et al., 1996, JBC, 271:19935-19942).

There is a known and definite correspondence between the amino acid sequence of a particular protein and the nucleotide sequences that can code for the protein, as defined by the genetic code (shown below). Likewise, there is a known and definite correspondence between the nucleotide sequence of a particular nucleic acid molecule and the amino acid sequence encoded by that nucleic acid molecule, as defined by the genetic code.

| GENETIC CODE | |
|---|---|
| Alanine (Ala, A) | GCA, GCC, GCG, GCT |
| Arginine (Arg, R) | AGA, ACG, CGA, CGC, CGG, CGT |
| Asparagine (Asn, N) | AAC, AAT |
| Aspartic acid (Asp,D) | GAC, GAT |
| Cysteine (Cys, C) | TGC, TGT |
| Glutamic acid (Glu,E) | GAA, GAG |
| Glutamine (Gln, Q) | CAA, CAG |
| Glycine (Gly, G) | GGA, GGC, GGG, GGT |
| Histidine (His, H) | CAC, CAT |
| Isoleucine (Ile, I) | ATA, ATC, ATT |
| Leucine (Leu, L) | CTA, CTC, CTG, CTT, TTA, TTG |
| Lysine (Lys, K) | AAA, AAG |
| Methionine (Met, M) | ATG |
| Phenylalanine (Phe, F) | TTC, TTT |
| Proline (Pro, P) | CCA, CCC, CCG, CCT |
| Serine (Ser, S) | AGC, AGT, TCA, TCC, TCG, TCT |
| Threonine (Thr, T) | ACA, ACC, ACG, ACT |
| Tryptophan (Trp, W) | TGG |
| Tyrosine (Tyr, Y) | TAC, TAT |
| Valine (Val, V) | GTA, GTC, GTG, GTT |
| Termination signal (end) | TAA, TAG, TGA |

An important and well known feature of the genetic code is its redundancy, whereby, for most of the amino acids used to make proteins, more than one coding nucleotide triplet may be employed (illustrated above). Therefore, a number of different nucleotide sequences may code for a given amino acid sequence. Such nucleotide sequences are considered functionally equivalent since they result in the production of the same amino acid sequence in all organisms (although certain organisms may translate some sequences more efficiently than they do others). Moreover, occasionally, a methylated variant of a purine or pyrimidine may be found in a given nucleotide sequence. Such methylations do not affect the coding relationship between the trinucleotide codon and the corresponding amino acid.

In view of the foregoing, the nucleotide sequence of a DNA or RNA molecule coding for a TRADE polypeptide of the invention (or a portion thereof) can be used to derive the TRADE amino acid sequence, using the genetic code to translate the DNA or RNA molecule into an amino acid sequence. Likewise, for any TRADE -amino acid sequence, corresponding nucleotide sequences that can encode TRADE protein can be deduced from the genetic code (which, because of its redundancy, will produce multiple nucleic acid sequences for any given amino acid sequence). Thus, description and/or disclosure herein of a TRADE nucleotide sequence should be considered to also include description and/or disclosure of the amino acid sequence encoded by the nucleotide sequence. Similarly, description and/or disclosure of a TRADE amino acid sequence herein should be considered to also include description and/or disclosure of all possible nucleotide sequences that can encode the amino acid sequence.

One aspect of the invention pertains to isolated nucleic acid molecules that encode TRADE proteins or biologically active portions thereof, as well as nucleic acid fragments sufficient for use as hybridization probes to identify TRADE -encoding nucleic acids (e.g., TRADE mRNA) and fragments for use as PCR primers for the amplification or mutation of TRADE nucleic acid molecules. It will be understood that in discussing the uses of TRADE nucleic acid molecules, e.g., as shown in SEQ. ID NO:1 or 3 or a nucleotide sequence encoding another TRADE family polypeptide, that fragments of such nucleic acid molecules as well as full length TRADE nucleic acid molecules can be used. As used herein, the term "nucleic acid molecule" is intended to include DNA molecules (*e.g.,* cDNA or genomic DNA) and RNA molecules (*e.g.,* mRNA) and analogs of the DNA or RNA generated using nucleotide analogs. The nucleic acid molecule can be single-stranded or double-stranded, but preferably is double-stranded DNA.

A nucleic acid molecule of the present invention, e.g., a nucleic acid molecule having the nucleotide sequence of SEQ ID NO: 1 or 3 or a nucleotide sequence encoding another TRADE family polypeptide, or a portion thereof, can be isolated using standard molecular biology techniques and the sequence information provided herein. For example, using all or portion of the nucleic acid sequence of SEQ ID NO:1 or 3 or a nucleotide sequence encoding another TRADE family polypeptide as a hybridization probe, TRADE nucleic acid molecules can be isolated using standard hybridization and cloning techniques (*e.g.,* as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989).

Moreover, a nucleic acid molecule encompassing all or a portion of SEQ ID NO:1 or 3 or a nucleotide sequence encoding another TRADE family polypeptide can be isolated by the polymerase chain reaction (PCR) using synthetic oligonucleotide primers designed based upon the sequence of SEQ ID NO: 1 or 3 or a nucleotide sequence encoding another TRADE family polypeptide respectively.

Nucleic acid sequences encoding other TRADE family polypeptides can be identified based on nucleic acid and/or amino acid identity with TRADE, possession of TRADE domains, and/or possession of a TRADE activity as defined herein. In addition, several TRADE family members are known in the art which have common functional and structural characteristics. These include: Apo4 (WO99/11791), TRAIN (WO99/13078), AX92_3 (WO98/01554, WO99/20644), αOAF065 and βOAF065 (WO98/38304).

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to TRADE nucleotide sequences can be prepared by standard synthetic techniques, e.g., using an automated DNA synthesizer.

In a preferred embodiment, an isolated nucleic acid molecule of the invention comprises the nucleotide sequence shown in SEQ ID NO:1 or 3 a nucleic acid molecule encoding another TRADE family polypeptide.

In another preferred embodiment, an isolated nucleic acid molecule of the invention comprises a nucleic acid molecule which is a complement of the nucleotide sequence shown in SEQ ID NO:1 or 3 or a nucleotide sequence encoding another TRADE family polypeptide or a portion of any of these nucleotide sequences. A nucleic acid molecule which is complementary to the nucleotide sequence shown in SEQ ID NO:1 or 3 or a nucleotide sequence encoding another TRADE family polypeptide is one which is sufficiently complementary to the nucleotide sequence shown in SEQ ID NO:1 or 3 or a nucleotide sequence encoding another TRADE family polypeptide respectively, such that it can hybridize to the nucleotide sequence shown in SEQ ID NO:1 or 3 or a nucleotide sequence encoding another TRADE family polypeptide respectively, thereby forming a stable duplex.

In still another preferred embodiment, an isolated nucleic acid molecule of the present invention comprises a nucleotide sequence which is at least about 50%, 55%, 60%, 65%, 70%, 75%, 80%, 85%, 90%, 95%, 98% or more homologous to the nucleotide sequence (*e*.*g*., to the entire length of the nucleotide sequence) shown in SEQ ID NO:1 or 3 or a nucleotide sequence encoding another TRADE family polypeptide or a portion thereof, *e.g.,* an intracellular domain (*e*.*g*. comprising nucleotides 577-1251 of SEQ ID NO:1 or 577-1269 of SEQ ID NO:3), an extracellular domain (*e.g.* comprising nucleotides 1-504 of SEQ ID NO:1 or 3), a transmembrane domain (*e.g.* comprising nucleotides 505-576 of SEQ ID NO:1 or 3), a first cysteine-rich domain (*e.g.*, comprising nucleotides 85-189 of SEQ ID NO:1 or 3), a second cysteine-rich domain (*e.g.,* comprising nucleotides 214-342 of SEQ ID NO:1 or 3), a third, partial cysteine-rich domain (*e*.*g*., comprising nucleotides 340-417 of SEQ ID NO:1 or 3), a serine/threonine/proline-rich domain (*e*.*g*., comprising nucleotides 409-504 of SEQ ID NO:1 or 3), a TRADE-related death effector domain (*e.g.,* comprising nucleotides 652-1251 of SEQ ID NO:1 or 652-1269 of SEQ ID NO:3), an N-glycosylation site (*e.g.,* comprising nucleotides 313-324 of SEQ ID NO: 1 or 3), a cAMP/cGMP-dependent protein kinase phosphorylation site (*e.g.,* comprising nucleotides 598 to 609 of SEQ ID NO:1 or 3), a cAMP/cGMP-dependent protein kinase phosphorylation site (*e.g*., comprising nucleotides 712-723 of SEQ ID NO:1 or 3), at least one protein kinase C phosphorylation site (*e.g.,* comprising nucleotides 613 to 621 of SEQ ID NO:1 or 3), a first casein kinase II phosphorylation site (*e.g.,* comprising nucleotides 655 to 666 of SEQ ID NO:1 or 3), a second casein kinase II phosphorylation site (*e.g.,* comprising nucleotides 973 to 984 of SEQ ID NO:1 or 3), a tyrosine kinase phosphorylation site (*e*.*g*., comprising nucleotides 619 to 639 of SEQ ID NO:1 or 3), an N-myristoylation site (*e.g.*, comprising nucleotides 643 to 660 of SEQ ID NO:1 or 3), a kinase activating domain (*e.g.*, comprising nucleotides 1 to 1104 of SEQ ID NO:1 or 3), a TRAF binding domain (*e.g.*, comprising nucleotides 1 to 984 of SEQ ID NO:1 or 3; or comprising nucleotides 1 to 1104 of SEQ ID NO:1 or 3) or a NFkB activating domain (*e.g.,* comprising nucleotides 1 to 1104 of SEQ ID NO:1 or 3). TRADE molecules also lack a TNF receptor death domain consensus sequence in the intracellular portion of the TRADE nucleic acid.

Moreover, the nucleic acid molecule of the invention can comprise only a portion of the nucleic acid sequence of SEQ ID NO:1 or 3 a nucleic acid molecule encoding another TRADE family polypeptide for example a fragment which can be used as a probe or primer or a fragment encoding a biologically active portion of a TRADE protein. The nucleotide sequence determined from the cloning of the TRADE genes allows for the generation of probes and primers designed for use in identifying and/or cloning yet other TRADE family members, as well as TRADE family homologues from other species. The probe/primer typically comprises a substantially purified oligonucleotide. In one embodiment, the oligonucleotide comprises a region of nucleotide sequence that hybridizes under stringent conditions to at least about 12 or 15, preferably about 20 or 25, more preferably about 30, 35, 40, 45, 50, 55, 60, 65, 75, or 100 consecutive nucleotides of a sense sequence of SEQ ID NO:1 or 3 or a nucleotide sequence encoding another TRADE family polypeptide or of a naturally occurring allelic variant or mutant of SEQ ID NO:1 or 3 or a nucleotide sequence encoding another TRADE family polypeptide. In another embodiment, a nucleic acid molecule of the present invention comprises a nucleotide sequence which is at least about 400, 450, 500, 550, 600, 650, 700, 750, 800, 900, 1000, 1100, 1200, 1300, 1400, 1500, or more nucleotides in length and hybridizes under stringent hybridization conditions to a nucleic acid molecule of SEQ ID NO:1 or 3 or a nucleotide sequence encoding another TRADE family polypeptide or the complement thereof.

In another embodiment, a nucleic acid molecule of the invention comprises at least about 100, 200, 300, 400, 500, 600, 700, 800, 900, 1000, 1100, 1200, 1300, 1400, 1500, or more contiguous nucleotides of SEQ ID NO: 1 or 3 or a nucleic acid molecule encoding another TRADE family polypeptide.

In other embodiments, a nucleic acid molecule of the invention has at least 70% identity, more preferably 80% identity, and even more preferably 90% identity with a nucleic acid molecule comprising: at least about 300, 400, 500, 600, 700, 800, 900, 1000, 1100, 1200, 1300, 1400 or about 1500 nucleotides of SEQ ID NO: 1 or 3 or a nucleic acid molecule encoding another TRADE family polypeptide.

Probes based on the TRADE nucleotide sequences can be used to detect transcripts or genomic sequences encoding the same or homologous proteins. In preferred embodiments, the probe further comprises a label group attached thereto, e.g., the label group can be a radioisotope, a fluorescent compound, an enzyme, or an enzyme co-factor. Such probes can be used as a part of a diagnostic test kit for identifying cells or tissues, particularly epithelial cells or tissues, particularly epithelial cells or tissues, which misexpress a TRADE protein, such as by measuring a level of a TRADE - encoding nucleic acid in a sample of cells from a subject e.g., detecting TRADE mRNA levels or determining whether a genomic TRADE gene has been mutated or deleted.

A nucleic acid fragment encoding a "biologically active portion of a TRADE protein" can be prepared by isolating a portion of the nucleotide sequence of SEQ ID NO:1 or 3 or a nucleotide sequence encoding another TRADE family polypeptide which encodes a polypeptide having a TRADE biological activity (e.g., the ability to modulate proliferation, apoptosis, and/or signaling via an NFkB or JNK signaling pathway), expressing the encoded portion of the TRADE protein (e.g., by recombinant expression *in vitro*) and assessing the activity of the encoded portion of the TRADE protein.

Nucleic acid molecules that differ from SEQ ID NO: 1 or 3 or a nucleic acid molecule encoding another TRADE family polypeptide due to degeneracy of the genetic code, and thus encode the same TRADE protein as that encoded by SEQ ID NO: 1 or 3 or a nucleic acid molecule encoding another TRADE family polypeptide are encompassed by the invention. Accordingly, in another embodiment, an isolated nucleic acid molecule of the invention has a nucleotide sequence encoding a protein having an amino acid sequence shown in SEQ ID NO: 2 or 4 or an amino acid sequence of another TRADE family polypeptide.

In addition to the TRADE nucleotide sequence shown in SEQ ID NO:1 or 3 or a nucleotide sequence encoding another TRADE family polypeptide, it will be appreciated by those skilled in the art that DNA sequence polymorphisms that lead to changes in the amino acid sequences of the TRADE proteins may exist within a population (e.g., the human population). Such genetic polymorphism in the TRADE genes may exist among individuals within a population due to natural allelic variation. As used herein, the terms "gene" and "recombinant gene" refer to nucleic acid molecules which include an open reading frame encoding a TRADE protein, preferably a mammalian TRADE protein, and can further include non-coding regulatory sequences, and introns. Such natural allelic variations include both functional and non-functional TRADE proteins and can typically result in 1-5% variance in the nucleotide sequence of a TRADE gene. Any and all such nucleotide variations and resulting amino acid polymorphisms in TRADE genes that are the result of natural allelic variation and that do not alter the functional activity of a TRADE protein can be used in the claimed methods.

Moreover, nucleic acid molecules encoding other TRADE family members and, thus, which have a nucleotide sequence which differs from the TRADE family sequence of SEQ ID NO:1 or 3 or a nucleotide sequence encoding another TRADE family polypeptide are intended to be within the scope of the invention. Moreover, nucleic acid molecules encoding TRADE proteins from different species, and thus which have a nucleotide sequence which differs from the TRADE sequence of SEQ ID NO:1 or 3 or a nucleotide sequence encoding another TRADE family polypeptide can be used in the claimed methods.

Nucleic acid molecules corresponding to natural allelic variants and homologues of the TRADE molecules of the invention can be isolated, e.g., based on their homology to the TRADE nucleic acids disclosed herein using the cDNAs disclosed herein, or portions thereof, as a hybridization probe according to standard hybridization techniques. For example, a TRADE DNA can be isolated from a human genomic DNA library using all or portion of SEQ ID NO:1 or 3 or a nucleotide sequence encoding another TRADE family polypeptide as a hybridization probe and standard hybridization techniques (*e.g.*, as described in Sambrook, J., et al. Molecular Cloning: A Laboratory Manual. 2nd, ed., Cold Spring Harbor Laboratory, Cold Spring Harbor, NY, 1989). Moreover, a nucleic acid molecule encompassing all or a portion of a TRADE gene can be isolated by the polymerase chain reaction using oligonucleotide primers designed based upon the sequence of SEQ ID NO: 1 or 3 or a nucleic acid molecule encoding another TRADE family polypeptide. For example, mRNA can be isolated from cells (*e.g*., by the guanidinium-thiocyanate extraction procedure of Chirgwin et al., 1979, Biochemistry 18: 5294-5299) and cDNA can be prepared using reverse transcriptase (*e.g.*, Moloney MLV reverse transcriptase, available from Gibco/BRL, Bethesda, MD; or AMV reverse transcriptase, available from Seikagaku America, Inc., St. Petersburg, FL). Synthetic oligonucleotide primers for PCR amplification can be designed based upon the nucleotide sequence shown in SEQ ID NO: 1 or 3 or a nucleic acid molecule encoding another TRADE family polypeptide. A nucleic acid molecule of the invention can be amplified using cDNA or, alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to a TRADE nucleotide sequence can be prepared by standard synthetic techniques, *e.g.*, using an automated DNA synthesizer.

In another embodiment, an isolated nucleic acid molecule of the invention can be identified based on shared nucleotide sequence identity using a mathematical algorithm. Such algorithms are outlined in more detail below (see, e.g., section III).

In another embodiment, an isolated nucleic acid molecule of the invention is at least 15, 20, 25, 30 or more nucleotides in length and hybridizes under stringent conditions to the nucleic acid molecule comprising the nucleotide sequence of SEQ ID NO:1 or 3 or a nucleotide sequence encoding another TRADE family polypeptide or its complement. In other embodiment, the nucleic acid molecule is at least 30, 50, 100, 150,200, 250, 300, 350, 400, 450, 500, 550, or 600 nucleotides in length. As used herein, the term "hybridizes under stringent conditions" is intended to describe conditions for hybridization and washing under which nucleotide sequences at least 30%, 40%, 50%, or 60% homologous to each other typically remain hybridized to each other. Preferably, the conditions are such that sequences at least about 70%, more preferably at least about 80%, even more preferably at least about 85% or 90% homologous to each other typically remain hybridized to each other. Such stringent conditions are known to those skilled in the art and can be found in Current Protocols in Molecular Biology, John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Preferably, an isolated nucleic acid molecule of the invention that hybridizes under stringent conditions to the sequence of SEQ ID NO:1 or 3 or a nucleic acid molecule encoding another TRADE family polypeptide or its complement corresponds to a naturally-occurring nucleic acid molecule.

As used herein, a "naturally-occurring" nucleic acid molecule refers to an RNA or DNA molecule having a nucleotide sequence that occurs in nature (e.g., encodes a natural protein). In addition to the TRADE nucleotide sequences shown in SEQ ID NO: 1 or 3 or a nucleic acid molecule encoding another TRADE family polypeptide it will be appreciated by those skilled in the art that DNA sequence polymorphisms that lead to minor changes in the nucleotide or amino acid sequences of a TRADE may exist within a population. Such genetic polymorphism in a TRADE gene may exist among individuals within a population due to natural allelic variation. Such natural allelic variations can typically result in 1-2 % variance in the nucleotide sequence of the gene. Such nucleotide variations and resulting amino acid polymorphisms in a TRADE that are the result of natural allelic variation and that do not alter the functional activity of a TRADE polypeptide are within the scope of the invention.

In addition to naturally-occurring allelic variants of TRADE sequences that may exist in the population, the skilled artisan will further appreciate that minor changes may be introduced by mutation into nucleotide sequences, e.g., of SEQ ID NO: 1 or 3 or a nucleic acid molecule encoding another TRADE family polypeptide, thereby leading to changes in the amino acid sequence of the encoded protein, without altering the functional activity of a TRADE protein. For example, nucleotide substitutions leading to amino acid substitutions at "non-essential" amino acid residues may be made in the sequence of SEQ ID NO: 1 or 3 or a nucleic acid molecule encoding another TRADE family polypeptide. A "non-essential" amino acid residue is a residue that can be altered from the wild-type sequence of a TRADE nucleic acid molecule (*e.g.*, the sequence of SEQ ID NO: 1 or 3 or a nucleic acid molecule encoding another TRADE family polypeptide) without altering the functional activity of a TRADE molecule. Exemplary residues which are non-essential and, therefore, amenable to substitution, can be identified by one of ordinary skill in the art by performing an amino acid alignment of TRADE-related molecules and determining residues that are not conserved. Such residues, because they have not been conserved, are more likely amenable to substitution.

Accordingly, another aspect of the invention pertains to nucleic acid molecules encoding TRADE proteins that contain changes in amino acid residues that are not essential for a TRADE activity. Such TRADE proteins differ in amino acid sequence from SEQ ID NO: 2 or 4 or an amino acid sequence of another TRADE family polypeptide yet retain an inherent TRADE activity. An isolated nucleic acid molecule encoding a non-natural variant of a TRADE protein can be created by introducing one or more nucleotide substitutions, additions or deletions into the nucleotide sequence of SEQ ID NO: 1 or 3 or a nucleic acid molecule encoding another TRADE family polypeptide such that one or more amino acid substitutions, additions or deletions are introduced into the encoded protein. Mutations can be introduced into SEQ ID NO:1 or 3 or a nucleic acid molecule encoding another TRADE family polypeptide by standard techniques, such as site-directed mutagenesis and PCR-mediated mutagenesis. Preferably, conservative amino acid substitutions are made at one or more non-essential amino acid residues. A "conservative amino acid substitution" is one in which the amino acid residue is replaced with an amino acid residue having a similar side chain. Families of amino acid residues having similar side chains have been defined in the art, including basic side chains (*e.g*., lysine, arginine, histidine), acidic side chains (*e.g*., aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g.*, tyrosine, phenylalanine, tryptophan, histidine). Thus, a nonessential amino acid residue in a TRADE is preferably replaced with another amino acid residue from the same side chain family.

Alternatively, in another embodiment, mutations can be introduced randomly along all or part of a TRADE coding sequence, such as by saturation mutagenesis, and the resultant mutants can be screened for their ability to bind to DNA and/or activate transcription, to identify mutants that retain functional activity. Following mutagenesis, the encoded a TRADE mutant protein can be expressed recombinantly in a host cell and the functional activity of the mutant protein can be determined using assays available in the art for assessing a TRADE activity.

Yet another aspect of the invention pertains to isolated nucleic acid molecules encoding a TRADE fusion proteins. Such nucleic acid molecules, comprising at least a first nucleotide sequence encoding a full-length TRADE protein, polypeptide or peptide having a TRADE activity operatively linked to a second nucleotide sequence encoding a non- TRADE protein, polypeptide or peptide, can be prepared by standard recombinant DNA techniques.

In a preferred embodiment, a variant or mutant TRADE protein can be assayed for TRADE activity as described herein.

In addition to the nucleic acid molecules encoding TRADE proteins described above, another aspect of the invention pertains to isolated nucleic acid molecules which are antisense thereto. An "antisense" nucleic acid comprises a nucleotide sequence which is complementary to a "sense" nucleic acid encoding a protein, e.g., complementary to the coding strand of a double-stranded cDNA molecule or complementary to an mRNA sequence. Accordingly, an antisense nucleic acid molecule can hydrogen bond to a sense nucleic acid molecule. The antisense nucleic acid molecule can be complementary to an entire TRADE coding strand, or only to a portion thereof. In one embodiment, an antisense nucleic acid molecule is antisense to a "coding region" of the coding strand of a nucleotide sequence encoding TRADE. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding TRADE. The term "noncoding region" refers to 5' and 3' sequences which flank the coding region that are not translated into amino acids (i.e., also referred to as 5' and 3' untranslated regions).

Given the coding strand sequences encoding TRADE disclosed herein, antisense nucleic acids of the invention can be designed according to the rules of Watson and Crick base pairing. The antisense nucleic acid molecule can be complementary to the entire coding region of TRADE mRNA, but more preferably is an oligonucleotide which is antisense to only a portion of the coding or noncoding region of TRADE mRNA. For example, the antisense oligonucleotide can be complementary to the region surrounding the translation start site of TRADE mRNA. An antisense oligonucleotide can be, for example, about 5, 10, 15, 20, 25, 30, 35, 40, 45 or 50 nucleotides in length. An antisense nucleic acid molecule of the invention can be constructed using chemical synthesis and enzymatic ligation reactions using procedures known in the art. For example, an antisense nucleic acid molecule (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acid molecules, e.g., phosphorothioate derivatives and acridine substituted nucleotides can be used. Examples of modified nucleotides which can be used to generate the antisense nucleic acid molecule include 5-fluorouracil, 5-bromouracil, 5-chlorouracil, 5-iodouracil, hypoxanthine, xantine, 4-acetylcytosine, S-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a TRADE protein to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention include direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids. Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330).

In still another embodiment, an antisense nucleic acid of the invention is a ribozyme. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach, 1988, Nature 334:585-591)) can be used to catalytically cleave TRADE mRNA transcripts to thereby inhibit translation of TRADE mRNA. A ribozyme having specificity for a TRADE -encoding nucleic acid can be designed based upon the nucleotide sequence of SEQ ID NO:1 or 3 a nucleic acid molecule encoding another TRADE family polypeptide. For example, a derivative of a *Tetrahymena* L-19 IVS RNA can be constructed in which the nucleotide sequence of the active site is complementary to the nucleotide sequence to be cleaved in a TRADE-encoding mRNA. See, e.g., Cech et al. U.S. Patent No. 4,987,071; and Cech et al. U.S. Patent No. 5,116,742. Alternatively, TRADE mRNA can be used to select a catalytic RNA having a specific ribonuclease activity from a pool of RNA molecules. See, e.g., Bartel, D. and Szostak, J.W., 1993, Science 261:1411-1418.

Alternatively, gene expression can be inhibited by targeting nucleotide sequences complementary to the regulatory region of TRADE (e.g., the TRADE promoter and/or enhancers) to form triple helical structures that prevent transcription of the TRADE gene in target cells. See generally, Helene, C., 1991, Anticancer Drug Des. 6(6):569-84; Helene, C. et al., 1992, Ann. N.Y. Acad. Sci. 660:27-36; and Maher, L.J., 1992, Bioassays 14(12):807-15.

In yet another embodiment, the TRADE nucleic acid molecules of the present invention can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. et al., 1996, Bioorganic & Medicinal Chemistry 4 (1): 5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. *et al.,* 1996, *supra;* Perry-O'Keefe et al., 1996, Proc. Natl. Acad. Sci. USA 93: 14670-675.

PNAs of TRADE nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of TRADE nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup B., 1996, *supra*)); or as probes or primers for DNA sequencing or hybridization (Hyrup B. *et al.,* 1996, *supra;* Perry-O'Keefe *supra*).

In another embodiment, PNAs of TRADE can be modified, (e.g., to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras of TRADE nucleic acid molecules can be generated which may combine the advantageous properties of PNA and DNA. Such chimeras allow DNA recognition enzymes, (e.g., RNAse H and DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup B., 1996, *supra).* The synthesis of PNA-DNA chimeras can be performed as described in Hyrup B., 1996, supra and Finn P.J. et al., 1996, Nucleic Acids Res. 24 (17): 3357-63. For example, a DNA chain can be synthesized on a solid support using standard phosphoramidite coupling chemistry and modified nucleoside analogs, e.g., 5'-(4-methoxytrityl)amino-5'-deoxy-thymidine phosphoramidite, can be used as a between the PNA and the 5' end of DNA (Mag, M. et al., 1989, Nucleic Acid Res. 17: 5973-88). PNA monomers are then coupled in a stepwise manner to produce a chimeric molecule with a 5' PNA segment and a 3' DNA segment (Finn P.J. *et al.,* 1996, *supra).* Alternatively, chimeric molecules can be synthesized with a 5' DNA segment and a 3' PNA segment (Peterser, K.H. et al., 1975, Bioorganic Med. Chem. Lett. 5: 1119-11124).

In other embodiments, the oligonucleotide may include other appended groups such as peptides (e.g., for targeting host cell receptors *in vivo*), or agents facilitating transport across the cell membrane (see, e.g., Letsinger et al., 1989, Proc. Natl. Acad. Sci. US. 86:6553-6556; Lemaitre et al., 1987, Proc. Natl. Acad Sci. USA 84:648-652; PCT Publication No. WO88/09810) or the blood-brain barrier (see, e.g., PCT Publication No. WO89/10134). In addition, oligonucleotides can be modified with hybridization-triggered cleavage agents (See, e.g., Krol et al., 1988, Bio-Techniques 6:958-976) or intercalating agents. (See, e.g., Zon, 1988, Pharm. Res. 5:539-549). To this end, the oligonucleotide may be conjugated to another molecule, (e.g., a peptide, hybridization triggered cross-linking agent, transport agent, or hybridization-triggered cleavage agent).

Antisense polynucleotides may be produced from a heterologous expression cassette in a transfectant cell or transgenic cell. Alternatively, the antisense polynucleotides may comprise soluble oligonucleotides that are administered to the external milieu, either in the culture medium *in vitro* or in the circulatory system or in interstitial fluid *in vivo.* Soluble antisense polynucleotides present in the external milieu have been shown to gain access to the cytoplasm and inhibit translation of specific mRNA species.

### B. Isolated TRADE Proteins, Fragments Thereof, and Anti-TRADE Antibodies

Isolated TRADE proteins, and biologically active portions thereof can also be used as modulating agents, as well as polypeptide fragments suitable for use as immunogens to raise anti-TRADE antibodies. In one embodiment, native TRADE proteins can be isolated from cells or tissue sources by an appropriate purification scheme using standard protein purification techniques. In another embodiment, TRADE proteins are produced by recombinant DNA techniques. Alternative to recombinant expression, a TRADE protein or polypeptide can be synthesized chemically using standard peptide synthesis techniques. It will be understood that in discussing the uses of TRADE proteins (*e.g.*, as shown in SEQ. ID NO:2 or 4 or an amino acid sequence encoding another TRADE family polypeptide), that fragments of such proteins that are not full length TRADE polypeptides (*e.g.*, that comprise one or more TRADE domains, *e.g.* an intracellular domain comprising amino acid residues corresponding to residues 193-417 of SEQ ID NO:2 or 193-423 of SEQ ID NO:4, an extracellular domain comprising amino acid residues corresponding to residues 1-168 of SEQ ID NO:2 or 4, a transmembrane domain comprising amino acid residues corresponding to residues 169-192 of SEQ ID NO:2 or 4, a first cysteine-rich domain comprising amino acid residues corresponding to residues 29-63 of SEQ ID NO:2 or 4, a second cysteine-rich domain comprising amino acid residues corresponding to residues 72-114 of SEQ ID NO:2 or 4, a third cysteine-rich domain comprising amino acid residues corresponding to residues 114-139 of SEQ ID NO:2 or 4, a serine/threonine/proline-rich domain comprising amino acid residues corresponding to residues 137-168 of SEQ ID NO:2 or 4, a TRADE-related death effector domain comprising amino acid residues corresponding to residues 218-417 of SEQ ID NO:2 or 218-423 of SEQ ID NO:4, an N-glycosylation site at a site corresponding to residues 105-108 of SEQ ID NO:2 or 4, a cAMP/cGMP-dependent protein kinase phosphorylation site at a site corresponding to residues 200 to 203 of SEQ ID NO: 2 or 4, a cAMP/cGMP-dependent protein kinase phosphorylation site at a site corresponding to residues 238 to 241 of SEQ ID NO: 2 or 4, a protein kinase C phosphorylation site at a site corresponding to residues 205 to 207 of SEQ. ID NO: 2 or 4, a casein kinase II phosphorylation site at a site corresponding to residues 219 to 222 of SEQ ID NO: 2 or 4, and at a site corresponding to residues 325 to 328 of SEQ ID NO:2 or 4, a tyrosine kinase phosphorylation site at a site corresponding to residues 207-213 of SEQ ID NOs:2 or 4, or an N-myristoylation site at a site corresponding to residues 215-220 of SEQ ID NO:2 or 4) are also within the scope of the invention.

Another aspect of the invention pertains to isolated TRADE proteins. Preferably, the TRADE proteins comprise the amino acid sequence encoded by SEQ ID NO:1 or 3 or a nucleotide sequence encoding another TRADE family polypeptide or a portion thereof. In another preferred embodiment, the protein comprises the amino acid sequence of SEQ ID NO: 2 or 4 or an amino acid sequence of another TRADE family polypeptide or a portion thereof. In other embodiments, the protein has at least 50%, at least 60 % amino acid identity, more preferably 70% amino acid identity, more preferably 80%, and even more preferably, 90% or 95% amino acid identity with the amino acid sequence shown in SEQ ID NO: 2 or 4 or an amino acid sequence of another TRADE family polypeptide or a portion thereof, e.g., the consensus domains set forth above.

Preferred portions of TRADE polypeptide molecules are biologically active, i.e., encode a portion of the TRADE polypeptide having the ability to activate NFkB and/or JNK to thereby modulate proliferation and/or having the ability to modulate apoptosis in a cell. Preferably, the cell is an epithelial cell, e.g., a ductile epithelial cell.

Biologically active portions of a TRADE protein include peptides comprising amino acid sequences sufficiently homologous to or derived from the amino acid sequence of the TRADE protein, which include fewer amino acids than the full length TRADE proteins, and exhibit at least one activity of a TRADE protein.

To determine the percent identity of two amino acid sequences or of two nucleic acid sequences, the sequences are aligned for optimal comparison purposes (e.g., gaps can be introduced in one or both of a first and a second amino acid or nucleic acid sequence for optimal alignment). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence. The residues at corresponding positions are then compared and when a position in one sequence is occupied by the same residue as the corresponding position in the other sequence, then the molecules are identical at that position. The percent identity between two sequences, therefore, is a function of the number of identical positions shared by two sequences (*i.e.*, % identity = # of identical positions/total # of positions x 100). The percent identity between the two sequences is a function of the number of identical positions shared by the sequences, taking into account the number of gaps, and the length of each gap, which are introduced for optimal alignment of the two sequences. As used herein amino acid or nucleic acid "identity" is equivalent to amino acid or nucleic acid "homology".

The comparison of sequences and determination of percent identity between two sequences can be accomplished using a mathematical algorithm. A non-limiting example of a mathematical algorithm utilized for comparison of sequences is the algorithm of Karlin and Altschul, 1990, Proc. Natl. Acad. Sci. USA 87:2264, modified as in Karlin and Altschul, 1993, Proc. Natl. Acad. Sci. USA 90:5873. Such an algorithm is incorporated into the NBLAST and XBLAST programs (version 2.0) of Altschul, et al., 1990, J. Mol. Biol. 215:403. BLAST nucleotide searches can be performed with the NBLAST program score=100, wordlength=12 to obtain nucleotide sequences homologous to the nucleic acid molecules of the invention. BLAST protein searches can be performed with the XBLAST program, score=50, wordlength =3 to obtain amino acid sequences homologous to the protein molecules of the invention. To obtain gapped alignments for comparison purposes, Gapped BLAST can be utilized as described in Altschul et al., 1997, Nucleic Acids Research 25(17):3389. When utilizing BLAST and Gapped BLAST programs, the default parameters of the respective programs (e.g., XBLAST and NBLAST) can be used. See http://www.ncbi.nlm.nih.gov. Another preferred, non-limiting algorithm utilized for the comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

Another non-limiting example of a mathematical algorithm utilized for the alignment of protein sequences is the Lipman-Pearson algorithm (Lipman and Pearson, 1985, Science 227:1435). When using the Lipman-Pearson algorithm, a PAM250 weight residue table, a gap length penalty of 12, a gap penalty of 4, and a Kutple of 2 can be used. A preferred, non-limiting example of a mathematical algorithm utilized for the alignment of nucleic acid sequences is the Wilbur-Lipman algorithm (Wilbur and Lipman, 1983, Proc. Natl. Acad. Sci. USA 80:726). When using the Wilbur-Lipman algorithm, a window of 20, gap penalty of 3, Ktuple of 3 can be used. Both the Lipman-Pearson algorithm and the Wilbur-Lipman algorithm are incorporated, for example, into the MEGALIGN program (e.g., version 3.1.7) which is part of the DNASTAR sequence analysis software package.

Additional algorithms for sequence analysis are known in the art, and include ADVANCE and ADAM., described in Torelli and Robotti, 1994, Comput. Appl. Biosci. 10:3; and FASTA, described in Pearson and Lipman, 1988, Proc. Natl. Acad. Sci USA 85:2444.

In a preferred embodiment, the percent identity between two amino acid sequences is determined using the GAP program in the GCG software package, using either a Blosum 62 matrix or a PAM250 matrix, and a gap weight of 16, 14, 12; 10, 8, 6, or 4 and a length weight of 1, 2, 3, 4, 5, or 6. In yet another preferred embodiment, the percent identity between two nucleotide sequences is determined using the GAP program in the GCG software package, using a NWSgapdna. CMP matrix and a gap weight of 40, 50, 60, 70, or 80 and a length weight of 1, 2, 3, 4, 5, or 6.

Protein alignments can also be made using the Geneworks global protein alignment program (e.g., version 2.5.1) with the cost to open gap set at 5, the cost to lengthen gap set at 5, the minimum diagonal length set at 4, the maximum diagonal offset set at 130, the consensus cutoff set at 50% and utilizing the Pam 250 matrix.

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul et al., 1990, J. Mol. Biol. 215:403-10. BLAST nucleotide searches can be performed with the NBLAST program, score = 100, wordlength = 12 to obtain nucleotide sequences homologous to TRADE nucleic acid molecules of the invention. BLAST protein searches can be performed with the XBLAST program, score = 50, wordlength = 3 to obtain amino acid sequences homologous to TRADE protein molecules of the invention. To obtain gapped alignments for comparison purposes, Gapped BLAST can be utilized as described in Altschul et al., 1997, Nucleic Acids Res. 25(17):3389-3402. When utilizing BLAST and Gapped BLAST programs, the default parameters of the respective programs (e.g., XBLAST and NBLAST) can be used. For example, the nucleotide sequences of the invention can be analyzed using the default BLASTN matrix 1-3 with gap penalties set at: existence 11 and extension 1. The amino acid sequences of the invention can be analyzed using the default settings: the Blosum62 matrix with gap penalties set at existence 11 and extension 1. See http://www.ncbi.nlm.nih.gov.

The invention also provides TRADE chimeric or fusion proteins. As used herein, a TRADE "chimeric protein" or "fusion protein" comprises a TRADE polypeptide operatively linked to a non- TRADE polypeptide. An " TRADE polypeptide" refers to a polypeptide having an amino acid sequence corresponding to TRADE polypeptide, whereas a "non-TRADE polypeptide" refers to a polypeptide having an amino acid sequence corresponding to a protein which is not substantially homologous to the TRADE protein, e.g., a protein which is different from the TRADE protein and which is derived from the same or a different organism. Within a TRADE fusion protein the TRADE polypeptide can correspond to all or a portion of a TRADE protein. In a preferred embodiment, a TRADE fusion protein comprises at least one biologically active portion of a TRADE protein, e.g., a TRADE consensus domain. Within the fusion protein, the term "operatively linked" is intended to indicate that the TRADE polypeptide and the non-TRADE polypeptide are fused in-frame to each other. The non-TRADE polypeptide can be fused to the N-terminus or C-terminus of the TRADE polypeptide.

For example, in one embodiment, the fusion protein is a GST-TRADE member fusion protein in which the TRADE member sequences are fused to the C-terminus of the GST sequences. In another embodiment, the fusion protein is a TRADE -HA fusion protein in which the TRADE member nucleotide sequence is inserted in a vector such as pCEP4-HA vector (Herrscher, R.F. et al., 1995, Genes Dev. 9:3067-3082) such that the TRADE member sequences are fused in frame to an influenza haemagglutinin epitope tag. Such fusion proteins can facilitate the purification of a recombinant TRADE member.

Fusion proteins and peptides produced by recombinant techniques may be secreted and isolated from a mixture of cells and medium containing the protein or peptide. Alternatively, the protein or peptide may be retained cytoplasmically and the cells harvested, lysed and the protein isolated. A cell culture typically includes host cells, media and other byproducts. Suitable media for cell culture are well known in the art. Protein and peptides can be isolated from cell culture media, host cells, or both using techniques known in the art for purifying proteins and peptides. Techniques for transfecting host cells and purifying proteins and peptides are known in the art.

Preferably, a TRADE fusion protein of the invention is produced by standard recombinant DNA techniques. For example, DNA fragments coding for the different polypeptide sequences are ligated together in-frame in accordance with conventional techniques, for example employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (*e.g.,* a GST polypeptide or an HA epitope tag). A TRADE encoding nucleic acid can be cloned into such an expression vector such that the fusion moiety is linked in-frame to the TRADE protein.

In another embodiment, the fusion protein is a TRADE protein containing a heterologous signal sequence at its N-terminus. In certain host cells (e.g., mammalian host cells), expression and/or secretion of TRADE can be increased through use of a heterologous signal sequence. The TRADE fusion proteins of the invention can be incorporated into pharmaceutical compositions and administered to a subject *in vivo.* Use of TRADE fusion proteins may be useful therapeutically for the treatment of disorders, e.g., as soluble antagonists of the TRADE ligand. Disorders that would benefit from such treatment include, e.g. cancer or Alzheimer's disease. Such Fc fusion proteins can be used as soluble antagonists of the TRADE ligand. Moreover, the TRADE-fusion proteins of the invention can be used as immunogens to produce anti-TRADE antibodies in a subject.

Preferably, a TRADE chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). A TRADE-encoding nucleic acid can be cloned into such an expression vector such that the fusion moiety is linked in-frame to the TRADE protein.

In one embodiment, a TRADE-Fc fusion protein can be made using techniques that are known in the art. For example, as taught in the instant examples, a soluble TRADE-Fc fusion protein can be constructed by joining the cDNA sequence encoding the extracellular region of TRADE to the hinge-C_{H}2-C_{H}3 regions of human immunoglobulin (Ig). Any isotype may be used in making such a construct, for example, Fc γ1, γ2, γ3, ε or α. Cells can be transfected with a plasmid carying the TRADE-Ig construct, cultured, and conditioned medium harvested. The fusion protein can then be purified, e.g., using a column of immobilized protein A.

In another embodiment, a spacer of glycine and serine residues may be incorporated between the TRADE and Fc sequences. For example, a TRADE portion of a TRADE fusion protein might ordinarily terminate with the C-terminal sequence of the TRADE extracellular region: STASSPRDT (SEQ ID NO:9); or at other residues between the second cysteine-rich domain and the transmembrane and the residues of the Ig γ1 hinge could be DKTHTCP (e.g., starting at residue 104 of the polypeptide sequence underaccession number P01857 in the SwissProt database, http://www.expasy.ch/sprot). These could be followed by the C_{H}2-C_{H}3 domain residues or a spacer of glycine and serine residues may be incorporated between the TRADE and Fc sequences

In another embodiment, allotypic variants of Fc sequences could be used to construct Fc fusion proteins. In another embodiment, mutations which block effector functions, such as, for example, complement and Fc receptor binding (Armour et al., 1999, Eur. J. Immunol., 29:2613; Morgan et al., 1995, Immunology 86: 319; Lund et al., 1991, J. Immunol. 147:2657) could be incorporated into a fustion protein.

The present invention also pertains to variants of the TRADE proteins which function as either TRADE agonists (mimetics) or as TRADE antagonists. Variants of the TRADE proteins can be generated by mutagenesis, e.g., discrete point mutation or truncation of a TRADE protein. An agonist of the TRADE proteins can retain substantially the same, or a subset, of the biological activities of the naturally occurring form of a TRADE protein. An antagonist of a TRADE protein can inhibit one or more of the activities of the naturally occurring form of the TRADE protein by, for example, competitively modulating a cellular activity of a TRADE protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the TRADE protein. In one embodiment, the invention pertains to derivatives of TRADE which may be formed by modifying at least one amino acid residue of TRADE by oxidation, reduction, or other derivatization processes known in the art.

In one embodiment, variants of a TRADE protein which function as either TRADE agonists (mimetics) or as TRADE antagonists can be identified by screening combinatorial libraries of mutants, *e.g.,* truncation mutants, of a TRADE protein for TRADE protein agonist or antagonist activity. In one embodiment, a variegated library of TRADE variants is generated by combinatorial mutagenesis at the nucleic acid level and is encoded by a variegated gene library. A variegated library of TRADE variants can be produced by, for example, enzymatically ligating a mixture of synthetic oligonucleotides into gene sequences such that a degenerate set of potential TRADE sequences is expressible as individual polypeptides, or alternatively, as a set of larger fusion proteins (e.g., for phage display) containing the set of TRADE sequences therein. There are a variety of methods which can be used to produce libraries of potential TRADE variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential TRADE sequences. Methods for synthesizing degenerate oligonucleotides are known in the art (see, e.g., Narang, S.A., 1983, Tetrahedron 39:3; Itakura et al., 1984, Annu. Rev. Biochem. 53:323; Itakura et al., 1984, Science 198:1056; Ike et al., 1983, Nucleic Acid Res. 11:477).

In addition, libraries of fragments of a TRADE protein coding sequence can be used to generate a variegated population of TRADE fragments for screening and subsequent selection of variants of a TRADE protein. In one embodiment, a library of coding sequence fragments can be generated by treating a double stranded PCR fragment of a TRADE coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the TRADE protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of TRADE proteins. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify TRADE variants (Arkin and Yourvan, 1992, Proc. Natl. Acad Sci. USA 89:7811-7815; Delgrave et al., 1993, Protein Engineering 6(3):327-331).

In one embodiment, cell based assays can be exploited to analyze a variegated TRADE library. For example, a library of expression vectors can be transfected into a cell line which ordinarily synthesizes and secretes TRADE. The transfected cells are then cultured such that TRADE and a particular mutant TRADE are secreted and the effect of expression of the mutant on TRADE activity in cell supernatants can be detected, e.g., by any of a number of enzymatic assays. Plasmid DNA can then be recovered from the cells which score for inhibition, or alternatively, potentiation of TRADE activity, and the individual clones further characterized.

In addition to TRADE polypeptides consisting only of naturally-occurring amino acids, TRADE peptidomimetics are also provided. Peptide analogs are commonly used in the pharmaceutical industry as non-peptide drugs with properties analogous to those of the template peptide. These types of non-peptide compound are termed "peptide mimetics" or "peptidomimetics" (Fauchere, J., 1986, Adv. Drug Res. 15:29; Veber and Freidinger, 1985, TINS p.392; and Evans et al., 1987, J. Med. Chem 30:1229, which are incorporated herein by reference) and are usually developed with the aid of computerized molecular modeling. Peptide mimetics that are structurally similar to therapeutically useful peptides may be used to produce an equivalent therapeutic or prophylactic effect. Generally, peptidomimetics are structurally similar to a paradigm polypeptide (i.e., a polypeptide that has a biological or pharmacological activity), such as human TRADE, but have one or more peptide linkages optionally replaced by a linkage selected from the group consisting of: -CH2NH-, -CH2S-, -CH2-CH2-,-CH=CH- (cis and trans), -COCH2-, -CH(OH)CH2-, and -CH2SO-, by methods known in the art and further described in the following references: Spatola, A.F. in "Chemistry and Biochemistry of Amino Acids, Peptides, and Proteins," B. Weinstein, eds., Marcel Dekker, New York, p. 267 (1983); Spatola, A. F., Vega Data (March 1983), Vol. 1, Issue 3, "Peptide Backbone Modifications" (general review); Morley, J. S., 1980, Trends Pharm Sci pp. 463-468 (general review); Hudson, D. et al., 1979, Int J Pept Prot Res 14:177-185 (-CH2NH-, -CH2CH2-); Spatola, A. F. et al., 1986, Life Sci 38:1243-1249 (-CH2-S); Hann, M. M., 1982, J Chem Soc Perkin Trans I 307-314 (-CH-CH-, cis and trans); Almquist, R. G. et al., 1980, J Med Chem 23:1392-1398 (-COCH2-); Jennings-White, C. et al., 1992, Tetrahedron Lett 23:2533 (-COCH2-); Szelke, M. et al., 1982, European Appln. EP 45665 CA: 97:39405 (1982)(-CH(OH)CH2-); Holladay, M. W. et al., 1983, Tetrahedron Lett 24:4401-4404 (-CH(OH)CH2-); and Hruby, V. J., 1982, Life Sci 31:189-199 (-CH2-S-); each of which is incorporated herein by reference. A particularly preferred non-peptide linkage is -CH2NH-. Such peptide mimetics may have significant advantages over polypeptide embodiments, including, for example: more economical production, greater chemical stability, enhanced pharmacological properties (half-life, absorption, potency, efficacy, etc.), altered specificity (e.g., a broad-spectrum of biological activities), reduced antigenicity, and others. Labeling of peptidomimetics usually involves covalent attachment of one or more labels, directly or through a spacer (e.g., an amide group), to non-interfering position(s) on the peptidomimetic that are predicted by quantitative structure-activity data and/or molecular modeling. Such non-interfering positions generally are positions that do not form direct contacts with the macromolecules(s) to which the peptidomimetic binds to produce the therapeutic effect. Derivitization (e.g., labeling) of peptidomimetics should not substantially interfere with the desired biological or pharmacological activity of the peptidomimetic.

Systematic substitution of one or more amino acids of a TRADE amino acid sequence with a D-amino acid of the same type (e.g., D-lysine in place of L-lysine) may be used to generate more stable peptides. In addition, constrained peptides comprising a TRADE amino acid sequence or a substantially identical sequence variation may be generated by methods known in the art (Rizo and Gierasch, 1992, Ann. Rev. Biochem. 61: 387, incorporated herein by reference); for example, by adding internal cysteine residues capable of forming intramolecular disulfide bridges which cyclize the peptide.

The amino acid sequences of TRADE polypeptides identified herein will enable those of skill in the art to produce polypeptides corresponding to TRADE peptide sequences and sequence variants thereof. Such polypeptides may be produced in prokaryotic or eukaryotic host cells by expression of polynucleotides encoding a TRADE peptide sequence, frequently as part of a larger polypeptide. Alternatively, such peptides may be synthesized by chemical methods. Methods for expression of heterologous proteins in recombinant hosts, chemical synthesis of polypeptides, and in vitro translation are well known in the art and are described further in Maniatis et al., Molecular Cloning: A Laboratory Manual (1989), 2nd Ed., Cold Spring Harbor, N.Y.; Berger and Kimmel, Methods in Enzymology, Volume 152, Guide to Molecular Cloning Techniques (1987), Academic Press, Inc., San Diego, Calif.; Merrifield, J., 1969, J. Am. Chem. Soc. 91: 501; Chaiken I. M. ,1981, CRC Crit. Rev. Biochem. 11: 255; Kaiser et al., 1989, Science 243: 187; Merrifield, B., 1986, Science 232: 342; Kent, S. B. H., 1988, Ann. Rev. Biochem. 57: 957; and Offord, R. E., 1980, Semisynthetic Proteins, Wiley Publishing.

Peptides can be produced, typically by direct chemical synthesis, and used e.g., as agonists or antagonists of a TRADE/TRADE binding protein interaction. Peptides can be produced as modified peptides, with nonpeptide moieties attached by covalent linkage to the N-terminus and/or C-terminus. In certain preferred embodiments, either the carboxy-terminus or the amino-terminus, or both, are chemically modified. The most common modifications of the terminal amino and carboxyl groups are acetylation and amidation, respectively. Amino-terminal modifications such as acylation (e.g., acetylation) or alkylation (e.g., methylation) and carboxy-terminal-modifications such as amidation, as well as other terminal modifications, including cyclization, may be incorporated into various embodiments of the invention. Certain amino-terminal and/or carboxy-terminal modifications and/or peptide extensions to the core sequence can provide advantageous physical, chemical, biochemical, and pharmacological properties, such as: enhanced stability, increased potency and/or efficacy, resistance to serum proteases, desirable pharmacokinetic properties, and others. Peptides may be used therapeutically to treat disease, e.g., by altering the process of cell proliferation or apoptosis in a cell population of a patient

An isolated TRADE protein, or a portion or fragment thereof, can also be used as an immunogen to generate antibodies that bind TRADE using standard techniques for polyclonal and monoclonal antibody preparation. A full-length TRADE protein can be used or, alternatively, the invention provides antigenic peptide fragments of TRADE for use as immunogens. The antigenic peptide of TRADE comprises at least 8 amino acid residues and encompasses an epitope of TRADE such that an antibody raised against the peptide forms a specific immune complex with TRADE. Preferably, the antigenic peptide comprises at least 10 amino acid residues, more preferably at least 15 amino acid residues, even more preferably at least 20 amino acid residues, and most preferably at least 30 amino acid residues.

Alternatively, an antigenic peptide fragment of a TRADE polypeptide can be used as the immunogen. An antigenic peptide fragment of a TRADE polypeptide typically comprises at least 8 amino acid residues of the amino acid sequence shown in SEQ ID NO: 2 or 4 or an amino acid sequence of another TRADE family polypeptide and encompasses an epitope of a TRADE polypeptide such that an antibody raised against the peptide forms an immune complex with a TRADE molecule. Preferred epitopes encompassed by the antigenic peptide are regions of TRADE that are located on the surface of the protein, e.g., hydrophilic regions. In one embodiment, an antibody binds substantially specifically to a TRADE molecule. In another embodiment, an antibody binds specifically to a TRADE polypeptide.

Preferably, the antigenic peptide comprises at least about 10 amino acid residues, more preferably at least about 15 amino acid residues, even more preferably at least 20 about amino acid residues, and most preferably at least about 30 amino acid residues. Preferred epitopes encompassed by the antigenic peptide are regions of a TRADE polypeptide that are located on the surface of the protein, *e.g.*, hydrophilic regions, and that are unique to a TRADE polypeptide. In one embodiment such epitopes can be specific for a TRADE proteins from one species, such as mouse or human (i.e., an antigenic peptide that spans a region of a TRADE polypeptide that is not conserved across species is used as immunogen; such non conserved residues can be determined using an alignment such as that provided herein). A standard hydrophobicity analysis of the protein can be performed to identify hydrophilic regions.

A TRADE immunogen typically is used to prepare antibodies by immunizing a suitable subject, (*e.g*., rabbit, goat, mouse or other mammal) with the immunogen. An appropriate immunogenic preparation can contain, for example, a recombinantly expressed TRADE protein or a chemically synthesized TRADE peptide. The preparation can further include an adjuvant, such as Freund's complete or incomplete adjuvant, or similar immunostimulatory agent. Immunization of a suitable subject with an immunogenic TRADE preparation induces a polyclonal anti- TRADE antibody response.

Accordingly, another aspect of the invention pertains to the use of anti- TRADE family polypeptide antibodies. Such antibodies can be used as agonists and/or antagonists of TRADE family polypeptides. In a prefered embodiment antibodies specifically recognize TRADEα or β and not another TRADE family polypeptide. Polyclonal anti-TRADE antibodies can be prepared as described above by immunizing a suitable subject with a TRADE immunogen. The anti-TRADE antibody titer in the immunized subject can be monitored over time by standard techniques, such as with an enzyme linked immunosorbent assay (ELISA) using immobilized a TRADE polypeptide. If desired, the antibody molecules directed against a TRADE polypeptide can be isolated from the mammal (*e.g.*, from the blood) and further purified by well known techniques, such as protein A chromatography to obtain the IgG fraction. At an appropriate time after immunization, *e.g*., when the anti- TRADE antibody titers are highest, antibody-producing cells can be obtained from the subject and used to prepare monoclonal antibodies by standard techniques, such as the hybridoma technique originally described by Kohler and Milstein (Kohler and Milstein, 1975, Nature 256:495-497) (see also, Brown et al., 1981, J. Immunol 127:539-46; Brown et al., 1980, J Biol Chem 255:4980-83; Yeh et al., 1976, Proc. Natl. Acad Sci USA 76:2927-31; and Yeh et al., 1982, Int. J. Cancer 29:269-75), the more recent human B cell hybridoma technique (Kozbor et al., 1983, Immunol Today 4:72), the EBV-hybridoma technique (Cole et al., 1985, Monoclonal Antibodies and Cancer Therapy, Alan R. Liss, Inc., pp. 77-96) or trioma techniques. The technology for producing monoclonal antibody hybridomas is well known (see generally R. H. Kenneth, in Monoclonal Antibodies: A New Dimension In Biological Analyses, Plenum Publishing Corp., New York, New York (1980); E. A. Lerner, 1981, Yale J Biol. Med., 54:387-402; M. L. Gefter et al., 1977, Somatic Cell Genet., 3:231-36). Briefly, an immortal cell line (typically a myeloma) is fused to lymphocytes (typically splenocytes) from a mammal immunized with a TRADE immunogen as described above, and the culture supernatants of the resulting hybridoma cells are screened to identify a hybridoma producing a monoclonal antibody that binds specifically to a TRADE polypeptide.

Any of the many well known protocols used for fusing lymphocytes and immortalized cell lines can be applied for the purpose of generating an anti- TRADE monoclonal antibody (see, *e.g.,* G. Galfre et al., 1977, Nature 266:55052; Gefter *et al. Somatic Cell Genet.,* cited *supra;* Lerner, *Yale J. Biol. Med.,* cited *supra;* Kenneth, *Monoclonal Antibodies,* cited *supra*). Moreover, the ordinary skilled worker will appreciate that there are many variations of such methods which also would be useful. Typically, the immortal cell line (*e.g.*, a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines may be used as a fusion partner according to standard techniques, *e.g.*, the P3-NS1/1-Ag4-1, P3-x63-Ag8.653 or Sp2/O-Ag14 myeloma lines. These myeloma lines are available from the American Type Culture Collection (ATCC), Rockville, Md. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind a TRADE molecule, *e.g.*, using a standard ELISA assay.

As an alternative to preparing monoclonal antibody-secreting hybridomas, a monoclonal anti-TRADE antibody can be identified and isolated by screening a recombinant combinatorial immunoglobulin library (*e.g.*, an antibody phage display library) with a TRADE to thereby isolate immunoglobulin library members that bind a TRADE polypeptide. Kits for generating and screening phage display libraries are commercially available (*e.g.*, the Pharmacia *Recombinant Phage Antibody System,* Catalog No. 27-9400-01; and the Stratagene *SurfZAP*^{™} *Phage Display Kit,* Catalog No. 240612). Additionally, examples of methods and reagents particularly amenable for use in generating and screening antibody display library can be found in, for example, Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al., 1991, Bio/Technology 9:1370-1372; Hay et al., 1992, Hum Antibod Hybridomas 3:81-85; Huse et al., 1989, Science 246:1275-1281; Griffiths et al., 1993, EMBO J 12:725-734; Hawkins et al., 1992, J Mol Biol 226:889-896; Clarkson et al., 1991, Nature 352:624-628; Gram et al., 1992, Proc. Natl. Acad. Sci USA 89:3576-3580; Garrad et al., 1991, Bio/Technology 9:1373-1377; Hoogenboom et al., 1991, Nuc Acid Res 19:4133-4137; Barbas et al., 1991, Proc. Natl. Acad. Sci USA 88:7978-7982; and McCafferty et al., 1990, Nature 348:552-554.

Additionally, recombinant anti- TRADE antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in Robinson et al. International Patent Publication PCTIUS86/02269; Akira, et al. European Patent Application 184,187; Taniguchi, M., European Patent Application 171,496; Morrison et al. European Patent Application 173,494; Neuberger et al. PCT Application WO 86/01533; Cabilly et al. U.S. Patent No. 4,816,567; Cabilly et al. European Patent Application 125,023; Better et al., 1988, Science 240:1041-1043; Liu et al., 1987, Proc. Natl. Acad. Sci USA 84:3439-3443; Liu et al., 1987, J. Immunol. 139:3521-3526; Sun et al., 1987, Proc. Natl. Acad. Sci USA 84:214-218; Nishimura et al., 1987, Canc. Res. 47:999-1005; Wood et al., 1985, Nature 314:446-449; and Shaw et al., 1988, J. Natl Cancer Inst. 80:1553-1559); Morrison, S. L., 1985, Science 229:1202-1207; Oi et al., 1986, BioTechniques 4:214; Winter U.S. Patent 5,225,539; Jones et al., 1986, Nature 321:552-525; Verhoeyan et al., 1988, Science 239:1534; and Beidler et al., 1988, J. Immunol. 141:4053-4060.

In addition, humanized antibodies can be made according to standard protocols such as those disclosed in US patent 5,565,332. In another embodiment, antibody chains or specific binding pair members can be produced by recombination between vectors comprising nucleic acid molecules encoding a fusion of a polypeptide chain of a specific binding pair member and a component of a replicable generic display package and vectors containing nucleic acid molecules encoding a second polypeptide chain of a single binding pair member using techniques known in the art, e.g., as described in US patents 5,565,332, 5,871,907, or 5,733,743.

An anti- TRADE antibody (*e.g.*, monoclonal antibody) can be used to isolate a TRADE polypeptide by standard techniques, such as affinity chromatography or immunoprecipitation. Anti- TRADE antibodies can facilitate the purification of natural TRADE polypeptides from cells and of recombinantly produced TRADE polypeptides expressed in host cells. Moreover, an anti- TRADE antibody can be used to detect a TRADE protein (*e.g.*, in a cellular lysate or cell supematant). Detection may be facilitated by coupling (*i.e.,* physically linking) the antibody to a detectable substance. Accordingly, in one embodiment, an anti- TRADE antibody of the invention is labeled with a detectable substance. Examples of detectable substances include various enzymes, prosthetic groups, fluorescent materials, luminescent materials and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of suitable radioactive material include ¹²⁵I, ¹³¹I, ³⁵S or ³H.

Accordingly, in one embodiment, anti-TRADE antibodies can be used, e.g., intracellularly to inhibit protein activity. The use of intracellular antibodies to inhibit protein function in a cell is known in the art (see *e.g.,* Carlson, J. R., 1988, Mol. Cell. Biol. 8:2638-2646; Biocca, S. et al., 1990, EMBO J. 9:101-108; Werge, T.M. et al., 1990, FEBS Letters 274:193-198; Carlson, J.R., 1993, Proc. Natl. Acad. Sci. USA 90:7427-7428; Marasco, W.A. et al., 1993, Proc. Natl. Acad. Sci. USA 90:7889-7893; Biocca, S. et al., 1994, Bio/Technology 12:396-399; Chen, S-Y. et al., 1994, Human Gene Therapy 5:595-601; Duan, L et al., 1994, Proc. Natl. Acad Sci. USA 91:5075-5079; Chen, S-Y. et al., 1994, Proc. Natl. Acad Sci. USA 91:5932-5936; Beerli, R.R. et al., 1994, J. Biol. Chem. 269:23931-23936; Beerli, R.R. et al., 1994, Biochem. Biophys. Res. Commun. 204:666-672; Mhashilkar, A.M. et al., 1995, EMBO J. 14:1542-1551; Richardson, J.H. et al., 1995, Proc. Natl. Acad. Sci. USA 92:3137-3141; PCT Publication No. WO 94/02610 by Marasco et al.*;* and PCT Publication No. WO 95/03832 by Duan et al*.*).

In one embodiment, a recombinant expression vector is prepared which encodes the antibody chains in a form such that, upon introduction of the vector into a cell, the antibody chains are expressed as a functional antibody in an intracellular compartment of the cell. For inhibition of TRADE activity according to the inhibitory methods of the invention, an intracellular antibody that specifically binds the TRADE protein is expressed in the cytoplasm of the cell. To prepare an intracellular antibody expression vector, antibody light and heavy chain cDNAs encoding antibody chains specific for the target protein of interest, *e.g.*, TRADE, are isolated, typically from a hybridoma that secretes a monoclonal antibody specific for the TRADE protein. Hybridomas secreting anti- TRADE monoclonal antibodies, or recombinant anti- TRADE monoclonal antibodies, can be prepared as described above. Once a monoclonal antibody specific for TRADE protein has been identified (*e.g.,* either a hybridoma-derived monoclonal antibody or a recombinant antibody from a combinatorial library), DNAs encoding the light and heavy chains of the monoclonal antibody are isolated by standard molecular biology techniques. For hybridoma derived antibodies, light and heavy chain cDNAs can be obtained, for example, by PCR amplification or cDNA library screening. For recombinant antibodies, such as from a phage display library, cDNA encoding the light and heavy chains can be recovered from the display package (*e.g*., phage) isolated during the library screening process. Nucleotide sequences of antibody light and heavy chain genes from which PCR primers or cDNA library probes can be prepared are known in the art. For example, many such sequences are disclosed in Kabat, E.A., et al., 1991, Sequences of Proteins of Immunological Interest, Fifth Editi*on,* U.S. Department of Health and Human Services, NIH Publication No. 91-3242 and in the "Vbase" human germline sequence database.

Once obtained, the antibody light and heavy chain sequences are cloned into a recombinant expression vector using standard methods. To allow for cytoplasmic expression of the light and heavy chains, the nucleotide sequences encoding the hydrophobic leaders of the light and heavy chains are removed. An intracellular antibody expression vector can encode an intracellular antibody in one of several different forms. For example, in one embodiment, the vector encodes full-length antibody light and heavy chains such that a full-length antibody is expressed intracellularly. In another embodiment, the vector encodes a full-length light chain but only the VH/CH1 region of the heavy chain such that a Fab fragment is expressed intracellularly. In the most preferred embodiment, the vector encodes a single chain antibody (scFv) wherein the variable regions of the light and heavy chains are linked by a flexible peptide linker (*e.g*., (Gly₄Ser)₃) and expressed as a single chain molecule. To inhibit TRADE activity in a cell, the expression vector encoding the anti- TRADE intracellular antibody is introduced into the cell by standard transfection methods, as discussed herein.

An antibody or antibody portion of the invention can be derivatized or linked to another functional molecule (e.g., a peptide or polypeptide). Accordingly, the antibodies and antibody portions of the invention are intended to include derivatized and otherwise modified forms of the anti-TRADE antibodies described herein, including, e.g., antibodies conjugated to other molecules (e.g., antibodies or polypeptides which bind to other cell markers). For example, an antibody or antibody portion of the invention can be functionally linked (by chemical coupling, genetic fusion, noncovalent association or otherwise) to one or more other molecular entities, such as another antibody (*e.g.,* to create a bispecific antibody or a diabody), a detectable agent, a cytotoxic agent, a pharmaceutical agent, and/or a protein or peptide that can mediate associate of the antibody or antibody portion with another molecule (such as a streptavidin core region or a polyhistidine tag).

One type of derivatized antibody is produced by crosslinking two or more antibodies (of the same type or of different types, *e.g*., to create bispecific antibodies). Suitable crosslinkers include those that are heterobifunctional, having two distinctly reactive groups separated by an appropriate spacer (*e.g.*, m-maleimidobenzoyl-N-hydroxysuccinimide ester) or homobifunctional (*e.g.*, disuccinimidyl suberate). Such linkers are available from Pierce Chemical Company, Rockford, IL.

Useful detectable agents with which an antibody or antibody portion of the invention may be derivatized include fluorescent compounds. Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a colored reaction product, which is detectable. An antibody may also be derivatized with biotin, and detected through indirect measurement of avidin or streptavidin binding.

In one embodiment, anti-TRADE antibodies can be used to target cells expressing TRADE molecules. For example, an antibody can be used which recognizes a TRADE family molecules or which specifically recognizes a single TRADE family molecule and not another TRADE family molecule, e.g., an antibody which recognizes TRADEβ. In one embodiment, such an antibody-toxin conjugate comprising the antibody and a toxin molecule can be used to deplete cells bearing a TRADE family or a specific TRADE molecule (e.g., by ablation). In a preferred embodiment, an anti-TRADE immunotoxin is used to target a tumor cell, e.g., *in vivo* or *ex vivo.* As used herein, the term "toxin" is meant to include molecules that are toxic to cells, *e.g.* chemotherapeutic agents and bacterial toxins.

A wide variety of toxins are known in the art and may be conjugated to the antibodies of the invention (see Hertler and Frankel, 1989, J Clin Oncol. 7:1932-1942). For example, toxins may disrupt the cell membrane without internalization, toxins may be internalized via a non-specific mechanism, or toxins may be specifically internalized, *e.g.*, by direct interaction with specific receptor proteins on the cell. Toxins for use in the claimed invention can be e.g., naturally occurring or synthetic. Toxins may be proteinaceous or non-proteinanceous, *e.g.*, oligosaccharides. Examples include: numerous useful plant-, fungus- or even bacteria-derived toxins, which, by way of example, include various A chain toxins, particularly ricin A chain, ribosome inactivating proteins such as saporin or gelonin, .alpha.-sarcin, aspergillin, restrictocin, ribonucleases such as placental ribonuclease, angiogenic, diphtheria toxin, and pseudomonas exotoxin, and calicheamicin and will be discussed in more detail below.

For example, in one embodiment, exemplary toxins include "ribosome inactivating proteins" (RIPs) which by definition are able to directly inhibit the ribosomal translational machinery. The heterodimer peptide ricin is derived from the castor bean plant (*Ricinus communis*) and is an example of such a toxin. The toxic activity of ricin is found entirely in one of its subunits (ricin A-chain). In one embodiment, a toxin for use in the claimed invention is an active subunit of a toxin molecule. Ricin A-chain is thought to deactivate ribosome function by specifically depurinating the single adenine at position 4324 of 28S rRNA (Chen et al., 1998, Biochemistry 37:11605, Koehler et al., 1994, Bone Marrow Transplant 13:571-575; Duke-Cohan et al., 1993, Blood, 82:2224-34). Another bipartite RIP toxin is abrin, which is derived from the jequirity bean (*Abrus precatorius*) and is known to deactivate protein translation by the same mechanism as ricin-A (Krupakar et al., 1999, Biochem. Journal 338:273-279). Other RIPs which can be used in connection with the invention include the plant cytotoxins saporin and gelonin. The Shiga-A toxin from the microorganism *Shigella dysenteriae* also functions as an RIP (Fraser, M. E., 1994, Nature Structural Biology 1:59-64), as does the sarcin-A toxin, derived from the mold *Aspergillus giganteus* (Lacadena et al., 1999, Proteins, 37:474-484). Antibody-toxin conjugates which include ricin-A and similar toxins have been described previously in U.S. Patent Nos. 4,590,017,4,906,469,4,919,927, and 5,980,896.

Toxins which ADP-ribosylate the protein elongation factor 2 (EF-2), e.g., bacterial diptheria toxin (from *Corynebacterium diphtheriae*) and inhibit protein synthesis (Foley et al., 1995, J Biol Chem, 270:23218-23225) can also be used in the antibody-toxin conjugates of the invention. Antibody-toxin conjugates which include diptheria toxin or related toxins which ADP-ribosylate the EF-2 have been described previously, e.g., in U.S. Patent Nos. 4,545,985.

Other potent toxins are able to able to bring about eukaryotic cell death by interfering with microtubule function, thus causing mitotic arrest (Iwasaki, 1998, Yakugaku Zasshi 118:112-126). Examples of such toxins are the maytansinoid compounds (Takahashi et al., 1989, Mol. Gen. Genet. 220:53-59) which are found in certain mosses (e.g. *maytenus buchananii;* see Larson et al., 1999, J. of Nat. Prod. 62:361-363). Antibody-toxin conjugates which include maytansinoid have been described previously in U.S. Patent No. 5,208,020.

Still other toxins are able to activate the adenylate cyclase cAMP system, causing unregulated transport of anions and cations through the membrane. An example of this type of toxin is the cholera toxin (de Haan et al., 1998 Immunol Cell Biol, 76:270-279) derived from *Vibrio cholerae,* a microorganism that can cause fluid secretion and hemorrhage of intestinal cells.

The bacterial pertussis toxin (derived from *Bordetella pertussis*) is able to specifically target the eukaryotic G protein complex, a key element in the transduction of many extracellular signal pathways, including those triggered by cytokine and hormone receptors. The pertussis toxin can ADP-ribosylate a subunit of the G protein complex, causing an uncoupling of its regulatory activity (Locht and Antoine, 1995, Biochimie, 77:333-340).

In one embodiment, a toxin for use in the antibody-toxin conjugates of the invention is an oligosaccharide. For example, the oligosaccharide calicheamicin is a bacterial product which was identified as one of a class of carbohydrates which preferentially bind the minor groove of DNA (Kahne, 1995, Chem Biol, 2:7-12). Calicheamicin is known to non-specifically abstract the hydrogen atom from the 4'carbon of DNA deoxyribose groups causing double stranded DNA breaks with terminal 3'-phosphoglycolate groups which are refractory to normal cellular repair mechanisms (Chaudhry et al., 1999, Biochem Pharmacol, 57:531-538). Calicheamicin is a preferred toxin moiety for use in connection with the invention. Antibody calicheamicin conjugates have been described (Sievers et al., 1999, Blood, 93:3678-3684; Lode et al., 1998, Cancer Research, 58:2925-2928). Other synthetic cytotoxic compounds, such as CC-1065, have similar DNA-fragmenting mechanisms as calicheamicin and are also known in the art (Gunz and Naegeli, 1996, Biochem. Pharmacol, 52:447-453). Antibody-toxin conjugates, in which calicheamicin is covalently attached to an antibody through disulfide bonds, have been described previously in U.S. Patent Nos. 5,773,001 and 5,739,116.

Another exemplary class of toxins are bacterial toxins which are able to form lethal holes in eukaryotic membranes, thus causing cell death without the need for endocytotic internalization. Aerolysin is one example of such a toxin. Aerolysin can form hepatomer channels through:a membrane upon binding to a cell surface (Parker et al., 1996 Mol Microbiol 19:205-212; Buckley, 1991, Experimentia 47:418-419). Molecular conjugates which include aerolysin have been described previously in U.S. Patent No. 5,824,776 and 5,817,771.

There are numerous methods known in the art for conjugating a toxin to an antibody such that the activity of the toxin is appropriately delivered upon binding of the antibody to a cell (Ghose and Blair, 1987, Crit Rev Ther Drug Carrier Syst, 3:263-359; Hermentin and Seiler, 1988, Behring Inst. Mitt., 82:197-215:). For example, when the cytotoxic agent is a protein and the second component is an intact immunoglobulin, the linkage may be by way of heterobifunctional cross-linkers, e.g., SPDP, carbodiimide, glutaraldehyde, or the like. Production of various immunotoxins is well-known with the art, and can be found, for example in "Monoclonal Antibody-Toxin Conjugates: Aiming the Magic Bullet," Thorpe et al., Monoclonal Antibodies in Clinical Medicine, Academic Press, pp. 168-190 (1982), which is incorporated herein by reference. The components may also be linked genetically (see, Chaudhary et al., 1989, Nature 339:394).

For example, in one embodiment, a covalent linkage can be formed between the antibody and the toxin. In some cases, the existing cell-binding portion of a toxin must first be removed or altered to suppress its non-specific activity (Hertler and Frankel, 1989, J. Clin Oncol 7:1932-1942). The covalent linkage of antibody to toxin generally involves formation of a thioester or a disulfide bond. For example, conjugate compounds can be prepared by using N-succinimidyl-3-2(pyridyldithio)propionate, which can generate a disulfide linkage between an antibody and a toxin (Colombatti et al., 1983, J Immunology, 131:3091-3095). Numerous types of disulfide-bond containing linkers are known which can successfully be employed to conjugate the toxin moiety with a polypeptide. In one embodiment, linkers that contain a disulfide bond that is sterically "hindered" are preferred, due to their greater stability in vivo, thus preventing release of the toxin moiety prior to binding at the site of action. Other methods forming covalent linkages between have been described in U.S. Patent Nos. 4,894,443, 5,208,021, 4,340,535, and EP 44167.

Another aspect of this invention pertains to the identification of new TRADE modulators. Many techniques are known in the art and can be utilized to identify new modulators. For example, mobility shift DNA-binding assays that utilize gel electrophoresis is a simple, rapid, and extremely sensitive method for the detection of sequence-specific DNA-binding proteins in crude extracts. This assay also allows for the quantitative determination of the affinity, abundance, association rate constants, dissociation rate constants, and binding specificity of DNA-binding proteins. Proteins that bind specifically to an end-labeled DNA fragment retard the mobility of the fragment during electrophoresis, resulting in discrete bands corresponding to the individual protein-DNA complex. Briefly, an end-labeled DNA probe from the invention containing a particular protein binding site is bound with a protein mixture and then separated by SDS-PAGE, which is then dried and autoradiographed.

### IV. Recombinant Expression Vectors and Host Cells

Another aspect of the invention pertains to vectors, preferably expression vectors, containing a nucleic acid encoding a TRADE protein (or a portion thereof). The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell). The term "regulatory sequence" is intended to includes promoters, enhancers and other expression control elements (e.g., polyadenylation signals). Such regulatory sequences are described, for example, in Goeddel; Gene Expression Technology: Methods in Enzymology 185, Academic Press, San Diego, CA (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, and the like. The expression vectors of the invention can be introduced into host cells to thereby produce proteins or peptides, including fusion proteins or peptides, encoded by nucleic acids as described herein (e.g., TRADE proteins, mutant forms of TRADE proteins, fusion proteins, and the like).

The recombinant expression vectors of the invention can be designed for expression of TRADE proteins or protein fragments in prokaryotic or eukaryotic cells. For example, TRADE proteins can be expressed in bacterial cells such as *E. coli,* insect cells (using baculovirus expression vectors) yeast cells or mammalian cells. Suitable host cells are discussed further in Goeddel, 1990, Gene Expression Technology: Methods in Enzymology 185, Academic Press, San Diego, CA. Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S., 1988, Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be utilized in TRADE activity assays, (e.g., direct assays or competitive assays described in detail below), or to generate antibodies specific for TRADE proteins, for example.

Examples of suitable inducible non-fusion *E. coli* expression vectors include pTrc (Amann et al., 1988, Gene 69:301-315) and pET 11d (Studier et al., 1990, Gene Expression Technology: Methods in Enzymology 185, Academic Press, San Diego, CA, 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gn1). This viral polymerase is supplied by host strains BL21(DE3) or HMS174(DE3) from a resident prophage harboring a T7 gn1 gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, S., 1990, Gene Expression Technology: Methods in Enzymology 185, Academic Press, San Diego, CA, 119-128). Another strategy is to alter the nucleic acid sequence of the nucleic acid to be inserted into an expression vector so that the individual codons for each amino acid are those preferentially utilized in *E. coli* (Wada et al., 1992, Nucleic Acids Res. 20:2111-2118). Such alteration of nucleic acid sequences of the invention can be carried out by standard DNA synthesis techniques.

In another embodiment, the TRADE expression vector is a yeast expression vector. Examples of vectors for expression in yeast *S. cerevisiae* include pYepSecl (Baldari et al., 1987, Embo J. 6:229-234), pMFa (Kurjan and Herskowitz, 1982, Cell 30:933-943), pJRY88 (Schultz et al., 1987, Gene 54:113-123), pYES2 (Invitrogen Corporation, San Diego, CA), and picZ (In Vitrogen Corp, San Diego, CA).

Alternatively, TRADE proteins can be expressed in insect cells using baculovirus expression vectors. Baculovirus vectors available for expression of proteins in cultured insect cells (e.g., Sf 9 cells) include the pAc series (Smith et al., 1983, Mol. Cell Biol. 3:2156-2165) and the pVL series (Lucklow and Summers, 1989, Virology 170:31-39).

In yet another embodiment, a nucleic acid of the invention is expressed in mammalian cells using a mammalian expression vector. Examples of mammalian expression vectors include pCDM8 (Seed, B., 1987, Nature 329:840) and pMT2PC (Kaufman et al., 1987, EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert et al., 1987, Genes Dev. 1:268-277), lymphoid-specific promoters (Calame and Eaton, 1988, Adv. Immunol. 43:235-275), in particular promoters of T cell receptors (Winoto and Baltimore, 1989, EMBO J. 8:729-733) and immunoglobulins (Banerji et al., 1983, Cell 33:729-740; Queen and Baltimore, 1983, Cell 33:741-748), neuron-specific promoters (e.g., the neurofilament promoter; Byrne and Ruddle, 1989, Proc. Natl. Acad. Sci. USA 86:5473-5477), pancreas-specific promoters (Edlund et al., 1985, Science 230:912-916), and mammary gland-specific promoters (e.g., milk whey promoter; U.S. Patent No. 4,873,316 and European Application Publication No. 264,166). Developmentally-regulated promoters are also encompassed, for example the murine hox promoters (Kessel and Gruss, 1990, Science 249:374-379) and the α-fetoprotein promoter (Campes and Tilghman, 1989, Genes Dev. 3:537-546).

Alternatively, a TRADE polypeptide can be expressed in insect cells using baculovirus expression vectors. Baculovirus vectors available for expression of proteins in cultured insect cells *(e.g.,* Sf 9 cells) include the pAc series (Smith et al., 1983, Mol. Cell Biol. 3:2156-2165) and the pVL series (Lucklow, V.A., and Summers, M.D.; 1989, Virology 170:31-39).

In yet another embodiment, a nucleic acid molecule of the invention is expressed in mammalian cells using a mammalian expression vector. Examples of mammalian expression vectors include pMex-NeoI, pCDM8 (Seed, B., 1987, Nature 329:840) and pMT2PC (Kaufman et al., 1987, EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40.

Moreover, inducible regulatory systems for use in mammalian cells are known in the art, for example systems in which gene expression is regulated by heavy metal ions (see *e.g.,* Mayo et al., 1982, Cell 29:99-108; Brinster et al., 1982, Nature 296:39-42; Searle et al., 1985, Mol. Cell. Biol. 5:1480-1489), heat shock (see *e.g*., Nouer et al., 1991, in Heat Shock Response, e.d. Nouer, L. , CRC, Boca Raton , FL, pp167-220), hormones (see *e.g.,* Lee et al., 1981, Nature 294:228-232; Hynes et al., 1981, Proc. Natl. Acad Sci. USA 78:2038-2042; Klock et al., 1987, Nature 329:734-736; Israel & Kaufman, 1989, Nucl. Acids Res. 17:2589-2604; and PCT Publication No. WO 93/23431), FK506-related molecules (see *e.g.,* PCT Publication No. WO 94/18317) or tetracyclines (Gossen, M. and Bujard, H., 1992, Proc. Natl. Acad Sci. USA 89:5547-5551; Gossen, M. et al., 1995, Science 268:1766-1769; PCT Publication No. WO 94/29442; and PCT Publication No. WO 96/01313). Accordingly, in another embodiment, the invention provides a recombinant expression vector in which a TRADE DNA is operatively linked to an inducible eukaryotic promoter, thereby allowing for inducible expression of a TRADE protein in eukaryotic cells.

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to TRADE mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, H. et al., Antisense RNA as a molecular tool for genetic analysis, Reviews - Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a TRADE protein can be expressed in bacterial cells such as *E*. *coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (Molecular Cloning: A Laboratory Manual. 2nd, ed., Cold Spring Harbor Laboratory, Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding a TRADE protein or can be introduced on a separate vector. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce (i.e., express) a TRADE protein. Accordingly, the invention further provides methods for producing a TRADE protein using the host cells of the invention. In one embodiment, the method comprises culturing the host cell of invention (into which a recombinant expression vector encoding a TRADE protein has been introduced) in a suitable medium such that a TRADE protein is produced. In another embodiment, the method further comprises isolating a TRADE protein from the medium or the host cell.

Certain host cells of the invention can also be used to produce non-human transgenic animals. For example, in one embodiment, a host cell of the invention is a fertilized oocyte or an embryonic stem cell into which TRADE-coding sequences have been introduced. Such host cells can then be used to create non-human transgenic animals in which exogenous TRADE sequences have been introduced into their genome or homologous recombinant animals in which endogenous TRADE sequences have been altered. Such animals are useful for studying the function and/or activity of a TRADE polypeptide and for identifying and/or evaluating modulators of TRADE activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, and the like. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous TRADE gene has been altered by homologous recombination between the endogenous gene and an exogenous DNA molecule introduced into a cell of the animal, e.g., an embryonic cell of the animal, prior to development of the animal.

A transgenic animal of the invention can be created by introducing a TRADE-encoding nucleic acid into the male pronucleus of a fertilized oocyte, e.g., by microinjection, retroviral infection, and allowing the oocyte to develop in a pseudopregnant female foster animal. The TRADE sequence of SEQ ID NO:1 or 3 a nucleic acid molecule encoding another TRADE family polypeptide or portion thereof can be introduced as a transgene into the genome of a non-human animal. Alternatively, a nonhuman homologue of a TRADE gene, such as a mouse or rat TRADE gene, can be used as a transgene. Alternatively, a TRADE gene homologue, such as another TRADE family member, can be isolated based on hybridization to the TRADE family cDNA sequences of SEQ ID NO:1 or 3 or a nucleotide sequence encoding another TRADE family polypeptide and used as a transgene. Intronic sequences and polyadenylation signals can also be included in the transgene to increase the efficiency of expression of the transgene. A tissue-specific regulatory sequence(s) can be operably linked to a TRADE transgene to direct expression of a TRADE protein to particular cells. Methods for generating transgenic animals via embryo manipulation and microinjection, particularly animals such as mice, have become conventional in the art and are described, for example, in U.S. Patent Nos. 4,736,866 and 4,870,009, both by Leder et al., U.S. Patent No. 4,873,191 by Wagner et al*.* and in Hogan, B., Manipulating the Mouse Embryo, (Cold Spring Harbor Laboratory Press, Cold Spring Harbor, N.Y., 1986). Similar methods are used for production of other transgenic animals. A transgenic founder animal can be identified based upon the presence of a TRADE transgene in its genome and/or expression of TRADE mRNA in tissues or cells of the animals. A transgenic founder animal can then be used to breed additional animals carrying the transgene. Moreover, transgenic animals carrying a transgene encoding a TRADE protein can further be bred to other transgenic animals carrying other transgenes.

To create a homologous recombinant animal, a vector is prepared which contains at least a portion of a TRADE gene into which a deletion, addition or substitution has been introduced to thereby alter, e.g., functionally disrupt, the TRADE gene. For example, a mouse TRADE gene can be used to construct a homologous recombination vector suitable for altering an endogenous TRADE gene in the mouse genome. In a preferred embodiment, the vector is designed such that, upon homologous recombination, the endogenous TRADE gene is functionally disrupted (i.e., no longer encodes a functional protein; also referred to as a "knock out" vector). Alternatively, the vector can be designed such that, upon homologous recombination, the endogenous TRADE gene is mutated or otherwise altered but still encodes a functional protein (e.g., the upstream regulatory region can be altered to thereby alter the expression of the endogenous TRADE protein). In the homologous recombination vector, the altered portion of the TRADE gene is flanked at its 5' and 3' ends by additional nucleic acid sequence of the TRADE gene to allow for homologous recombination to occur between the exogenous TRADE gene carried by the vector and an endogenous TRADE gene in an embryonic stem cell. The additional flanking TRADE nucleic acid sequence is of sufficient length for successful homologous recombination with the endogenous gene. Typically, several kilobases of flanking DNA (both at the 5' and 3' ends) are included in the vector (see e.g., Thomas, K.R. and Capecchi, M. R., 1987, Cell 51:503 for a description of homologous recombination vectors). The vector is introduced into an embryonic stem cell line (e.g., by electroporation) and cells in which the introduced TRADE gene has homologously recombined with the endogenous TRADE gene are selected (see, e.g., Li, E. et al., 1992, Cell 69:915). The selected cells are then injected into a blastocyst of an animal (e.g., a mouse) to form aggregation chimeras (see e.g., Bradley, A. in Teratocarcinomas and Embryonic Stem Cells: A Practical Approach, E.J. Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley, A., 1991, Current Opinion in Biotechnology 2:823-829 and in PCT International Publication Nos.: WO 90/11354 by Le Mouellec et al*.;* WO 91/01140 by Smithies et al.*;* WO 92/0968 by Zijlstra et al*.;* and WO 93/04169 by Berns et al*.*

In addition to the foregoing, the skilled artisan will appreciate that other approaches known in the art for homologous recombination can be applied to the instant invention. Enzyme-assisted site-specific integration systems are known in the art and can be applied to integrate a DNA molecule at a predetermined location in a second target DNA molecule. Examples of such enzyme-assisted integration systems include the Cre recombinase-lox target system (*e.g.*, as described in Baubonis, W. and Sauer, B., 1993, Nucl. Acids Res. 21:2025-2029; and Fukushige, S. and Sauer, B., 1992, Proc. Natl. Acad. Sci. USA 89:7905-7909) and the FLP recombinase-FRT target system (*e.g.,* as described in Dang, D.T. and Perrimon, N., 1992, Dev. Genet. 13:367-375; and Fiering, S. et al., 1993, Proc. Natl. Acad Sci. USA 90:8469-8473). Tetracycline-regulated inducible homologous recombination systems, such as described in PCT Publication No. WO 94/29442 and PCT Publication No. WO 96/01313, also can be used.

For example, in another embodiment, transgenic non-humans animals can be produced which contain selected systems which allow for regulated expression of the transgene. One example of such a system is the *cre*/*loxP* recombinase system of bacteriophage P1. For a description of the *cre*/*loxP* recombinase system, see, e.g., Lakso et al., 1992, Proc. Natl. Acad. Sci. USA 89:6232-6236. Another example of a recombinase system is the FLP recombinase system of *Saccharomyces cerevisiae* (O'Gorman et al., 1991, Science 251:1351-1355. If a *cre*/*loxP* recombinase system is used to regulate expression of the transgene, animals containing transgenes encoding both the *Cre* recombinase and a selected protein are required. Such animals can be provided through the construction of "double" transgenic animals, e.g., by mating two transgenic animals, one containing a transgene encoding a selected protein and the other containing a transgene encoding a recombinase.

Clones of the non-human transgenic animals described herein can also be produced according to the methods described in Wilmut, I. et al., 1997, Nature 385:810-813 and PCT International Publication Nos. WO 97/07668 and WO 97/07669. In brief, a cell, e.g., a somatic cell, from the transgenic animal can be isolated and induced to exit the proliferation cycle and enter Go phase. The quiescent cell can then be fused, e.g., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell, e.g., the somatic cell, is isolated.

### Identification of other TRADE modulating Agents

The invention provides a method (also referred to herein as a "screening assay") for identifying modulators, i.e., candidate or test compounds or agents (e.g., peptides, peptidomimetics, small molecules or other drugs) which are capable of modulating TRADE, e.g., bind to TRADE proteins, have a stimulatory or inhibitory effect on, for example, TRADE expression or TRADE activity. In addition, assays can be used to test the effect of a TRADE modulator on TRADE expression or activity (e.g., to determine whether apoptosis or expression is modulated in the desired direction in a cell-specific situation by using a screening assay such as those described herein.

The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, K.S., 1997, Anticancer Drug Des. 12:145).

Examples of methods for the synthesis of molecular libraries can be found in the art, for example in: DeWitt et al., 1993, Proc. Natl. Acad Sci. U.S.A. 90:6909; Erb et al., 1994, Proc. Natl. Acad Sci. USA 91:11422; Zuckermann et al., 1994, J. Med Chem. 37:2678; Cho et al., 1993, Science 261:1303; Carrell et al., 1994, Angew. Chem. Int. Ed Engl. 33:2059; Carell et al., 1994, Angew. Chem. Int. Ed Engl. 33:2061; and in Gallop et al., 1994, J. Med. Chem. 37:1233.

Libraries of compounds may be presented in solution (e.g., Houghten, 1992, Biotechniques 13:412-421), or on beads (Lam, 1991, Nature 354:82-84), chips (Fodor, 1993, Nature 364:555-556), bacteria (Ladner USP 5,223,409), spores (Ladner USP '409), plasmids (Cull et al., 1992, Proc Natl Acad Sci USA 89:1865-1869) or on phage (Scott and Smith, 1990; Science 249:386-390; Devlin, 1990, Science 249:404-406; Cwirla et al., 1990, Proc. Natl. Acad Sci. 87:6378-6382; Felici, 1991, J. Mol. Biol. 222:301-310; Ladner *supra.*).

In many drug screening programs which test libraries of modulating agents and natural extracts, high throughput assays are desirable in order to maximize the number of modulating agents surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test modulating agent. Moreover, the effects of cellular toxicity and/or bioavailability of the test modulating agent can be generally ignored in the *in vitro* system, the assay instead being focused primarily on the effect of the drug on the molecular target as may be manifest in an alteration of binding affinity with upstream or downstream elements.

Also described herein are assays for screening candidate or test compounds which bind to or modulate the activity of a TRADE protein or polypeptide or biologically active portion thereof, e.g., modulate the ability of TRADE polypeptide to activate an NFkB or JNK signaling pathway, *e.g.*, by binding to or affecting an active surface of the TRADE polypeptide which is involved in interactions with *e.g.*, a molecule in an NFkB or JNK signaling pathways such as a TRAF molecule or an associated kinase.

Assays can be used to screen for modulating agents, including TRADE homologs, which are either agonists or antagonists of the normal cellular function of the subject TRADE polypeptides. For example, the invention provides a method in which an indicator composition is provided which has a TRADE protein having a TRADE activity. The indicator composition can be contacted with a test compound. The effect of the test compound on TRADE activity, as measured by a change in the indicator composition, can then be determined to thereby identify a compound that modulates the activity of a TRADE protein. A statistically significant change, such as a decrease or increase, in the level of TRADE activity in the presence of the test compound (relative to what is detected in the absence of the test compound) is indicative of the test compound being a TRADE modulating agent. The indicator composition can be, for example, a cell or a cell extract. In one embodiment, TRADE activity is assessed as described in the appended Examples.

In an exemplary screening assay of the present invention, the modulating agent of interest is contacted with interactor molecules, *e.g.* proteins, which may Function upstream (including both activators and repressors of its activity) or to molecules which may function downstream of the TRADE protein, whether they are positively or negatively regulated by it. To the mixture of the modulating agent and the upstream or downstream element is then added a composition containing a TRADE protein. Detection and quantification of the interaction of TRADE with it's upstream or downstream elements provide a means for determining a modulating agent's efficacy at inhibiting (or potentiating) complex formation between TRADE and the TRADE binding elements. Exemplary interaction molecules include TRAF molecules, for example, TRAF3 (which binds to the intracellular domain, preferably to a region from amino acid 193 to 328) and TRAF6.

In another exemplary screening assay, deletion constructs of the present invention are used to determine key binding sites within the present invention and also for the identification of novel binding proteins to those binding sites. For example, constructs carrying deletions from (1) the C-terminal end to amino acid residue 328, (2) from the C-terminal end to amino acid 218, and (3) from the C-terminal end to amino acid 368 can be used. These constructs can be utilized in techniques such as mobility shift DNA-binding assays and yeast two-hybrid systems as previously described in the art.

In another embodiment, these constructs can also be used to determine specific activities of the invention, such as the ability of the invention to interact with a kinase. Briefly, *in vitro* kinase assays involve expressing the deletion constructs in a host cell, isolating the expressed protein protein, immunoprecipitating the expressed protein with an antibody, and incubating the immune complex with ³²P labeled ATP. This reaction is then run on SDS-PAGE and autoradiographed. This method is well known to one skilled in the art.

Another aspect of this invention uses these constructs to determine what regions of the invention are necessary for a known protein to bind. For example, the deletion constructs can be expressed in a host cell and protein lysates prepared. The proteins can then be subjected to Western blot analysis where the protein lysates are separated by SDS-PAGE, transferred to a membrane (i.e. nitrocellulose or nylon) and probed with an antibody against a protein of interest. This method of detection is well known in the art.

Another aspect of this invention involves using these deletion constructs to identify the portion of the invention required for the activation of a signaling protein. For example, a selected construct of the invention can be coexpressed with a luciferase reporter construct (engineered to have the promoter of the signaling protein of interest, i.e. NFkB promoter) in a host cell. After a given time, constructs that interact with the signaling protein of interest can be assayed by calculating the relative luciferase activity.

The efficacy of the modulating agent can be assessed by generating dose response curves from data obtained using various concentrations of the test modulating agent. Moreover, a control assay can also be performed to provide a baseline for comparison. In the control assay, isolated and purified TRADE protein is added to a composition containing the TRADE -binding element, and the formation of a complex is quantitated in the absence of the test modulating agent.

In yet another embodiment, an assay of the present invention is a cell-free assay in which a TRADE protein or biologically active portion thereof is contacted with a test compound and the ability of the test compound to bind to the TRADE protein or biologically active portion thereof is determined. Binding of the test compound to the TRADE protein can be determined either directly or indirectly as described above. In a preferred embodiment, the assay includes contacting the TRADE protein or biologically active portion thereof with a known compound which binds TRADE to form an assay mixture, contacting the assay mixture with a test compound, and determining the ability of the test compound to interact with a TRADE protein, wherein determining the ability of the test compound to interact with a TRADE protein comprises determining the ability of the test compound to preferentially bind to TRADE polypeptide or biologically active portion thereof as compared to the known compound.

In another embodiment, the assay is a cell-free assay in which a TRADE protein or biologically active portion thereof is contacted with a test compound and the ability of the test compound to modulate (e.g., stimulate or inhibit) the activity of the TRADE protein or biologically active portion thereof is determined. The TRADE protein can be provided as a lysate of cells that express TRADE, as a purified or semipurified polypeptide, or as a recombinantly expressed polypeptide. In one embodiment, a cell-free assay system further comprises a cell extract or isolated components of a cell, such as mitochondria. Such cellular components can be isolated using techniques which are known in the art. Preferably, a cell free assay system further comprises at least one target molecule with which TRADE interacts, and the ability of the test compound to modulate the interaction of the TRADE with the target molecule(s) is monitored to thereby identify the test compound as a modulator of TRADE. Determining the ability of the test compound to modulate the activity of a TRADE protein can be accomplished, for example, by determining the ability of the TRADE protein to bind to a TRADE target molecule by one of the methods described above for determining direct binding. Determining the ability of the TRADE protein to bind to a TRADE target molecule can also be accomplished using a technology such as real-time Biomolecular Interaction Analysis (BIA). Sjolander, S. and Urbaniczky, C., 1991, Anal. Chem. 63:2338-2345 and Szabo et al., 1995, Curr. Opin. Struct. Biol. 5:699-705. As used herein, "BIA" is a technology for studying biospecific interactions in real time, without labeling any of the interactants (e.g., BIAcore). Changes in the optical phenomenon of surface plasmon resonance (SPR) can be used as an indication of real-time reactions between biological molecules.

In yet another embodiment, the cell-free assay involves contacting a TRADE protein or biologically active portion thereof with a known compound which binds the TRADE protein to form an assay mixture, contacting the assay mixture with a test compound, and determining the ability of the test compound to interact with the TRADE protein, wherein determining the ability of the test compound to interact with the TRADE protein comprises determining the ability of the TRADE protein to preferentially bind to or modulate the activity of a TRADE target molecule.

The cell-free assays of the present invention are amenable to use of both soluble and/or membrane-bound forms of proteins (e.g., TRADE proteins or receptors having intracellular domains to which TRADE binds). In the case of cell-free assays in which a membrane-bound form of a protein is used it may be desirable to utilize a solubilizing agent such that the membrane-bound form of the protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

A TRADE target molecule can be a protein or a DNA sequence. Suitable assays are known in the art that allow for the detection of protein-protein interactions (*e.g*., immunoprecipitations, two-hybrid assays and the like) or that allow for the detection of interactions between a DNA binding protein with a target DNA sequence (*e.g.*, electrophoretic mobility shift assays, DNAse I footprinting assays and the like). By performing such assays in the presence and absence of test compounds, these assays can be used to identify compounds that modulate (*e.g.*, inhibit or enhance) the interaction of TRADE with a target molecule(s).

Determining the ability of the TRADE protein to bind to or interact with a ligand of a TRADE molecule can be accomplished, e.g., by direct binding. In a direct binding assay, the TRADE protein could be coupled with a radioisotope or enzymatic label such that binding of the TRADE protein to a TRADE target molecule can be determined by detecting the labeled TRADE protein in a complex. For example, TRADE molecules, e.g., TRADE proteins, can be labeled with ¹²⁵I, ³⁵S, ¹⁴C, or ³H, either directly or indirectly, and the radioisotope detected by direct counting of radioemmission or by scintillation counting. Alternatively, TRADE molecules can be enzymatically labeled with, for example, horseradish peroxidase, alkaline phosphatase, or luciferase, and the enzymatic label detected by determination of conversion of an appropriate substrate to product.

Typically, it will be desirable to immobilize either TRADE or its binding protein to facilitate separation of complexes from uncomplexed forms of one or both of the proteins, as well as to accommodate automation of the assay. Binding of TRADE to an upstream or downstream binding element, in the presence and absence of a candidate agent, can be accomplished in any vessel suitable for containing the reactants. Examples include microtitre plates, test tubes, and micro-centrifuge tubes. In one embodiment, a fusion protein can be provided which adds a domain that allows the protein to be bound to a matrix. For example, glutathione-S-transferase/ TRADE (GST/ TRADE) fusion proteins can be adsorbed onto glutathione sepharose beads (Sigma Chemical, St. Louis, MO) or glutathione derivatized microtitre plates, which are then combined with the cell lysates, e.g. an ³⁵S-labeled, and the test modulating agent, and the mixture incubated under conditions conducive to complex formation, e.g., at physiological conditions for salt and pH, though slightly more stringent conditions may be desired. Following incubation, the beads are washed to remove any unbound label, and the matrix immobilized and radiolabel determined directly (e.g. beads placed in scintilant), or in the supernatant after the complexes are subsequently dissociated. Alternatively, the complexes can be dissociated from the matrix, separated by SDS-PAGE, and the level of TRADE -binding protein found in the bead fraction quantitated from the gel using standard electrophoretic techniques.

Other techniques for immobilizing proteins on matrices are also available for use in the subject assay. For instance, either TRADE or its cognate binding protein can be immobilized utilizing conjugation of biotin and streptavidin. For instance, biotinylated TRADE molecules can be prepared from biotin-NHS (N-hydroxy-succinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with TRADE but which do not interfere with binding of upstream or downstream elements can be derivatized to the wells of the plate, and TRADE trapped in the wells by antibody conjugation. As above, preparations of a TRADE -binding protein and a test modulating agent are incubated in the TRADE - presenting wells of the plate, and the amount of complex trapped in the well can be quantitated. Exemplary methods for detecting such complexes, in addition to those described above for the GST-immobilized complexes, include immunodetection of complexes using antibodies reactive with the TRADE binding element, or which are reactive with TRADE protein and compete with the binding element; as well as enzyme-linked assays which rely on detecting an enzymatic activity associated with the binding element, either intrinsic or extrinsic activity. In the instance of the latter, the enzyme can be chemically conjugated or provided as a fusion protein with the TRADE -BP. To illustrate, the TRADE -BP can be chemically cross-linked or genetically fused with horseradish peroxidase, and the amount of protein trapped in the complex can be assessed with a chromogenic substrate of the enzyme, e.g. 3,3'-diamino-benzadine terahydrochloride or 4-chloro-1-napthol. Likewise, a fusion protein comprising the protein and glutathione-S-transferase can be provided, and complex formation quantitated by detecting the GST activity using 1-chloro-2,4-dinitrobenzene (Habig et al., 1974, J Biol Chem 249:7130).

For processes which rely on immunodetection for quantitating one of the proteins trapped in the complex, antibodies against the protein, such as anti- TRADE antibodies, can be used. Alternatively, the protein to be detected in the complex can be "epitope tagged" in the form of a fusion protein which includes, in addition to the TRADE sequence, a second protein for which antibodies are readily available (e.g. from commercial sources). For instance, the GST fusion proteins described above can also be used for quantification of binding using antibodies against the GST moiety. Other useful epitope tags include myc-epitopes (e.g., see Ellison et al., 1991, J Biol Chem 266:21150-21157) which includes a 10-residue sequence from c-myc, as well as the pFLAG system (International Biotechnologies, Inc.) or the pEZZ-protein A system (Pharamacia, NJ).

It is also within the scope of this invention to determine the ability of a compound to modulate the interaction between TRADE and its target molecule, without the labeling of any of the interactants. For example, a microphysiometer can be used to detect the interaction of TRADE with its target molecule without the labeling of either TRADE or the target molecule. McConnell, H. M. et al., 1992, Science 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between compound and receptor.

In addition to cell-free assays, the readily available source of TRADE proteins provided by the present invention also facilitates the generation of cell-based assays for identifying small molecule agonists/antagonists and the like. For example, cells can be caused to express or overexpress a recombinant TRADE protein in the presence and absence of a test modulating agent of interest, with the assay scoring for modulation in TRADE responses by the target cell mediated by the test agent. For example, as with the cell-free assays, modulating agents which produce a statistically significant change in TRADE -dependent responses (either an increase or decrease) can be identified.

Recombinant expression vectors that can be used for expression of TRADE are known in the art (see discussions above). In one embodiment, within the expression vector the TRADE-coding sequences are operatively linked to regulatory sequences that allow for constitutive or inducible expression of TRADE in the indicator cell(s). Use of a recombinant expression vector that allows for constitutive or inducible expression of TRADE in a cell is preferred for identification of compounds that enhance or inhibit the activity of TRADE. In an alternative embodiment, within the expression vector the TRADE coding sequences are operatively linked to regulatory sequences of the endogenous TRADE gene *(i.e.,* the promoter regulatory region derived from the endogenous gene). Use of a recombinant expression vector in which TRADE expression is controlled by the endogenous regulatory sequences is preferred for identification of compounds that enhance or inhibit the transcriptional expression of TRADE.

In one embodiment, an assay is a cell-based assay comprising contacting a cell expressing a TRADE target molecule (or another TRADE intracellular interacting molecule) with a test compound and determining the ability of the test compound to modulate (e.g. stimulate or inhibit) the activity of the TRADE target molecule. Determining the ability of the test compound to modulate the activity of a TRADE target molecule can be accomplished, for example, by determining the ability of the TRADE protein to bind to or interact with the TRADE target molecule or its ligand.

In an illustrative embodiment, the expression or activity of a TRADE is modulated in cells and the effects of modulating agents of interest on the readout of interest (such as apoptosis) are measured. In one embodiment, the regulatory regions of genes whose transcription is altered by a modulation in TRADE expression or activity, e.g., the 5' flanking promoter and enhancer regions, are operatively linked to a marker (such as luciferase) which encodes a gene product that can be readily detected.

In another embodiment, modulators of TRADE expression are identified in a method wherein a cell is contacted with a candidate compound and the expression of TRADE mRNA or protein in the cell is determined. The level of expression of TRADE mRNA or protein in the presence of the candidate compound is compared to the level of expression of TRADE mRNA or protein in the absence of the candidate compound. The candidate compound can then be identified as a modulator of TRADE expression based on this comparison. For example, when expression of TRADE mRNA or protein is greater (e.g., statistically significantly greater) in the presence of the candidate compound than in its absence, the candidate compound is identified as a stimulator of TRADE mRNA or protein expression. Alternatively, when expression of TRADE mRNA or protein is less (e.g., statistically significantly less) in the presence of the candidate compound than in its absence, the candidate compound is identified as an inhibitor of TRADE mRNA or protein expression. The level of TRADE mRNA or protein expression in the cells can be determined by methods described herein for detecting TRADE mRNA or protein.

In a preferred embodiment, determining the ability of the TRADE protein to bind to or interact with a TRADE target molecule can be accomplished by measuring a read out of the activity of TRADE or of the target molecule. For example, the activity of TRADE or a target molecule can be determined by detecting induction of a cellular second messenger of the target (e.g., a second messenger modulated by activation of an NFkB or JNK pathway), detecting catalytic/enzymatic activity of the target an appropriate substrate, detecting the induction of a reporter gene (comprising a target-responsive regulatory element operatively linked to a nucleic acid encoding a detectable marker, e.g., chloramphenicol acetyl transferase), or detecting a target-regulated cellular response, e.g., apoptosis. For example, determining the ability of the TRADE protein to bind to or interact with a TRADE target molecule can be accomplished, for example, by measuring the ability of a compound to modulate proliferation and/or apoptosis, preferably in a epithelial cell. The hallmark of apoptosis is degradation of DNA. Early in the process, this degradation occurs in internucleosomal DNA linker regions. The DNA cleavage may yield double-stranded and single-stranded DNA breaks. Apoptosis can be measured in cells using standard techniques. For example, degradation of genomic DNA of a population of cells can be analyzed by agarose gel electrophoresis, or DNA fragmentation assays based on 3H-thymidine or 5-Bromo-2'-deoxy-uridine can be used.

To analyze apoptosis in individual cells, apoptotic cells may be recognized microscopically because of the characteristic appearance of nuclear chromatin condensation and fragmentation. Apoptosis can be measured in individual cells, for example, using Hoechst stain and looking for cells with pyknotic nuclei as described in the appended Examples. Alternatively, double and single-stranded DNA breaks can be detected by labeling the free 3'-OH termini with modified nucleotides (e.g., biotin-dUTP, DIG-dUTP, fluorescein-dUTP) in an enzymatic reaction. Terminal deoxynucleotidyl transferase (TdT) catalyzes the template independent polymerization of deoxyribonucleotides to the 3' end of the DNA. This method is referred to as TUNEL (TdT-mediated dUTP-X nick end labeling). Alternatively, free 3'OH groups may be labeled using DNA polymerases by nick translation, tunnel staining can be used to identify cells with double stranded DNA breaks. Labeled free 3'OH groups that have incorporated labeled dUTP can be visualized by flow cytometry and/or fluorescence microscopy. Reagents for performing these assays are available e.g., from Roche Molecular Biochemicals USA (*In situ* cell death detection kit). In addition, annexin (e.g., Annexin-V-Alexa^{™} 568 commercially available from Roch molecular Biochemicals USA) can be used for this purpose. One of the early plasma membrane changes associated with cells undergoing apoptosis is the translocation of phosphatidylserine from the inner leaflet of the plasma membrane to the outer layer, thereby exposing phosphatidylserine at the surface of the cell. Annexin-V is a phospholipid binding protein which binds to phosphatidyl serine and can be used as a probe for phosphatidylserine on cell surfaces. Annexin-V can be used in combination with a DNA stain (e.g., BOBO^{™} -1) to differentiate apoptotic cells from necrotic cells.

The invention provides a method (also referred to herein as a "screening assay") for identifying modulators, i.e., candidate or test compounds or agents (*e.g.*, peptides, peptidomimetics, small molecules or other drugs) which bind to TRADE proteins, have a stimulatory or inhibitory effect on, for example, TRADE expression or TRADE activity.

The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, K.S. (1997) Anticancer Drug Des. 12:145).

Examples of methods for the synthesis of molecular libraries can be found in the art, for example in: DeWitt et al., 1993, Proc. Natl. Acad. Sci. U.S.A. 90:6909; Erb et al., 1994, Proc. Natl. Acad. Sci. USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and in Gallop et al., 1994, J. Med Chem. 37:1233.

Libraries of compounds may be presented in solution (e.g., Houghten, 1992, Biotechniques 13:412-421), or on beads (Lam, 1991, Nature 354:82-84), chips (Fodor, 1993, Nature 364:555-556), bacteria (Ladner USP 5,223,409), spores (Ladner USP '409), plasmids (Cull et al., 1992, Proc Natl Acad Sci USA 89:1865-1869) or on phage (Scott and Smith, 1990, Science 249:386-390; Devlin, 1990, Science 249:404-406; Cwirla et al., 1990, Proc. Natl. Acad. Sci. 87:6378-6382; Felici, 1991, J. Mol. Biol. 222:301-310; Ladner *supra*.).

In many drug screening programs which test libraries of modulating agents and natural extracts, high throughput assays are desirable in order to maximize the number of modulating agents surveyed in a given period of time. Assays which are performed in cell-free systems, such as those which may be derived with purified or semi-purified proteins, are often preferred as "primary" screens in that they can be generated to permit rapid development and relatively easy detection of an alteration in a molecular target which is mediated by a test modulating agent. Moreover, the effects of cellular toxicity and/or bioavailability of the test modulating agent can be generally ignored in the *in vitro* system, the assay instead being focused primarily on the effect of the drug on the molecular target as may be manifest in an alteration of binding affinity with upstream or downstream elements.

In one embodiment, the invention provides assays for screening candidate or test compounds which bind to or modulate the activity of a TRADE protein or polypeptide or biologically active portion thereof, e.g., modulate the ability of TRADE polypeptide to interact with a TRADE binding partner.

Assays can be used to screen for modulating agents, including TRADE homologs, which are either agonists or antagonists of the normal cellular function of the subject TRADE polypeptides. For example, the invention provides a method in which an indicator composition is provided which has a TRADE protein having a TRADE activity. The indicator composition can be contacted with a test compound. The effect of the test compound on TRADE activity, as measured by a change in the indicator composition, can then be determined to thereby identify a compound that modulates the activity of a TRADE protein. A statistically significant change, such as a decrease or increase, in the level of TRADE activity in the presence of the test compound (relative to what is detected in the absence of the test compound) is indicative of the test compound being a TRADE modulating agent. The indicator composition can be, for example, a cell or a cell extract. In one embodiment, TRADE activity is assessed as described in the appended Examples.

In an exemplary screening assay of the present invention, the modulating agent of interest is contacted with interactor proteins which may function upstream (including both activators and repressors of its activity) or to proteins which may function downstream of the TRADE protein, whether they are positively or negatively regulated by it. To the mixture of the modulating agent and the upstream or downstream element is then added a composition containing a TRADE protein. Detection and quantification of the interaction of TRADE with it's upstream or downstream elements provide a means for determining a modulating agent's efficacy at inhibiting (or potentiating) complex formation between TRADE and the TRADE binding elements.

The efficacy of the modulating agent can be assessed by generating dose response curves from data obtained using various concentrations of the test modulating agent. Moreover, a control assay can also be performed to provide a baseline for comparison. In the control assay, isolated and purified TRADE protein is added to a composition containing the TRADE -binding element, and the formation of a complex is quantitated in the absence of the test modulating agent.

In yet another embodiment, an assay of the present invention is a cell-free assay in which a TRADE protein or biologically active portion thereof is contacted with a test compound and the ability of the test compound to bind to the TRADE protein or biologically active portion thereof is determined. Binding of the test compound to the TRADE protein can be determined either directly or indirectly as described above. In a preferred embodiment, the assay includes contacting the TRADE protein or biologically active portion thereof with a known compound which binds TRADE to form an assay mixture, contacting the assay mixture with a test compound, and determining the ability of the test compound to interact with a TRADE protein, wherein determining the ability of the test compound to interact with a TRADE protein comprises determining the ability of the test compound to preferentially bind to TRADE polypeptide or biologically active portion thereof as compared to the known compound.

In another embodiment, the assay is a cell-free assay in which a TRADE protein or biologically active portion thereof is contacted with a test compound and the ability of the test compound to modulate (e.g., stimulate or inhibit) the activity of the TRADE protein or biologically active portion thereof is determined. The TRADE protein can be provided as a lysate of cells that express TRADE, as a purified or semipurified polypeptide, or as a recombinantly expressed polypeptide. In one embodiment, a cell-free assay system further comprises a cell extract or isolated components of a cell, such as mitochondria. Such cellular components can be isolated using techniques which are known in the art. Preferably, a cell free assay system further comprises at least one target molecule with which TRADE interacts, and the ability of the test compound to modulate the interaction of the TRADE with the target molecule(s) is monitored to thereby identify the test compound as a modulator of TRADE, activity. Determining the ability of the test compound to modulate the activity of a TRADE protein can be accomplished, for example, by determining the ability of the TRADE protein to bind to a TRADE target molecule, e.g., proliferation and/or apoptosis by one of the methods described above for determining direct binding. Determining the ability of the TRADE protein to bind to a TRADE target molecule can also be accomplished using a technology such as real-time Biomolecular Interaction Analysis (BIA). Sjolander, S. and Urbaniczky, C., 1991, Anal. Chem. 63:2338-2345 and Szabo et al., 1995, Curr. Opin. Struct. BioL 5:699-705. As used herein, "BIA" is a technology for studying biospecific interactions in real time, without labeling any of the interactants (e.g., BIAcore). Changes in the optical phenomenon of surface plasmon resonance (SPR) can be used as an indication of real-time reactions between biological molecules.

In yet another embodiment, the cell-free assay involves contacting a TRADE protein or biologically active portion thereof with a known compound which binds the TRADE protein to form an assay mixture, contacting the assay mixture with a test compound, and determining the ability of the test compound to interact with the TRADE protein, wherein determining the ability of the test compound to interact with the TRADE protein comprises determining the ability of the TRADE protein to preferentially bind to or modulate the activity of a TRADE target molecule.

The cell-free assays of the present invention are amenable to use of both soluble and/or membrane-bound forms of proteins (e.g., TRADE proteins or receptors having intracellular domains to which TRADE binds). In the case of cell-free assays in which a membrane-bound form a protein is used it may be desirable to utilize a solubilizing agent such that the membrane-bound form of the protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

A TRADE target molecule can be a protein or a DNA sequence. Suitable assays are known in the art that allow for the detection of protein-protein interactions (*e.g.,* immunoprecipitations, two-hybrid assays and the like) or that allow for the detection of interactions between a DNA binding protein with a target DNA sequence (*e.g.,* electrophoretic mobility shift assays, DNAse I footprinting assays and the like). By performing such assays in the presence and absence of test compounds, these assays can be used to identify compounds that modulate (*e.g.*, inhibit or enhance) the interaction of TRADE with a target molecule(s).

Determining the ability of the TRADE protein to bind to or interact with a ligand of a TRADE molecule can be accomplished, e.g., by direct binding. In a direct binding assay, the TRADE protein could be coupled with a radioisotope or enzymatic label such that binding of the TRADE protein to a TRADE target molecule can be determined by detecting the labeled TRADE protein in a complex. For example, TRADE molecules, e.g., TRADE proteins, can be labeled with ¹²⁵I, ³⁵S, ¹⁴C, or ³H, either directly or indirectly, and the radioisotope detected by direct counting of radioemmission or by scintillation counting. Alternatively, TRADE molecules can be enzymatically labeled with, for example, horseradish peroxidase, alkaline phosphatase, or luciferase, and the enzymatic label detected by determination of conversion of an appropriate substrate to product.

Typically, it will be desirable to immobilize either TRADE or its binding protein to facilitate separation of complexes from uncomplexed forms of one or both of the proteins, as well as to accommodate automation of the assay. Binding of TRADE to an upstream or downstream binding element, in the presence and absence of a candidate agent, can be accomplished in any vessel suitable for containing the reactants. Examples include microtitre plates, test tubes, and micro-centrifuge tubes. In one embodiment, a fusion protein can be provided which adds a domain that allows the protein to be bound to a matrix. For example, glutathione-S-transferase/ TRADE (GST/ TRADE) fusion proteins can be adsorbed onto glutathione sepharose beads (Sigma Chemical, St. Louis, MO) or glutathione derivatized microtitre plates, which are then combined with the cell lysates, e.g. an ³⁵S-labeled, and the test modulating agent, and the mixture incubated under conditions conducive to complex formation, e.g., at physiological conditions for salt and pH, though slightly more stringent conditions may be desired. Following incubation, the beads are washed to remove any unbound label, and the matrix immobilized and radiolabel determined directly (e.g. beads placed in scintilant), or in the supernatant after the complexes are subsequently dissociated. Alternatively, the complexes can be dissociated from the matrix, separated by SDS-PAGE, and the level of TRADE -binding protein found in the bead fraction quantitated from the gel using standard electrophoretic techniques.

Other techniques for immobilizing proteins on matrices are also available for use in the subject assay. For instance, either TRADE or its cognate binding protein can be immobilized utilizing conjugation of biotin and streptavidin. For instance, biotinylated TRADE molecules can be prepared from biotin-NHS (N-hydroxy-succinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with TRADE but which do not interfere with binding of upstream or downstream elements can be derivatized to the wells of the plate, and TRADE trapped in the wells by antibody conjugation. As above, preparations of a TRADE -binding protein and a test modulating agent are incubated in the TRADE-presenting wells of the plate, and the amount of complex trapped in the well can be quantitated. Exemplary methods for detecting such complexes, in addition to those described above for the GST-immobilized complexes, include immunodetection of complexes using antibodies reactive with the TRADE binding element, or which are reactive with TRADE protein and compete with the binding element; as well as enzyme-linked assays which rely on detecting an enzymatic activity associated with the binding element, either intrinsic or extrinsic activity. In the instance of the latter, the enzyme can be chemically conjugated or provided as a fusion protein with the TRADE -BP. To illustrate, the TRADE -BP can be chemically cross-linked or genetically fused with horseradish peroxidase, and the amount of protein trapped in the complex can be assessed with a chromogenic substrate of the enzyme, e.g. 3,3'-diamino-benzadine terahydrochloride or 4-chloro-1-napthol. Likewise, a fusion protein comprising the protein and glutathione-S-transferase can be provided, and complex formation quantitated by detecting the GST activity using 1-chloro-2,4-dinitrobenzene (Habig et al., 1974, J Biol Chem 249:7130).

For processes which rely on immunodetection for quantitating one of the proteins trapped in the complex, antibodies against the protein, such as anti- TRADE antibodies, can be used. Alternatively, the protein to be detected in the complex can be "epitope tagged" in the form of a fusion protein which includes, in addition to the TRADE sequence, a second protein for which antibodies are readily available (e.g. from commercial sources). For instance, the GST fusion proteins described above can also be used for quantification of binding using antibodies against the GST moiety. Other useful epitope tags include myc-epitopes (e.g., see Ellison et al., 1991, J Biol Chem 266:21150-21157) which includes a 10-residue sequence from c-myc, as well as the pFLAG system (International Biotechnologies, Inc.) or the pEZZ-protein A system (Pharamacia, NJ).

It is also within the scope of this invention to determine the ability of a compound to modulate the interaction between TRADE and its target molecule, without the labeling of any of the interactants. For example, a microphysiometer can be used to detect the interaction of TRADE with its target molecule without the labeling of either TRADE or the target molecule. McConnell, H. M. et al., 1992, Science 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between compound and receptor.

In addition to cell-free assays, the readily available source of TRADE proteins provided by the present invention also facilitates the generation of cell-based assays for identifying small molecule agonists/antagonists and the like. For example, cells can be caused to express or overexpress a recombinant TRADE protein in the presence or absence of a test modulating agent of interest, with the assay scoring for modulation in TRADE responses by the target cell mediated by the test agent. For example, as with the cell-free assays, modulating agents which produce a statistically significant change in TRADE -dependent responses (either an increase or decrease) can be identified.

Recombinant expression vectors that can be used for expression of TRADE are known in the art (see discussions above). In one embodiment, within the expression vector the TRADE-coding sequences are operatively linked to regulatory sequences that allow for constitutive or inducible expression of TRADE in the indicator cell(s). Use of a recombinant expression vector that allows for constitutive or inducible expression of TRADE in a cell is preferred for identification of compounds that enhance or inhibit the activity of TRADE. In an alternative embodiment, within the expression vector the TRADE coding sequences are operatively linked to regulatory sequences of the endogenous TRADE gene *(i.e.,* the promoter regulatory region derived from the endogenous gene). Use of a recombinant expression vector in which TRADE expression is controlled by the endogenous regulatory sequences is preferred for identification of compounds that enhance or inhibit the transcriptional expression of TRADE.

In one embodiment, an assay is a cell-based assay comprising contacting a cell expressing a TRADE target molecule (or another TRADE intracellular interacting molecule) with a test compound and determining the ability of the test compound to modulate (e.g. stimulate or inhibit) the activity of the TRADE target molecule. Determining the ability of the test compound to modulate the activity of a TRADE target molecule can be accomplished, for example, by determining the ability of the TRADE protein to bind to or interact with the TRADE target molecule or its ligand.

In an illustrative embodiment, the expression or activity of a TRADE is modulated in cells and the effects of modulating agents of interest on the readout of interest (such as proliferation or apoptosis) are measured. In one embodiment, the regulatory regions of genes whose transcription is altered by a modulation in TRADE expression or activity, e.g., the 5' flanking promoter and enhancer regions, are operatively linked to a marker (such as luciferase) which encodes a gene product that can be readily detected.

In another embodiment, modulators of TRADE expression are identified in a method wherein a cell is contacted with a candidate compound and the expression of TRADE mRNA or protein in the cell is determined. The level of expression of TRADE mRNA or protein in the presence of the candidate compound is compared to the level of expression of TRADE mRNA or protein in the absence of the candidate compound. The candidate compound can then be identified as a modulator of TRADE expression based on this comparison. For example, when expression of TRADE mRNA or protein is greater (e.g., statistically significantly greater) in the presence of the candidate compound than in its absence, the candidate compound is identified as a stimulator of TRADE mRNA or protein expression. Alternatively, when expression of TRADE mRNA or protein is less (e.g., statistically significantly less) in the presence of the candidate compound than in its absence, the candidate compound is identified as an inhibitor of TRADE mRNA or protein expression. The level of TRADE mRNA or protein expression in the cells can be determined by methods described herein for detecting TRADE mRNA or protein.

In a preferred embodiment, determining the ability of the TRADE protein to bind to or interact with a TRADE target molecule can be accomplished by measuring a read out of the activity of TRADE or of the target molecule. For example, the activity of TRADE or a target molecule can be determined by detecting induction of a cellular second messenger of the target (e.g., a second messenger modulated by activation of a JNK or NFkB signaling pathway), detecting catalytic/enzymatic activity of the target an appropriate substrate, detecting the induction of a reporter gene (comprising a target-responsive regulatory element operatively linked to a nucleic acid encoding a detectable marker, e.g., chloramphenicol acetyl transferase), or detecting a target-regulated cellular response, e.g., apoptosis. For example, determining the ability of the TRADE protein to bind to or interact with a TRADE target molecule can be accomplished, for example, by measuring the ability of a compound to modulate apoptosis, preferably in a epithelial cell. The hallmark of apoptosis is degradation of DNA. Early in the process, this degradation occurs in internucleosomal DNA linker regions. The DNA cleavage may yield double-stranded and single-stranded DNA breaks. Apoptosis can be measured in cells using standard techniques. For example, degradation of genomic DNA of a population of cells can be analyzed by agarose gel electrophoresis, or DNA fragmentation assays based on 3H-thymidine or 5-Bromo-2'-deoxy-uridine can be used.

To analyze apoptosis in individual cells, apoptotic cells may be recognized microscopically because of the characteristic appearance of nuclear chromatin condensation and fragmentation. Apoptosis can be measured in individual cells; for example, using Hoechst stain and looking for cells with pyknotic nuclei as described in the appended Examples. Alternatively, double and single-stranded DNA breaks can be detected by labeling the free 3'-OH termini with modified nucleotides (e.g., biotin-dUTP, DIG-dUTP, fluorescein-dUTP) in an enzymatic reaction. Terminal deoxynucleotidyl transferase (TdT) catalyzes the template independent polymerization of deoxyribonucleotides to the 3' end of the DNA. This method is referred to as TUNEL (TdT-mediated dUTP-X nick end labeling). Alternatively, free 3'OH groups may be labeled using DNA polymerases by nick translation, tunnel staining can be used to identify cells with double stranded DNA breaks. Labeled free 3'OH groups that have incorporated labeled dUTP can be visualized by flow cytometry and/or fluorescence microscopy. Reagents for performing these assays are available e.g., from Roche Molecular Biochemicals USA (*In situ* cell death detection kit). In addition, annexin (e.g., Annexin-V-Alexa^{™} 568 commercially available from Roch molecular Biochemicals USA) can be used for this purpose. One of the early plasma membrane changes associated with cells undergoing apoptosis is the translocation of phosphatidylserine from the inner leaflet of the plasma membrane to the outer layer, thereby exposing phosphatidylserine at the surface of the cell. Annexin-V is a phospholipid binding protein which binds to phosphatidyl serine and can be used as a probe for phosphatidylserine on cell surfaces. Annexin-V can be used in combination with a DNA stain (e.g., BOBO^{™}-1) to differentiate apoptotic cells from necrotic cells.

In yet another aspect of the invention TRADE proteins or portions thereof can be used as "bait proteins" in a two-hybrid assay or three-hybrid assay (see, e.g., U.S. Patent No. 5,283,317; Zervos et al., 1993, Cell 72:223-232; Madura et al., 1993, J. Biol. Chem. 268:12046-12054; Bartel et al., 1993, Biotechniques 14:920-924; Iwabuchi et al., 1993, Oncogene 8:1693-1696; and Brent WO94/10300), to identify other proteins, which bind to or interact with TRADE ("TRADE-binding proteins" or "TRADE-bp") and are involved in TRADE activity. Such TRADE-binding proteins are also likely to be involved in the propagation of signals by the TRADE proteins or TRADE targets as, for example, downstream elements of a TRADE-mediated signaling pathway. Alternatively, such TRADE-binding proteins may be TRADE inhibitors.

The two-hybrid system is based on the modular nature of most transcription factors, which consist of separable DNA-binding and activation domains. Briefly, the assay utilizes two different DNA constructs. In one construct, the gene that codes for a TRADE protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a TRADE-dependent complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the TRADE protein.

The present invention also provides a kit comprising a two-hybrid system having (1) a first hybrid protein comprising TRADE and a transcriptional activation domain (2) a second hybrid protein comprising a TRADE binding partner and a DNA-binding domain, a host cell, and an instruction manual. Alternatively, the TRADE polypeptide may be fused to the DNA-binding domain and the binding partner fused to the activation domains. Such kits may optionally include a panel of agents for testing for the capacity to alter intermolecular binding between the first and second hybrid proteins.

This invention further pertains to novel agents identified by the above-described screening assays. Accordingly, it is within the scope of this invention to further use an agent identified as described herein in an appropriate animal model. For example, an agent identified as described herein (e.g., a TRADE modulating agent, an antisense TRADE nucleic acid molecule, a TRADE-specific antibody, or a TRADE -binding partner) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

### B. Methods Of Rational Drug Design

TRADE and TRADE binding polypeptides, especially those portions which form direct contacts in TRADE/binding partner heterodimers, can be used for rational drug design of candidate TRADE -modulating agents (e.g., antineoplastics for use in down modulation of proliferation or induction of apoptosis). The production of substantially pure TRADE polypeptide/binding partner complexes and computational models which can be used for protein X-ray crystallography or other structure analysis methods, such as the DOCK program (Kuntz et al., 1982, J. Mol. Biol. 161: 269; Kuntz ID, 1992, Science 257: 1078) and variants thereof. Potential therapeutic drugs may be designed rationally on the basis of structural information thus provided. In one embodiment, such drugs are designed to prevent or enhance formation of a TRADE polypeptide: binding partner complex. Thus, the present invention may be used to design drugs, including drugs with a capacity to inhibit or promote binding of TRADE to a binding partner, *e.g.* a TRAF molecule.

### V. Other Uses and Methods of the Invention

The nucleic acid molecules, proteins, protein homologues, and antibodies described herein can be used in one or more of the following a) use in the manufacture of a medicament for the treatment of neoplasia e.g., up- or down-modulating proliferation and/or apoptosis, preferably in epithelial cells; b) screening assays; c) predictive medicine (e.g., diagnostic assays, prognostic assays, monitoring clinical trials, or pharmacogenetics). The isolated nucleic acid molecules of the invention can be used, for example, to express TRADE protein (e.g., via a recombinant expression vector in a host cell in gene therapy applications), to detect TRADE mRNA (e.g., in a biological sample) or a genetic alteration in a TRADE gene, and to modulate TRADE activity, as described further herein. The TRADE proteins can be used to treat disorders characterized by insufficient or excessive production of TRADE inhibitors. In addition, the TRADE proteins can be used to screen for naturally occurring TRADE binding proteins, to screen for drugs or compounds which modulate TRADE activity, as well as to treat disorders that would benefit from modulation of TRADE, e.g., characterized by insufficient or excessive production of TRADE protein or production of TRADE protein forms which have decreased or aberrant activity compared to TRADE wild type protein. Moreover, the anti-TRADE antibodies of the invention can be used to detect and isolate TRADE proteins, regulate the bioavailability of TRADE proteins, and modulate a TRADE activity. In preferred embodiments the methods of the invention, e.g., detection, modulation of TRADE, etc. are performed in epithelial cells, e.g., ductal epithelial cells. In a preferred embodiment, the cells are derived from a tissue in which TRADE is expressed, e.g., a tissue selected from the group consisting of: liver, lung, prostate, brain, or intestine.

### A. Detection Assays

Agents capable of detecting the presence of a TRADE molecule in a sample, e.g., portions or fragments of the cDNA sequences identified herein (and the corresponding complete gene sequences), antibodies that recognize TRADE family polypeptides or specific TRADE polypeptides, can be used in numerous ways to detect TRADE nucleic acid or polypeptide molecules. For example, TRADE molecules can be detected in order to: (i) map their locus on a chromosome; and, thus, locate gene regions associated with genetic disease; (ii) identify an individual from a minute biological sample (tissue typing); (iii) aid in forensic identification of a biological sample; or (iv) detect whether or not TRADE is expressed in a cell or to quantitate the level of TRADE expression in a cell.

For example, diagnostic assays, prognostic assays, and monitoring clinical trials are used to identify individuals who would benefit from treatment of a TRADE-associated disorder or who might be at risk for developing a TRADE associated disorder. Accordingly, one aspect of the present invention relates to diagnostic/prognostic assays for determining TRADE protein and/or nucleic acid expression as well as TRADE activity, in the context of a biological sample (e.g., blood, serum, cells, tissue (preferably epithelial cells or tissue)) to determine whether an individual is afflicted with a disease or disorder, or is at risk of developing a disorder, associated with aberrant TRADE expression or activity. The invention also provides for prognostic (or predictive) assays for determining whether an individual is at risk of developing a disorder associated with TRADE protein, nucleic acid expression or activity. For example, mutations in a TRADE gene can be assayed in a biological sample. Such assays can be used for prognostic or predictive purpose to thereby prophylactically treat an individual prior to the onset of a disorder characterized by or associated with TRADE protein, nucleic acid expression or activity.

Another aspect of the invention pertains to monitoring the influence of agents (e.g., drugs, compounds) on the expression or activity of TRADE in clinical trials.

Preferably, the instant diagnostic, prognostic, or clinical assays are performed on tissue samples (or cells derived from tissue samples) in which TRADE is expressed, e.g., on epithelial cells such as: liver, prostate, lung, brain, or intestinal cells.

An exemplary method for detecting the presence or absence of TRADE protein or nucleic acid in a biological sample involves obtaining a biological sample from a test subject and contacting the biological sample with a compound or an agent capable of detecting TRADE protein or nucleic acid molecule (e.g., mRNA, genomic DNA) that encodes TRADE protein such that the presence of TRADE protein or nucleic acid molecule is detected in the biological sample. A preferred agent for detecting TRADE mRNA or genomic DNA is a labeled nucleic acid probe capable of hybridizing to TRADE mRNA or genomic DNA. The nucleic acid probe can be, for example, a TRADE nucleic acid, such as the nucleic acid of SEQ ID NO:1 or 3 or a nucleotide sequence encoding another TRADE family polypeptide, or a portion thereof, such as an oligonucleotide of at least 15, 30, 50, 100, 250 or 500 nucleotides in length and sufficient to specifically hybridize under stringent conditions to TRADE mRNA or genomic DNA or TRADEα or TRADEβ mRNA or genomic DNA. Other suitable probes for use in the diagnostic assays of the invention are described herein.

A preferred agent for detecting TRADE protein is an antibody capable of binding to TRADE protein, preferably an antibody with a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells (preferably epithelial cells or tissue) and fluids present within a subject. That is, the detection method for the invention can be used to detect TRADE mRNA, protein, or genomic DNA in a biological sample *in vitro* as well as *in vivo.* For example, *in vitro* techniques for detection of TRADE mRNA include Northern hybridizations and *in situ* hybridizations. *In vitro* techniques for detection of TRADE protein include enzyme linked immunosorbent assays (ELISAs), Western blots, immunoprecipitation and immunofluorescence. *In vitro* techniques for detection of TRADE genomic DNA include Southern hybridizations. Furthermore, *in vivo* techniques for detection of TRADE protein include introducing into a subject a labeled anti-TRADE antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject A preferred biological sample is a serum sample isolated by conventional means from a subject.

In another embodiment, the methods further involve obtaining a control biological sample from a control subject, contacting the control sample with a compound or agent capable of detecting TRADE protein, mRNA, or genomic DNA, such that the presence of TRADE protein, mRNA or genomic DNA is detected in the biological sample, and comparing the presence of TRADE protein, mRNA or genomic DNA in the control sample with the presence of TRADE protein, mRNA or genomic DNA in the test sample.

Also described herein are kits for detecting the presence of TRADE in a biological sample. For example; the kit can comprise a labeled compound or agent capable of detecting TRADE protein or mRNA in a biological sample; means for determining the amount of TRADE in the sample; and means for comparing the amount of TRADE in the sample with a standard. The compound or agent can be packaged in a suitable container. The kit can further comprise instructions for using the kit to detect TRADE protein or nucleic acid.

The diagnostic methods described herein can furthermore be utilized to identify subjects having or at risk of developing a disease or disorder associated with aberrant TRADE expression or activity. For example, the assays described herein, such as the preceding diagnostic assays or the following assays, can be utilized to identify a subject having or at risk of developing a disorder associated with TRADE protein, nucleic acid expression or activity. Thus, the present invention provides a method for identifying a disease or disorder associated with aberrant TRADE expression or activity in which a test sample is obtained from a subject and TRADE protein or nucleic acid (e.g., mRNA, genomic DNA) is detected, wherein the presence of TRADE protein or nucleic acid is diagnostic for a subject having or at risk of developing a disease or disorder associated with aberrant TRADE expression or activity. As used herein, a "test sample" refers to a biological sample obtained from a subject of interest. For example, a test sample can be a biological fluid (e.g., serum), cell sample, or tissue.

Furthermore, the prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant TRADE expression or activity. Thus, the present invention provides methods for determining whether a subject can be effectively treated with an agent for a disorder associated with aberrant TRADE expression or activity in which a test sample is obtained and TRADE protein or nucleic acid expression or activity is detected (e.g., wherein the abundance of TRADE protein or nucleic acid expression or activity is diagnostic for a subject that can be administered the agent to treat a disorder associated with aberrant TRADE expression or activity).

The methods of the invention can also be used to detect genetic alterations in a TRADE gene, thereby determining if a subject with the altered gene is at risk for a disorder associated with the TRADE gene. In preferred embodiments, the methods include detecting, in a sample of cells from the subject, the presence or absence of a genetic alteration characterized by at least one of an alteration affecting the integrity of a gene encoding a TRADE -protein, or the mis-expression of the TRADE gene. For example, such genetic alterations can be detected by ascertaining the existence of at least one of 1) a deletion of one or more nucleotides from a TRADE gene; 2) an addition of one or more nucleotides to a TRADE gene; 3) a substitution of one or more nucleotides of a TRADE gene, 4) a chromosomal rearrangement of a TRADE gene; 5) an alteration in the level of a messenger RNA transcript of a TRADE gene, 6) aberrant modification of a TRADE gene, such as of the methylation pattern of the genomic DNA, 7) the presence of a non-wild type splicing pattern of a messenger RNA transcript of a TRADE gene, 8) a non-wild type level of a TRADE protein, 9) allelic loss of a TRADE gene, and 10) inappropriate post-translational modification of a TRADE protein. As described herein, there are a large number of assay techniques known in the art which can be used for detecting alterations in a TRADE gene. A preferred biological sample is a tissue or serum sample isolated by conventional means from a subject, e.g., a neural tissue sample.

In certain embodiments, detection of the alteration involves the use of a probe/primer in a polymerase chain reaction (PCR) (see, e.g., U.S. Patent Nos. 4,683,195 and 4,683,202), such as anchor PCR or RACE PCR, or, alternatively, in a ligation chain reaction (LCR) (see, e.g., Landegran et al., 1988, Science 241:1077-1080; and Nakazawa et al., 1994, Proc. Natl. Acad. Sci. USA 91:360-364), the latter of which can be particularly useful for detecting point mutations in the TRADE gene (see Abravaya et al., 1995, Nucleic Acids Res .23:675-682). This method can include the steps of collecting a sample of cells from a patient, isolating nucleic acid (e.g., genomic, mRNA or both) from the cells of the sample, contacting the nucleic acid sample with one or more primers which specifically hybridize to a TRADE gene under conditions such that hybridization and amplification of the TRADE gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al., 1990, Proc. Natl. Acad Sci. USA 87:1874-1878), transcriptional amplification system (Kwoh, D.Y. et al., 1989, Proc. Natl. Acad. Sci. USA 86:1173-1177), Q-Beta Replicase (Lizardi, P.M. et al., 1988, Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a TRADE gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in TRADE can be identified by hybridizing a sample and control nucleic acids, e.g., DNA or RNA, to high density arrays containing hundreds or thousands of oligonucleotides probes (Cronin, M.T. et al. , 1996, Human Mutation 7: 244-255; Kozal, M.J. et al., 1996, Nature Medicine 2: 753-759). For example, genetic mutations in TRADE can be identified in two dimensional arrays containing light-generated DNA probes as described in Cronin, M.T. *et al. supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the TRADE gene and detect mutations by comparing the sequence of the sample TRADE with the corresponding wild-type (control) sequence. Examples of sequencing reactions include those based on techniques developed by Maxam and Gilbert (Maxim and Gilbert, 1977, Proc. Natl. Acad. Sci. USA 74:560) or Sanger (Sanger, 1977, Proc. Natl. Acad. Sci. USA 74:5463). It is also contemplated that any of a variety of automated sequencing procedures can be utilized when performing the diagnostic assays (Lefevre, CK, 1995, Biotechniques 19:448), including sequencing by mass spectrometry (see, e.g., PCT International Publication No. WO 94/16101; Cohen et al., 1996, Adv. Chromatogr. 36:127-162; and Griffin et al., 1993, Appl. Biochem. Biotechnol. 38:147-159).

Other methods for detecting mutations in the TRADE gene include methods in which protection from cleavage agents is used to detect mismatched bases in RNA/RNA or RNA/DNA heteroduplexes (Myers et al., 1985, Science 230:1242). In general, the art technique of "mismatch cleavage" starts by providing heteroduplexes formed by hybridizing (labeled) RNA or DNA containing the wild-type TRADE sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al., 1988, Proc. Natl Acad Sci USA 85:4397; Saleeba et al., 1992, Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in TRADE obtained from samples of cells. For example, the mutY enzyme of *E. coli* cleaves A at G/A mismatches and the thymidine DNA glycosylase from HeLa cells cleaves T at G/T mismatches (Hsu et al., 1994, Carcinogenesis 15:1657-1662). According to an exemplary embodiment, a probe based on a TRADE sequence, e.g., a wild-type TRADE sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in TRADE genes. For example, single strand conformation polymorphism (SSCP) may be used to detect differences in electrophoretic mobility between mutant and wild type nucleic acids (Orita et al., 1989, Proc Natl. Acad. Sci USA: 86:2766, see also Cotton, 1993, Mutat Res 285:125-144; and Hayashi, 1992, Genet Anal Tech Appl 9:73-79). Single-stranded DNA fragments of sample and control TRADE nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et.al. (1991) Trends Genet 7:5).

In yet another embodiment the movement of mutant or wild-type fragments in polyacrylamide gels containing a gradient of denaturant is assayed using denaturing gradient gel electrophoresis (DGGE) (Myers et al., 1985, Nature 313:495). When DGGE is used as the method for analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner, 1987, Biophys Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al., 1986, Nature 324:163); Saiki et al., 1989, Proc. Natl Acad. Sci USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al., 1989, Nucleic Acids Res. 17:2437-2448) or at the extreme 3' end of one primer where, under appropriate conditions, mismatch can prevent, or reduce polymerase extension (Prossner et al., 1993, Tibtech 11:238). In addition it may be desirable to introduce a novel restriction site in the region of the mutation to create cleavage-based detection (Gasparini et al., 1992, Mol. Cell Probes 6:1). It is anticipated that in certain embodiments amplification may also be performed using Taq ligase for amplification (Barany, 1991, Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing prepackaged diagnostic kits comprising at least one probe nucleic acid or antibody reagent described herein, which may be conveniently used, *e.g.*, in clinical settings to diagnose patients exhibiting symptoms or family history of a disease or illness involving a TRADE gene.

Furthermore, any cell type or tissue in which TRADE is expressed may be utilized in the prognostic assays described herein.

### VI. Administration of TRADE Modulating Agents

TRADE modulating agents of the invention are administered to subjects in a biologically compatible form suitable for pharmaceutical administration *in vivo* to either enhance or suppress T cell mediated immune response. By "biologically compatible form suitable for administration *in vivo"* is meant a form of the protein to be administered in which any toxic effects are outweighed by the therapeutic effects of the protein. The term subject is intended to include living organisms in which an immune response can be elicited, e.g., mammals. Examples of subjects include humans, dogs, cats, mice, rats, and transgenic species thereof. Administration of an agent as described herein can be in any pharmacological form including a therapeutically active amount of an agent alone or in combination with a pharmaceutically acceptable carrier.

Administration of a therapeutically active amount of the therapeutic compositions of the present invention is defined as an amount effective, at dosages and for periods of time necessary to achieve the desired result. For example, a therapeutically active amount of a TRADE modulating agent may vary according to factors such as the disease state, age, sex, and weight of the individual, and the ability of peptide to elicit a desired response in the individual. Dosage regimen may be adjusted to provide the optimum therapeutic response. For example, several divided doses may be administered daily or the dose may be proportionally reduced as indicated by the exigencies of the therapeutic situation.

The therapeutic or pharmaceutical compositions of the present invention can be administered by any suitable route known in the art including for example intravenous, subcutaneous, intramuscular, transdermal, intrathecal or intracerebral or administration to cells in ex vivo treatment protocols. Administration can be either rapid as by injection or over a period of time as by slow infusion or administration of slow release formulation. For treating tissues in the central nervous system, administration can be by injection or infusion into the cerebrospinal fluid (CSF). When it is intended that a TRADE polypeptide be administered to cells in the central nervous system, administration can be with one or more agents capable of promoting penetration of TRADE polypeptide across the blood-brain barrier.

TRADE molecules can also be linked, conjugated, or administered with agents that provide desirable pharmaceutical or pharmacodynamic properties. For example, TRADE can be coupled to any substance known in the art to promote penetration or transport across the blood-brain barrier such as an antibody to the transferrin receptor, and administered by intravenous injection. (See for example, Friden et al., 1993, Science 259: 373-377 which is incorporated by reference). Furthermore, TRADE can be stably linked to a polymer such as polyethylene glycol to obtain desirable properties of solubility, stability, half-life and other pharmaceutically advantageous properties. (See for example Davis et al., 1978, Enzyme Eng 4: 169-73; Burnham, 1994, Am J Hosp Pharm 51: 210-218, which are incorporated by reference).

Furthermore, a TRADE molecule can be in a composition which aids in delivery into the cytosol of a cell. For example, a TRADE molecule may be conjugated with a carrier moiety such as a liposome that is capable of delivering the peptide into the cytosol of a cell. Such methods are well known in the art (for example see Amselem et al., 1993, Chem Phys Lipids 64: 219-237, which is incorporated by reference). Alternatively, a TRADE molecule can be modified to include specific transit peptides or fused to such transit peptides which are capable of delivering the TRADE molecule into a cell. In addition, the molecule can be delivered directly into a cell by microinjection.

The compositions are usually employed in the form of pharmaceutical preparations. Such preparations are made in a manner well known in the pharmaceutical art. One preferred preparation utilizes a vehicle of physiological saline solution, but other pharmaceutically acceptable carriers such as physiological concentrations of other non-toxic salts, five percent aqueous glucose solution, sterile water or the like may also be used. As used herein "pharmaceutically acceptable carrier" includes any and all solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the active compound, use thereof in the therapeutic compositions is contemplated. Supplementary active compounds can also be incorporated into the compositions. It may also be desirable that a suitable buffer be present in the composition. Such solutions can, if desired, be lyophilized and stored in a sterile ampoule ready for reconstitution by the addition of sterile water for ready injection. The primary solvent can be aqueous or alternatively non-aqueous. TRADE can also be incorporated into a solid or semi-solid biologically compatible matrix which can be implanted into tissues requiring treatment.

The carrier can also contain other pharmaceutically-acceptable excipients for modifying or maintaining the pH, osmolarity, viscosity, clarity, color, sterility, stability, rate of dissolution, or odor of the formulation. Similarly, the carrier may contain still other pharmaceutically-acceptable excipients for modifying or maintaining release or absorption or penetration across the blood-brain barrier. Such excipients are those substances usually and customarily employed to formulate dosages for parenteral administration in either unit dosage or multi-dose form or for direct infusion by continuous or periodic infusion.

Dose administration can be repeated depending upon the pharmacokinetic parameters of the dosage formulation and the route of administration used. It is also provided that certain formulations containing the TRADE molecule or fragment thereof are to be administered orally. Such formulations are preferably encapsulated and formulated with suitable carriers in solid dosage forms. Some examples of suitable carriers, excipients, and diluents include lactose, dextrose, sucrose, sorbitol, mannitol, starches, gum acacia, calcium phosphate, alginates, calcium silicate, microcrystalline cellulose, olyvinylpyrrolidone, cellulose, gelatin, syrup, methyl cellulose, methyl- and propylhydroxybenzoates, talc, magnesium, stearate, water, mineral oil, and the like. The formulations can additionally include lubricating agents, wetting agents, emulsifying and suspending agents, preserving agents, sweetening agents or flavoring agents. The compositions may be formulated so as to provide rapid, sustained, or delayed release of the active ingredients after administration to the patient by employing procedures well known in the art. The formulations can also contain substances that diminish proteolytic degradation and/or substances which promote absorption such as, for example, surface active agents.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals. The specific dose can be readily calculated by one of ordinary skill in the art, e.g., according to the approximate body weight or body surface area of the patient or the volume of body space to be occupied. The dose will also be calculated dependent upon the particular route of administration selected. Further refinement of the calculations necessary to determine the appropriate dosage for treatment is routinely made by those of ordinary skill in the art. Such calculations can be made without undue experimentation by one skilled in the art in light of the activity disclosed herein in assay preparations of target cells. Exact dosages are determined in conjunction with standard dose-response studies. It will be understood that the amount of the composition actually administered will be determined by a practitioner, in the light of the relevant circumstances including the condition or conditions to be treated, the choice of composition to be administered, the age, weight, and response of the individual patient, the severity of the patient's symptoms, and the chosen route of administration.

Toxicity and therapeutic efficacy of such compounds can be determined by standard pharmaceutical procedures in cell cultures or experimental animals, *e.g.*, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method for the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

In one embodiment of this invention, a TRADE molecule may be therapeutically administered by implanting into patients vectors or cells capable of producing a biologically-active form of TRADE or a precursor of TRADE, i.e. a molecule that can be readily converted to a biological-active form of TRADE by the body. In one approach cells that secrete TRADE may be encapsulated into semipermeable membranes for implantation into a patient. The cells can be cells that normally express TRADE or a precursor thereof or the cells can be transformed to express TRADE or a biologically active fragment thereof or a precursor thereof. It is preferred that the cell be of human origin and that the TRADE molecule be human TRADE when the patient is human. However, the formulations and methods herein can be used for veterinary as well as human applications and the term "patient" or "subject" as used herein is intended to include human and veterinary patients.

Monitoring the influence of agents (e.g., drugs or compounds) on the expression or activity of a TRADE protein can be applied not only in basic drug screening, but also in clinical trials. For example, the effectiveness of an agent determined by a screening assay as described herein to increase TRADE gene expression, protein levels, or upregulate TRADE activity, can be monitored in clinical trials of subjects exhibiting decreased TRADE gene expression, protein levels, or downregulated TRADE activity. Alternatively, the effectiveness of an agent determined by a screening assay to decrease TRADE gene expression, protein levels, or downregulate TRADE activity, can be monitored in clinical trials of subjects exhibiting increased TRADE gene expression, protein levels, or upregulated TRADE activity. In such clinical trials, the expression or activity of a TRADE gene, and preferably, other genes that have been implicated in a disorder can be used as a "read out" or markers of the phenotype of a particular cell.

For example, and not by way of limitation, genes, including TRADE, that are modulated in cells by treatment with an agent (e.g., compound, drug or small molecule) which modulates TRADE activity (e.g., identified in a screening assay as described herein) can be identified. Thus, to study the effect of agents on a TRADE associated disorder, for example, in a clinical trial, cells can be isolated and RNA prepared and analyzed for the levels of expression of TRADE or other genes implicated in the TRADE associated disorder. The levels of gene expression (i.e., a gene expression pattern) can be quantified by Northern blot analysis or RT-PCR, as described herein, or alternatively by measuring the amount of protein produced, by one of the methods as described herein, or by measuring the levels of activity of TRADE or other genes. In this way, the gene expression pattern can serve as a marker, indicative of the physiological response of the cells to the agent. Accordingly, this response state may be determined before, and at various points during treatment of the individual with the agent.

In a preferred embodiment, the present invention provides a method for monitoring the effectiveness of treatment of a subject with an agent (e.g., an agonist, antagonist, peptidomimetic, protein, peptide, nucleic acid, small molecule, or other drug candidate identified by the screening assays described herein) comprising the steps of (i) obtaining a pre-administration sample from a subject prior to administration of the agent; (ii) detecting the level of expression of a TRADE protein, mRNA, or genomic DNA in the pre-administration sample; (iii) obtaining one or more post-administration samples from the subject; (iv) detecting the level of expression or activity of the TRADE protein, mRNA, or genomic DNA in the post-administration samples; (v) comparing the level of expression or activity of the TRADE protein, mRNA, or genomic DNA in the pre-administration sample with the TRADE protein, mRNA, or genomic DNA in the post administration sample or samples; and (vi) altering the administration of the agent to the subject accordingly. For example, increased administration of the agent may be desirable to increase the expression or activity of TRADE to higher levels than detected, i.e., to increase the effectiveness of the agent. Alternatively, decreased administration of the agent may be desirable to decrease expression or activity of TRADE to lower levels than detected, i.e. to decrease the effectiveness of the agent. According to such an embodiment, TRADE expression or activity may be used as an indicator of the effectiveness of an agent, even in the absence of an observable phenotypic response.

In a preferred embodiment, the ability of a TRADE modulating agent to modulate apoptosis in a epithelial cell of a subject that would benefit from modulation of the expression and/or activity of TRADE can be measured by detecting an improvement in the condition of the patient after the administration of the agent. Such improvement can be readily measured by one of ordinary skill in the art using indicators appropriate for the specific condition of the patient. Monitoring the response of the patient by measuring changes in the condition of the patient is preferred in situations were the collection of biopsy materials would pose an increased risk and/or detriment to the patient.

It is likely that the level of TRADE may be altered in a variety of conditions and that quantification of TRADE levels would provide clinically useful information. Furthermore, because it has been demonstrated herein that increased levels of TRADE expressed by a cell can shift the cell death regulatory mechanism of that cell to decrease viability, it is believed that measurement of the level of TRADE in a cell or cells such as in a group of cells, tissue or neoplasia, like will provide useful information regarding apoptotic state of that cell or cells. In addition, it can also be desirable to determine the cellular levels of these TRADE-interacting polypeptides.

Furthermore, in the treatment of disease conditions, compositions containing TRADE can be administered exogenously and it would likely be desirable to achieve certain target levels of TRADE polypeptide in sera, in any desired tissue compartment or in the affected tissue. It would, therefore, be advantageous to be able to monitor the levels of TRADE polypeptide in a patient or in a biological sample including a tissue biopsy sample obtained form a patient and, in some cases, also monitoring the levels of TRADE and, in some circumstances, also monitoring levels of TRAF or another TRADE-interacting polypeptide. Accordingly, the present invention also provides methods for detecting the presence of TRADE in a sample from a patient.

### VII. Kits of the Invention

Another aspect of the invention pertains to kits for carrying out the screening assays, modulatory methods or diagnostic assays of the invention. For example, a kit for carrying out a screening assay of the invention can include a cell comprising a TRADE polypeptide, means for determining TRADE polypeptide activity and instructions for using the kit to identify modulators of TRADE activity. In another embodiment, a kit for carrying out a screening assay of the invention can include an composition comprising a TRADE polypeptide, means for determining TRADE activity and instructions for using the kit to identify modulators of TRADE activity.

In another embodiment, the invention also described herein is a kit for carrying out a modulatory method for the invention. The kit can include, for example, a modulatory agent of the invention (*e.g*., a TRADE inhibitory or stimulatory agent) in a suitable carrier and packaged in a suitable container with instructions for use of the modulator to modulate TRADE activity.

Also described herein is a kit for diagnosing a disorder associated with aberrant TRADE expression and/or activity in a subject. The kit can include a reagent for determining expression of TRADE (*e.g.,* a nucleic acid probe(s) for detecting TRADE mRNA or one or more antibodies for detection of TRADE proteins), a control to which the results of the subject are compared, and instructions for using the kit for diagnostic purposes.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent NO: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### EXAMPLES

The present invention is further illustrated by the following examples which should not be construed as limiting in any way.

### Example 1. Molecular cloning and genetic mapping of TRADE

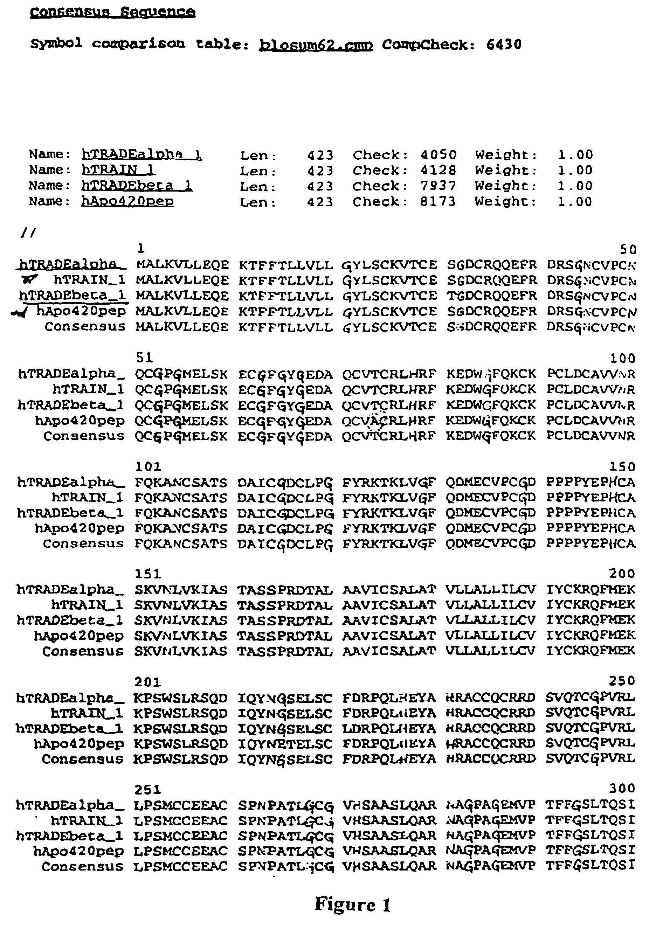

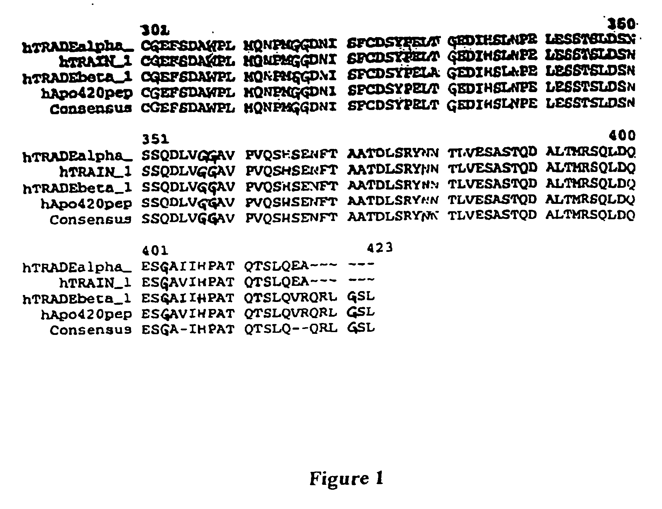

A yeast-based signal sequence trap was used to identify cDNAs encoding secreted proteins from a human bone marrow stromal cell line HAS303 (Jacobs et al., 1997, Gene, 198:289-296). Computational analysis of these cDNAs identified a clone, OAF065, that had homology with the cysteine-rich domains characteristic of the TNF receptor family (Figure 1). Full-length human cDNAs encoding this receptor, later called TRADE, were isolated from HAS303 and HUVEC cells using 3' rapid amplification of cDNA ends (3'-RACE).

Two distinct TRADE cDNAs were identified, TRADEα and TRADEβ. The nucleotide sequence of these cDNAs are identical at the 5' end, but diverge close to the region encoding the final C-terminal amino acids of TRADE (Figure 2). Both TRADEα and TRADEβ have identical putative N-terminal signal sequences of 25 amino acids, mature extracellular region of 168 amino acids and a single transmembrane domain. The extracellular region contains two domains homologous to the cysteine-rich domains of the TNF-R family (Figure 3). Conserved cysteines are boxed and spaces introduced for optimal alignment are indicated with a dot (Figure 3). The second domain is followed by a cysteine rich region that may be an incomplete match to the consensus cysteine rich domain. Cysteine-rich domain, as defined for HMM searches, have been assigned the PFAM Accession PF00020 (http://pfam.wustl.edu). Such an incomplete match is found in some other family members such as TNFRI (Wyllie, 1997, Eur J Cell Biol, 73:189-197) and HVEM (Harrop et al., 1998, J Biol Chem, 273:27548-27556). Additionally, there is a serine/threonine/proline-rich stretch in the extracellular juxtamembrane region, as found in some other family members such as 4-1BB and CD27 (Gravestein et al., 1993, Eur J Immunol, 23:943-950). The intracellular region of TRADEα consists of about 234 amino acids, with no apparent homologies to other TNF family members, including the lack of a death domain, *e*.g. TNF-RI (Kitson et al., 1996, Nature, 384:372-375). The intracellular region of TRADEβ shares this sequence with TRADEα, but diverges from TRADEα by 2 amino acids and has 6 additional amino acids at its C-terminus (Figure 2).

The murine ortholog of TRADE was isolated by hybridization at reduced stringency with a human TRADE cDNA probe labelled with ³²P by random priming. Hybridizations were carried out at 5x SSC, 5x Denhardt's Solution, 0.1% (w/v) SDS, 0.1 mg/ml denatured salmon sperm DNA for 16 hours at 57°C. Washing was performed at 1xSSC, 0.1% (w/v) SDS at 57°C. One cross-hybridizing clone was found in a total of 1X10⁶ λZiplox plaques of a C57/BL6 embryo day 17 cDNA library. The deduced amino acid sequence encoded by this cDNA has 84% identity (87% similarity) to human TRADEα in the mature extracellular and transmembrane regions, and 61 % identity (65% similarity) in the intracellular region (Figure 4). Conserved domains are depicted with bars, including the two cysteine-rich domains (CDR1 and CDR2) as well as a transmembrane domain (TM). A predicted site ofN-glycosylation is shown with an arrow (Figure 4).

Primers that amplify a portion of the 3' untranslated region of the murine TRADE cDNA were used to detect single strand conformation polymorphisms (SSCP) between C57BL/6J and *M. spretus,* a technique that has been described previously (Beier et al., 1992, Proc Nat Acad Sci USA, 89:9102-9106; Beier, 1993, Mammalian Genome, 4:627-631). The murine *trade* gene was found to map to chromosome 14 with a LOD liklihood score of X microsatellite markers. The following pair of primers (A) 5'- dAGGCCATCTTCCTGACGTGGAGGTGTG -3' (SEQ ID NO:7) and (B) 5'- dCGGAATTCGTTTCAGCTCAGCACATTCCAAGGCCG -3' (SEQ ID NO:8) identified a polymorphism between C57BL/6J and *Mus spretus,* and were then used to test DNA of a (C57BL/6J-M. *spretus*) F1 X C57BL/6J backcross. The strain distribution data were analyzed using the Map Manager program (Manley, 1991, Mammalian Genome, 4:30).

### Example 2. Tissue distribution of TRADE mRNA

Human and murine multiple tissue northern blots, from Clontech (Palo Alto, CA) and Invitrogen (San Diego, CA), were probed with human cDNA probes labelled by incorporation of α-³²P-dCTP by random priming. Blots were hybrized in QuikHyb (Stratagene, La Jolla, CA) and then washed twice at room temperature in 2X SSC, 0.1% (w/v) SDS for 30 minutes each, followed by 0.2X SSC, 0.1% (w/v) SDS at 65°C for 30 minutes and exposed to Kodak BioMax MR film (Rochester, NY). Northern blot hybridization analysis revealed a major TRADE mRNA transcript of approximately 4.4kb in poly A+ RNA of adult human heart, lung, spleen, kidney, thymus, bone marrow, ovary, uterus, cervix, placenta, testis, prostate, and pancreas. Analysis of a series of poly A+ RNAs derived from the human gastrointestinal tract revealed a low-level expression in jejunum, ileum, colon, and rectum, and a higher level in the stomach. Furthermore, a very strong northern blot hybridization signal to the 4.4kb TRADE mRNA was detected in human fetal lung and liver, compared with the lower signals in fetal kidney and brain poly A+ RNA. A transcript of 4.4kb was detected in murine polyA+ RNAs from brain, lung, skeletal muscle and liver. Transcripts were also detected in murine embryo RNAs, with relatively higher expression preceding day 15.

*In situ* hybridization was used to identify the cell populations responsible for TRADE expression in adult murine tissue sections and to determine the sites of TRADE expression during murine embryonic development. *In situ* hybridizations to embryonic and adult murine sections of the (129SV X C57/BL6) F₁ strain were performed using sense and antisense ³⁵S-UTP labeled probes (Genome Systems, St. Louis, MO), essentially as described (Lyons et al., 1990, J Cell Biol 111:1465-1476). The TRADE cDNA template used for *in vitro* RNA synthesis consisted of 400 bp of the 3' untranslated region. The hybridization probe employed was a radiolabeled antisense RNA transcript consisting of 400bp of the 3' untranslated region of the murine TRADE cDNA. Sections of murine embryo at embryonic days E9.5, E12.5, and E15.5, as well as adult prostate, lung and brain were analyzed with this probe. Hybridizations with a sense probe control were performed in parallel on serial sections. This sense probe gave a low level background hybridization.

At E9.5, when the embryo has approximately 25 somites, TRADE expression could be detected in the developing nervous system. At this stage, the normal embryo has a distinct head and the subregions of the brain are visibly forming. The TRADE transcripts were localized to the neuroepithelium of the brain, and the dorsal region of the neural tube, where sensory neurons are developing. Transcripts could also be detected in the paraxial mesoderm, in a region adjacent to the neural tube. This is a site of developing muscle.

At E12.5, many of the central nervous system structures seen in the adult begin to differentiate. At this point, expression of TRADE could be observed in the cortex and hippocampal regions of the forebrain, thalamus and colliculus of the midbrain and in the developing optic stalk. Expression could also be detected in the cochlea, tooth buds, jaw, and lung. Expression detected in muscle at this stage could be related to the paraxial mesoderm expression detected earlier at day E9.5. Expression of TRADE could also be observed in the entire ectoderm. Transcripts of TRADE were absent from other brain regions and organs such as the liver.

Consistent with the northern blot results which showed less TRADE expression at E17 relative to E15, there were fewer TRADE transcripts at E15.5 than at the earlier stages examined. Expression of TRADE was observed in the hippocampus and the tooth bud and the bronchi of the lung. As seen at E12.5, TRADE transcripts were absent, or very low, in organs such as the liver, kidney and heart.

In the adult murine prostate section, expression of TRADE could be observed in the glandular epithelium. The adult lung resembled the fetal organ in that TRADE expression was observed to be localized to the epithelium of the bronchi. Expression in the adult brain was very low and appeared restricted to the hippocampus.

Since northern blot analysis had revealed a high level of TRADE expression in the human prostate, immunohistochemical staining of human prostate sections was undertaken with mAbs #8 and #16 (see Example 3). In addition, immunohistochemistry was used to localize the cells responsible for the lower levels of TRADE expression detected by northern blot in the small intestine and liver.

Paraffin embedded human tissue sections were dewaxed in alcohol and endogenous peroxidase blocked with 0.5%(w/v) hydrogen peroxide in methanol. The mAbs were incubated with the sections in Tris-buffered saline solution (TBS) with 10% (w/v) normal swine serum for one hour. For non-liver sections, primary mAb was detected with biotin-conjugated goat anti-mouse/rabbit IgG (Dako) in TBS for 30 minutes. Staining was detected with streptABC complex/horseradish peroxidase (Dako) diluted 1:100 in TBS for 30 min before incubation with DAB for 5 minutes and counterstaining in Mayer's Hematoxlyin. In human liver sections, the secondary step was replaced with peroxidase-rabbit anti-mouse IgG and peroxidase-swine anti-rabbit IgG diluted in TBS with 10% (w/v) normal swine serum.

Eight human prostate specimens, collected by needle biopsy or curetting, were examined after treatment as described above. Four of these were benign prostate glands showing no evidence of malignancy and four contained prostatic adenocarcinoma. Both mAb #8 and # 16 were individually tested and the observed staining was scored from 0 (none) to 3+ (strong/diffuse). The results obtained are shown in Tables 1 and 2. Each mAb gave essentially identical results. A murine IgG1 isotype control gave no specific staining of these specimens. In benign prostate samples, moderate to strong diffuse staining was present in the glandular epithelium of each of the four specimens. Focal immunoreactivity was also found in smooth muscle, and two cases showed weaker endothelial staining. The four prostate cancer specimens gave similar results, with strong, diffuse staining in the glandular epithelium. This signal was stronger, presumably as a result of the malignant proliferation of the adenocarcinoma.

**Table 1: TRADE staining in benign prostatic tissue**

| **CASE NO.:** | **PROSTATIC ACINI** | **MUSCLE** | **VESSELS** | **COMMENT** |
|---|---|---|---|---|
| 1473/99 | 1+ | 2+ | - | Curettings RBCs Positive |
| 1496/99 | 2+ | 2+ | - | Curettings Occasional RBCs positive |
| 2199/99 | 1+ | 1+ | - | Needle biopsies |
| 2208/99 | 1 + | 1 + | - | Needle biopsies |

**Table 2: TRADE staining in prostatic adenocarcinoma**

| **CASE NO.:** | **CARCINOMA ACINI** | **MUSCLE** | **VESSELS** | **COMMENT** |
|---|---|---|---|---|
| 1301/99 | 3+ | 2+ | 1+ | Curettings RBCs positive |
| 1446/99 | 3+ | 1+ | - | Curettings |
| 1028/99 | 2+ | 1+ | +/- | Needle biopsies |
| 1029/99 | 3+ | 1 + | - | Needle biopsies |

Three further specimens were examined: 1) normal liver removed from a donor used for transplantation; 2) primary billiary cirrhosis obtained from a hepatectomy specimen from a transplantation patient with biochemical, serological and histological features of this disease; and 3) hepatocellular carcinoma obtained at surgical resection from a liver with well differentiated carcinoma. Both #8 and #16 mAbs gave similar results with each specimen. In the normal liver, there was intense staining of the bile ducts (3+), moderate panacinar cytoplasmic staining of hepatocytes (2+) and weak endothelial staining (1+) in hepatic arteries, portal veins, and hepatic veins. In the primary billiary cirrhosis, there was also intense staining of bile ducts (3+), and intense panacinar cytoplasmic staining of hepatocytes (3+). In the hepatocellular carcinoma specimen, there was intense staining of tumor cells with both mAbs.

The expression profile of TRADE defines the cellular context in which this receptor has the potential to act. Analysis showed human TRADE expression in various tissues and organs with the highest levels in adult prostate, lung, ovary, and fetal lung and liver. More importantly, immunohistochemistry was used to demonstrate that TRADE is primarily localized to ductal epithelial tissues of the prostate, parotid gland and testis. The strong staining observed for the bile duct may also be due to epithelial expression of TRADE. *In situ* hybridization was used to show that the predominant sites of murine TRADE expression in the adult prostate is also the glandular epithelium, and in the adult lung it is the bronchial epithelium. Furthermore, the TRADE gene is also expressed in distinct sites of the embryo, including the lining of the developing airway in the fetal lung, and in the developing nervous system. The recent positional cloning of a TNF-R family member, Ectodermal dysplasia receptor, loss of which is responsible for the defects in hair follicle specification found in the murine *downless* mutants (Headon and Overbeek, 1999, Nature Genetics, 22:370-374), underlies the importance of this receptor family in the developmental process.

Furthermore, TRADE was found in primary adenocarcinomas arising from ductal epithelial cells in the prostate and in adenocarcinoma cell lines. TRADE, CD40 and p75^{NGFR} are each expressed in epithelial cells (Delsite and Djakiew, 1999, Prostate, 41:39-48; Young et al., 1998, Immunology Today, 19:502-506). This may underline a novel physiological role for the TNF receptor family in this cell type. Expression of the p75^{NGFR} has been specifically described in prostate epithelial cells (Delsite and Djakiew, 1999, Prostate, 41:39-48). However, unlike TRADE, p75^{NGFR} expression has been reported to be lost in malignant specimens and it is not expressed in metastatic tumor lines derived from the prostate. The growth inhibition mediated by the CD40 ligand on tumor cells may have therapeutic value (Hirano et al., 1999, Blood, 93:2999-3007). Likewise, the expression of TRADE and its ability to induce apoptosis may open new approaches to the treatment of adenocarcinomas.

### Example 3. Immunochemical analysis of TRADE

A panel of twenty murine monoclonal antibodies (mAb) specific for the TRADE extracellular domain were prepared for analyzing TRADE protein expression.

Female BALB/c mice, 8-10 weeks old, were immunized using a gene gun bombardment technique (Barry et al., 1994, BioTechniques, 16:616-620) using 3 µg of a plasmid vector that expresses human TRADE from a cytomegalovirus immediate early promoter. Mice were re-inoculated 5-6 times at two-week intervals with the same plasmid DNA. Three days prior to fusion, mice were boosted intrasplenically with 10 µg of TRADE-Fc, a purified fusion protein consisting of the extracellular region of human TRADE fused to the hinge-CH2-CH3 domains of human IgG1. Spleen cells from immunized mice were fused with P3X63Ag8.653NS1 myeloma cells (ATCC, Rockville, MD) using conventional hybridoma techniques. Fused cells were cultured in HAT containing RPMI 1640 hybridoma selection medium for two weeks.

The hybridoma culture supernatants were screened by ELISA for binding to immobilized TRADE-.Fc and not other Fc fusion proteins. The antigen specific hybrid cells were subcloned by ClonaCell-HY hybridoma selection medium (Stem Cell Technologies, Vancouver, BC) and adapted to grow in ascites. An affinity column with immobilized protein A (Pierce, Rockford, IL) was used to purify monoclonal antibody from ascites fluids. Antibody class and subclass were tested by using Mouse Hybridoma Subtyping kit as per manufacturer's instructions (Boehringer Mannheim, Indianapolis, IN).

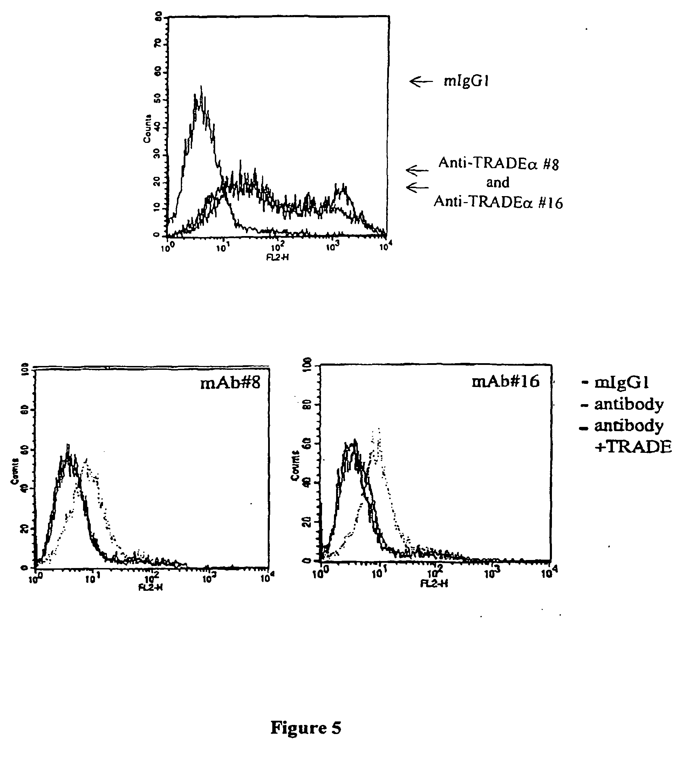

Three IgG1 mAbs appeared to give the strongest cell surface staining signals and were used in the experiments described. The specificities of these three mAbs, #8, #12 and #16, were tested by cell surface staining of transiently transfected COS cells expressing TRADE. As shown in Figure 5 (top), shows human TRADEα-transfected COS cells that were stained with mIgG1 as a control, anti-TRADEα #8, and anti-TRADEα#16. Both anti-TRADEα #8 and anti-TRADEα #16 stain the cells. The IgG1 mAbs #8 and #16 bound the surface of human TRADEα expressing COS cells. No binding was detected of any anti-TRADE mAbs to mock transfected cells. The same results were obtained with TRADEβ expressing cells. Additionally, binding of mAbs #8 and #16 was lost by co-incubation with TRADE-Fc, a soluble form of the TRADE extracellular region fused to human IgG1, thus establishing that the mAbs #8 and #16 mAbs bind specifically to the extracellular domain of human TRADE.

The #8 and #16 mAbs were used to screen human cell lines for TRADE expression by flow cytometry. Cell lines were obtained from the American Type Culture Collection (Rockville, MD). Cells were stained with anti-TRADE or isotype matched control monoclonal antibodies at 10 µg/ml. Binding of primary antibody was detected with goat F(ab')₂ anti-murine IgG conjugated to biotin, followed by streptavidin-phycoerythrin (Southern Biotechnology Associates, Birmingham, AL). Cells were analyzed with aBecton-Dickinson FACScan (San Jose, CA).

Three cell lines were each found to bind anti-TRADE mAbs: a prostatic adenocarcinoma, PC-3; an astrocytoma, U373 MG (Figure 5, bottom panels, dotted lines); and a colonic adenocarcinoma, CaCo2. Specifically, the bottom panels show the results of treatment of a human astrocytoma cell line with both antibodies in the presence and absence of TRADE-Fc fusion protein. The dotted lines represent the anti-TRADEα #8 and #16 (bottom left and bottom right panels, respectively). The solid lines represent the control mIgG1 and the antibody (either #8 or #16) in the presence of TRADE-Fc fusion protein. Specificity was confirmed by competing away the FACS staining by using excess soluble TRADE-Fc fusion protein as before (Figure 5, bottom panels, solid lines). The expression of TRADE in each of these cell lines was also confirmed by RT-PCR using TRADE specific primers. Two other prostate tumor cell lines, LNCaP.FGC and DU145, as well as other colon tumor lines, HCT116 and HT-29 were negative for TRADE expression by flow cytometry with these mAbs. Transiently transfected COS cells were analyzed after 48 hours for heterologous expression of TRADE. In order to radiolabel TRADE, monolayers of cells in 100mm tissue culture dishes, were starved for 15 minutes in medium without methionine or cysteine, followed by addition of Pro-mix L-³⁵S (Amersham), to a final concentration of 300 µCi/ml. After one hour at 37°C, the medium was replaced with fresh medium containing unlabelled methionine and cysteine. The cell monolayers were solubilized in ice-cold 1% (w/v) Nonidet P-40, 0.1 % (w/v) SDS, 0.25% (w/v) sodium deoxycholate, 25 mM Tris-HCl pH 7.5, 150 mM sodium chloride with Complete (Boehringer-Mannheim, Indianapolis, IN) proteinase inhibitor mix.

Immunoprecipitation was performed with 10 µg/ml of mAb (either #16 or #12) overnight at 4°C, followed by addition of goat anti-murine IgG-Sepharose (Zymed, San Diego, CA) for 2 hours at 4°C. Immunoprecipitates were examined by reducing PAGE followed by treatment with AmplifyAmersham, Arlington Heights, IL) and fluorography. This analysis revealed a protein species of the expected size of approximately 55,000 Mr from extracts of cells expressing TRADE and not in extracts of mock transfected cells. Digestion of an immunoprecipitate with PNGase F resulted in an increase in mobility of the TRADE polypeptide relative to undigested duplicate samples. Thus, it appears that TRADE is N-glycosylated at the single consensus site at residue N105

### Example 4. NFkB activation and JNK activation by ectopic expression of TRADE

Several TNF receptor family members have been shown to be potent activators of cell survival signaling pathways (Gravestein and Borst, 1998, Seminars in Immunology 10:423-434; Warzocha and Salles, 1998, Leukemia & Lymphoma 29:81-92). Both the NFkB and JNK signaling pathways are implicated in the cytoprotective and inflammatory effects of the TNF family (Wallach et al., 1999, Ann Rev of Immunology, 17:331-367). The transcription factor NFkB, once activated, enters the nucleus and activates transcription from several key genes involved in cell survival and proliferation checkpoints (Karin, 1998, Cancer J from Scientific American, 4:92-99). The JNK kinase phosphorylates critical serine residues in the activation domain of c-Jun, a component of the AP 1 transcription factor complex (Ui et al., 1998, FEBS Letters, 429:289-294).

In the absence of an identified ligand to use as an agonist, the observation that overexpression of a wide range of receptors leads to constitutive activation could be exploited (Chinnaiyan et al., 1996, Science, 274:990-992). Higher levels of cell surface expression of the receptor presumably lead to receptor oligomerization in a manner that mimics ligand-induced receptor activation. Lower levels of cell surface expression of the receptor, on the other hand, are presumably insufficient to cause receptor oligomerization in the absence of ligand and are thus, inadequate in initiating signaling events.

In order to assess TRADE-mediated activation of NFkB and JNK pathways, human embryonic kidney 293, and 293T cells, HeLa cells, and COS-1 (clone M6) cells were cultured in appropriate media (as recommended by ATCC). COS-1 (clone M6) cells were transfected with plasmid DNA using LipofectAMINE (Gibco BRL). 293 and 293T cells were transiently transfected with the indicated expression and reporter plasmids using the calcium phosphate co-precipitation of DNA transfection method. HeLa cells were transiently transfected with the indicated plasmids and pCMVβgal plasmid (Clontech), using the LipofectAMINE Plus (GibcoBRL) reagent as per manufacturer's instructions. Cells were harvested 18-36 hours following transfections depending upon the assay performed. The TRADE cDNA was subcloned into the adenovirus major late promoter driven expression plasmid pED (Kaufman, R.J. et al., 1991, Nucl. Acids Res. 19:4485), and into the CMV promoter driven expression plasmid pcDNA3 (commercially available from Invitrogen, Inc. San Diego, CA).

The indicated expression plasmids, a luciferase gene driven by an NFkB binding site-containing promoter (Stratagene) and the pCMVβgal plasmid (Clontech) were used in the NFkB activation assay. The cells were harvested 36 hours after transfection, lysed and assayed for luciferase activity using a luciferase substrate (Promega) as per manufacturer's instructions. The assayed luciferase activity was then adjusted for transfection efficiency by assaying β-galactosidase activity and reported as relative luciferase activity. For JNK activation assay, the indicated expression plasmids were co-transfected with the c-jun trans reporter and transactivator plasmids from Stratagene along with the pCMVβgal plasmid (Clontech). Luciferase assays were done as for the NFkB assays and reported as relative luciferase activity after adjusting for transfection efficiency as above. All experiments were performed in triplicates and results were reproduced at least three times.

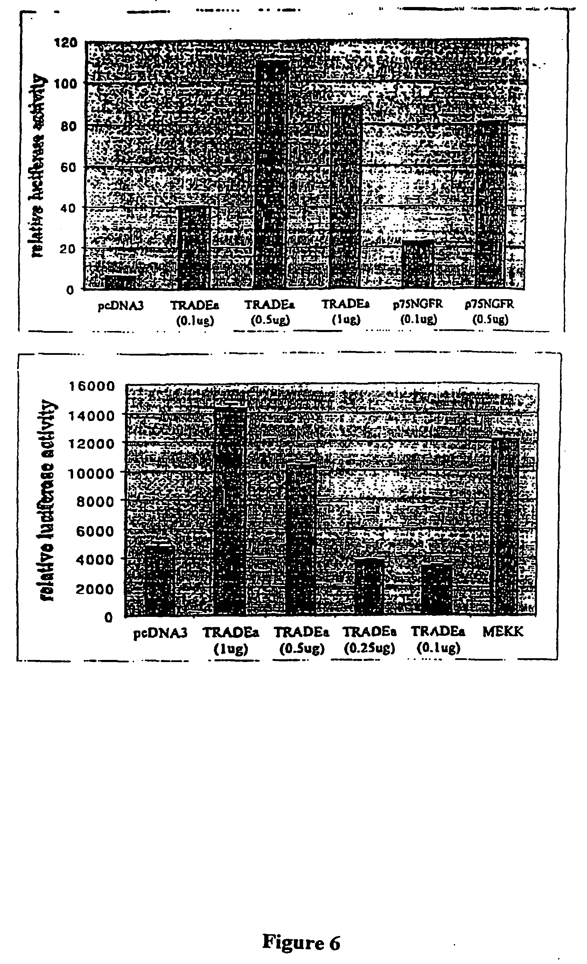

By overexpressing TRADEα or TRADEβ along with an NFkB driven luciferase reporter construct in 293T cells, the effect of TRADE expression on the NFkB activation pathway could be analyzed. The top panel of Figure 6 shows that human TRADEα and p75 ^{NGFR} were able to activate the NFkB signaling pathway at comparable levels. Human TRADEα expression plasmid, or p75^{NGFR} expression plasmid or vector alone were co-transfected with the luciferase reporter plasmid (0.5ug) and pCMVβgal (O.lug). Cells were harvested and relative luciferase activity was quantitated as described in Experimental procedures 36 hrs post transfection. The same levels of NFkB activation have been observed for TRADEβ.

Previous study of p75^{NGFR} has focused mainly on its role in neuronal cells. However, it is expressed in a range of other cells, including lymphocytes (Barker, 1998, Cell Death & Differentiation, 5:346-356). The activation of NFkB by TRADE was compared in parallel with that induced by p75^{NGFR}. TRADEα signals NFkB activation to modest, yet comparable levels as the signaling observed from overexpressing p75^{NGFR} (Figure 6, top). In its DNA binding and transcription activating function, the levels of activated NFkB may have been adequate to achieve the physiological effects of p75^{NGFR}. The NFkB activation response induced by p75^{NGFR} may be limited to conditions of cellular stress (Barker, 1998, Cell Death & Differentiation, 5:346-356).

Similar to TRADE, NFkB activation by p75^{NGFR} has been reported to be modest in comparison with other TNF receptor family members (Barker, 1998, Cell Death & Differentiation, 5:346-356), and this signal is mediated by TRAF6 (Khursigara et al, 1999, J of Biol Chemistry, 274:2597-2600). The p75^{NGFR} also stimulates apoptosis, and a novel zinc finger containing protein, NRIF (neurotrophin receptor interacting protein) mediates this signal (Casademunt et al., 1999, EMBO Journal, 18:6050-6061). NRIF binds two motifs in the intracellular region of p75^{NGFR}, at the juxtamembrane region which has been shown to be the TRAF6 binding domain and the death domain. This has been suggested to occur by a cooperative interaction or as a dimer, that is again structurally very different from the death domain/death domain interaction between a TNF receptor and a death domain containing signaling factor. The p75^{NGFR} has a structurally variant death domain, based on the homology in the first α-helix and the spacing differences described for the other helices (Barker, 1998, Cell Death & Differentiation, 5:346-356). This feature has been suggested to disallow the aggregation between the intracellular domains as are found in Fas and TNF-RI.

A trans reporter assay system, which uses a fusion protein containing the GAL4 DNA binding domain fused to the c-Jun transcriptional activator, was employed for assaying JNK activation. Specifically, human TRADEα expression plasmid (in the amounts indicated in Figure 6), or MEKK expression plasmid, or vector alone was co-transfected with the luciferase reporter constructs, c-jun transactivator plasmid and pCMVβgal. Cells were harvested and analyzed 36hrs post transfection as above. In this case, luciferase reporter gene expression is driven by a promoter containing GAL4 protein binding sites. Thus, basal expression from the reporter gene is compared with transcription induced as a result of c-Jun phosphorylation. It was found that overexpression of TRADEα led to increased luciferase activity as a result of JNK activation (Figure 6, bottom). This activation of the JNK signaling pathway was found to be dose dependent. JNK activation has been compared alongside the positive control MEKK, the MAP kinase kinase upstream of JNK in the MAP kinase cascade that leads to JNK phosphorylation (Xu and Cobb, 1997, J of Biol Chemistry, 272:32056-32060). TNF receptor induced JNK activation has been shown to be mediated primarily by the TNF receptor associated factor, TRAF2 (Lee et al., 1997, Immunity, 7:703-713).

JNK activation is implicated in cell death of sympathetic neurons, by a report that shows nerve growth factor deprivation induced apoptosis is abrogated by dominant negative c-Jun (Ham et al., 1995, Neuron, 14:927-939). Indeed, the neurotrophin receptor TrkA mediated rescue from p75^{NGFR} induced cell death, correlates with inhibition of p75^{NGFR} induced JNK activation, while not affecting p75^{NGFR} induced NFkB activation (Yoon et al., 1998, J of Neuroscience 18:3273-3281).

In the case of p75^{NGFR}, the decision between NRIF binding or TRAF6 binding may determine whether the receptor will signal death or survival. The outcome of this decision may depend upon other signals received by a cell. CD40 has been widely studied for its role in immune activation (Grewal and Flavell, 1998, Ann Review of Immunology, 16:111-135; Laman et al., 1998, Developmental Immunology, 6:215-222; Mackey et al., 1998, J of Leukocyte Biology, 63:418-428; Toes et al., 1998, Seminars in Immunology, 10:443-448). However, CD40 ligation signals growth inhibition on epithelial cells (Eliopoulos et al, 1996, Oncogene 13:2243-2254), and apoptotic cell death on transformed cells of mesenchymal and epithelial origin (Hess and Engelmann, 1996, J of Experimental Medicine, 183:159-167). A dominant negative version of TRAF3 blocked the CD40 induced growth inhibitory signal (Eliopoulos et al., 1996, Oncogene 13:2243-2254), implicating TRAF3 as a mediator of the CD40 induced epithelial growth inhibition. TRADE does not contain a death domain and therefore, it resembles family members such as CD40 and CD30 which also do not have a death domain but can induce apoptosis in specific cellular contexts (Grell et al., 1999, EMBO Journal, 18:3034-3043; Horie and Watanabe, 1998, Seminars in Immunology, 10:457-470). The two isoforms differ in C terminal eight amino acid residues 416 to 423, such that TRADEα has 417 amino acid residues and TRADEβ has 423 amino acid residues. Moreover, the precise cellular expression context of the two isoforms may define the specific cell fates.

### Example 5. TRADE-induced cell death

Several TNF receptor family members have been demonstrated to activate both survival and death signaling pathways (Casaccia-Bonnefil et al., 1999, Microscopy Research & Technique, 45:217-224; Wallach et al., 1996, Ann Rev of Immunology, 17:331-367). Therefore, experiments were designed to see if TRADE overexpression resulted in cell death signaling, as has been described for some TNF receptor family members that do not contain a conserved death domain.

Cells death assays were performed either 15hrs, 18hrs, or 24 hrs, post transfection with the indicated expression plasmids and the pCMVβgal plasmid (Clontech). The cells were fixed with 0.5% glutaraldehyde in PBS and stained using the chromogenic substrate Xgal (Sigma) for 5-12 hrs. Transfected cells stained positive for βgalactosidase expression and a representative population of live and dead cells were counted in triplicates using phase contrast microscopy. At least 400 β-galactosidase positive cells were counted for each transfection (n=3) and identified as apoptotic cells or non-apoptotic cells based on morphological alterations typical of adherent cells undergoing apoptosis including membrane blebbing, becoming condensed, and detaching from the plate surface. Percent apoptosis was calculated by dividing the number of cells undergoing apoptosis with the total number of β-galactosidase positive cells.

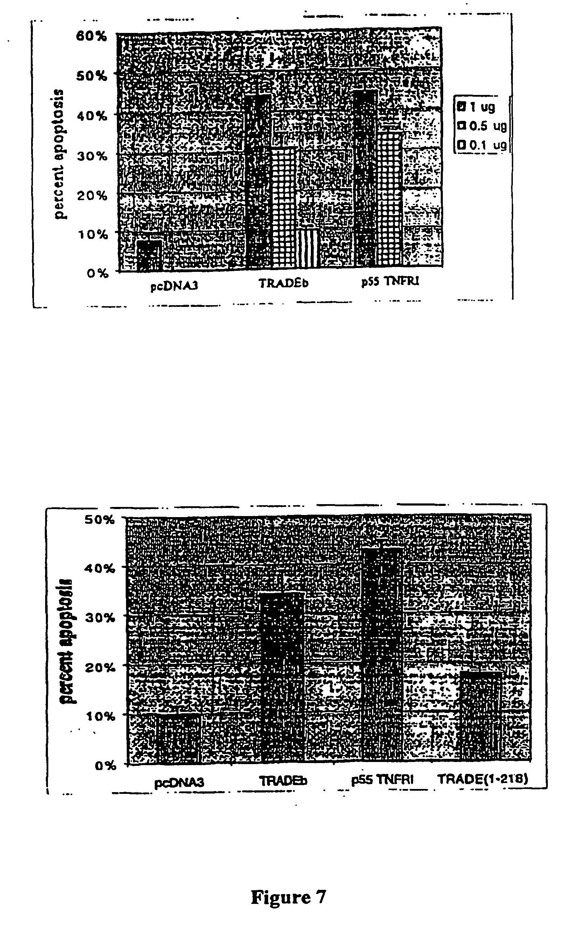

TRADE was overexpressed in HeLa cells along with βgalactosidase, which offered the ability to evaluate cell death in the transfected cells by studying the morphology of the transfected cells after X-gal staining. Cells undergoing programmed cell death display a blebbed surface morphology and condensed nucleus, as well as a rounding away from the plate surface before detaching completely. This is very distinct from the morphology of viable cells which have an extended structure, are attached firmly, and have no apparent nuclear condensation. Apoptosis was evaluated by counting representative fields of X-gal stained cells as dead or alive based on the morphology displayed. Upon calculating percent apoptosis in HeLa cells expressing either TRADEα or TRADEβ, significant cell death was observed. Specifically, TRADEα expression in HeLa cells resulted in programmed cell death in a dose dependent manner comparable to TNF-RI (Figure 7, top panel).. Indicated amounts of the expression plasmids or vector alone were co-transfected with pCMβgal plasmid and the cell death assays were performed as described in Experimental procedures. This experiment represents cell death seen 15 hours post transfection. The level of apoptosis was proportional to the amount of TRADE expressing DNA used, which presumably correlates with the achieved level of TRADE activation. It was observed that TRADE induces apoptosis occurs as early as 15 hrs post transfection. This did not significantly vary at 18 hours and remained at 24 hours post transfection. An apoptotic effect of TRADE comparable to that of TNF-RI and p75^{NGFR}, has also been evidenced upon expression in 293 cells. A severe deletion in the TRADE intracellular domain that retains only the membrane proximal 100 amino acids, TRADE(1-218), significantly attenuates the death signal (Figure 7, bottom). This indicated that the TRADE intracellular domain, residues 218-423, has a critical contribution to the death effector function of TRADE.

### Example 6. Construction of soluble TRADE-Fc fusion protein

A soluble TRADE-Fc fusion protein was constructed by joining the cDNA sequence encoding the extracellular region of TRADE to the hinge-C_{H}2-C_{H}3 regions of human immunoglobulin (Ig) Fc γ1, γ2, γ3, ε or α. PCR primers based on the nucleotide sequence of the extracellular domain of human TRADE were used to generate a fragment of the complete TRADE extracellular region. A XbaI site was incorporated at the 3' end, such that the fragment could be ligated to a plasmid vector containing the human Ig γ1 hinge-C_{H}2-C_{H}3 cDNA. This construct was based on pED (Kaufman, R.J. et al., 1991, Nucl. Acids Res. 19:4485), and contained an Adenovirus major late promoter and SV40 enhancer directing transcription of the TRADE-Fc fusion protein. An SV40 origin permited replication in COS-1(clone M6) cells. This vector also included an EMC-DHFR cassette for stable selection and amplification of the plasmid in CHOdhfr cells using methotrexate.

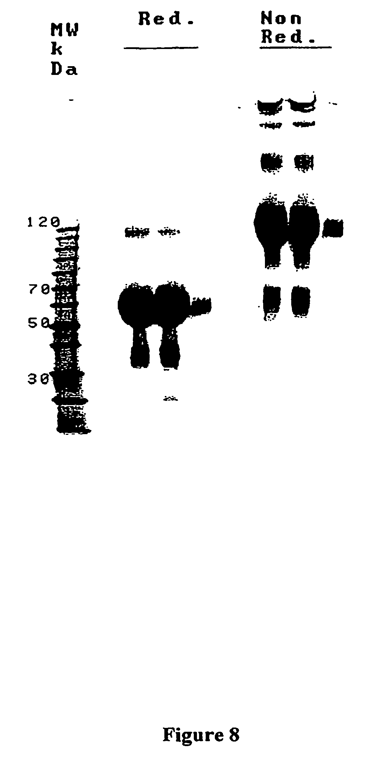

COS cells were transfected with this plasmid, cultured, and conditioned medium harvested. The fusion protein was purified using a column of immobilized protein A. Figure 8 shows SDS-PAGE analysis of elution fractions from the protein A column, in reduced and non-reduced conditions. The gel was stained with Coomassie Blue. In reducing conditions, a diffuse species of 50-60,000 Mr was noted, representing the TRADE-Fc monomer. In non-reducing conditions, a species of approximately 120,000 Mr was noted, illustrating the disulphide-linked dimer form of TRADE-Fc. This dimeric form is expected to be a potent, soluble antagonist of the TRADE ligand.

### Example 7. TRADEα and TRADEβ associated kinase activity

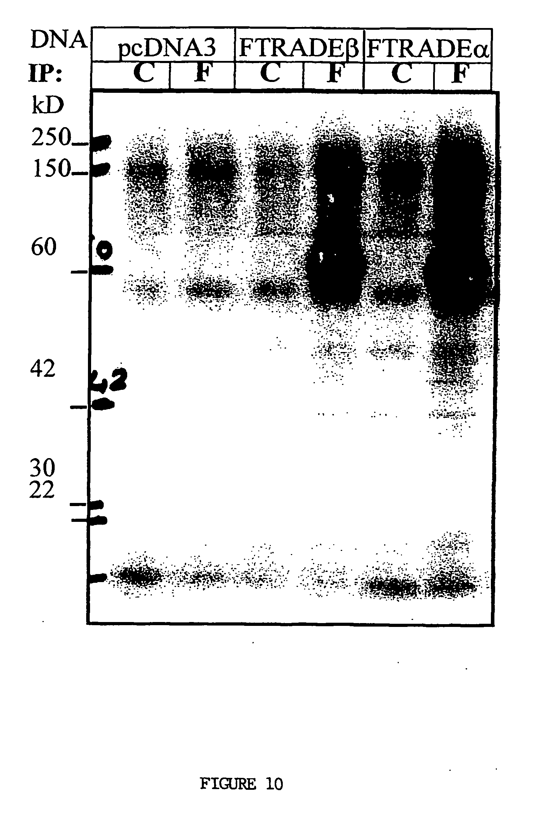

Other members of the of the TNF Receptor superfamily have been reported to be associated with members of the serine-threonine kinase family as components in their signaling pathways. To address whether TRADEα and TRADEβ associated with a kinase, TRADE associated kinase activity was analyzed by subjecting TRADE immunoprecipitated complexes to *in vitro* kinase assays. Specifically, cDNAs encoding Flag-tagged proteins (or vector control) were expressed in 293T cells and lysates immunoprecipitated using anti-Flag antibody or control antibody. The immune-complexes were subjected to kinase assays using ³²P labelled ATP and examined by SDS-PAGE. The gels were dried and analyzed by autoradiography. The results indicated that both isoforms are phosphorylated by an associating kinase activity (Figure 10).

### Example 8. Deletion analysis of TRADE and the associated kinase function.

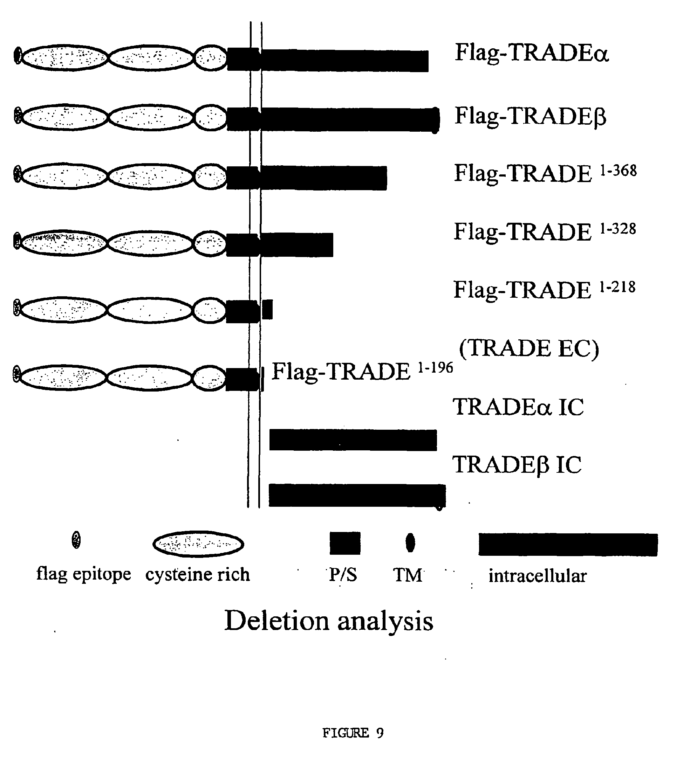

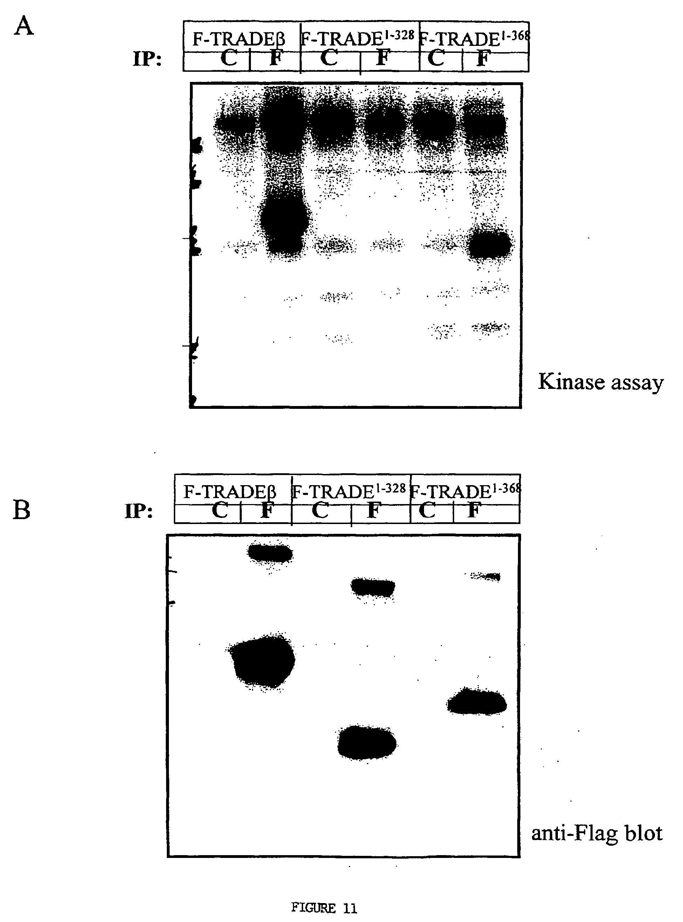

To map which domain is essential to associated kinase activity, various deletion constructs were developed and used in the biochemical and functional analysis of TRADE. A schematic diagram of the deletion constructs used in the TRADE biochemical analysis are depicted in Figure 9. *In vitro* kinase assays using deletion constructs TRADE¹⁻³⁶⁸ (i.e., consisting of amino acids 1-368) and TRADE¹⁻³²⁸ show that deletion construct TRADE¹⁻³⁶⁸ retains kinase associating function while deletion construct TRADE¹⁻³²⁸ loses kinase function (Figure 11A). This result shows that associated kinase function maps to an internal domain within the TRADE intracellular region since deleting a section stretching from the C terminal end to amino acid residue 328 abolishes the associated kinase activity. Figure 11B is a western blot of the immunoprecipitates used in Figure 11 A showing equivalent expression of all constructs.

### Example 9. TRAF6, but not TRAF2, bind TRADEα and TRADEβ.

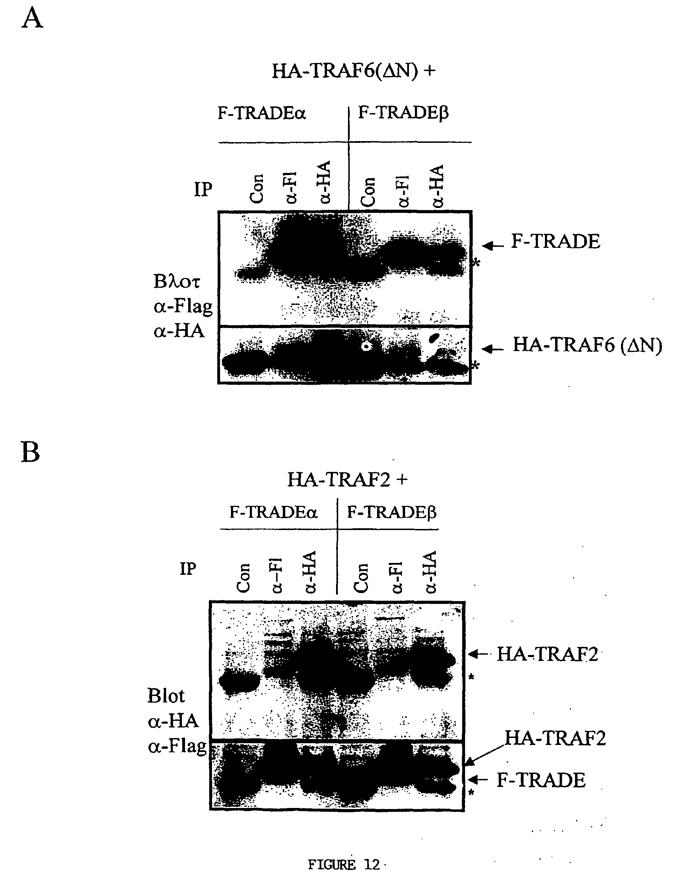

Signaling activities of members of the TNF receptor superfamily are mediated by binding TRAF intracellular adaptor molecules. To assess whether these molecules associate with TRADE, HA-TRAF6ΔN and the respective Flag tagged TRADE constructs were coexpressed in 293T cells. The cell lysates were divided equally for control, anti-HA, and anti-Flag immunoprecipitations and western blotted with anti-Flag (Figure 12A, upper panel) followed with anti-HA (Figure 12A, lower panel). These results show that TRADEα and TRADEβ bind TRAF6 and TRAF6, but do not bind TRAF2 (Figure 12A and B). The asterisk designates either Immunoglobulin (Ig) heavy chain (Figure 12A, upper panel) or Ig lower chain (Figure 12A, lower panel). Flag-tagged TRADE proteins coprecipitaing with HA-TRAF6ΔN in the anti-HA immunocomplexes are shown in upper panel of Figure 12A.

HA-TRAF2 and the respective Flagg-tagged TRADE constructs were coexpressed and analyzed for association as mentioned above. Specifically, HA-TRAF2 did not coprecipitate with the Flag tagged TRADE proteins (Figure 12B, upper panel). Figure 12B, lower panel is a Western blot showing the appropriate and equivalent expression levels of the constructs used. The asterisk in both panels designates Ig heavy chain present in the immune complex.

### Example 10. TRAF3 binds TRADE.

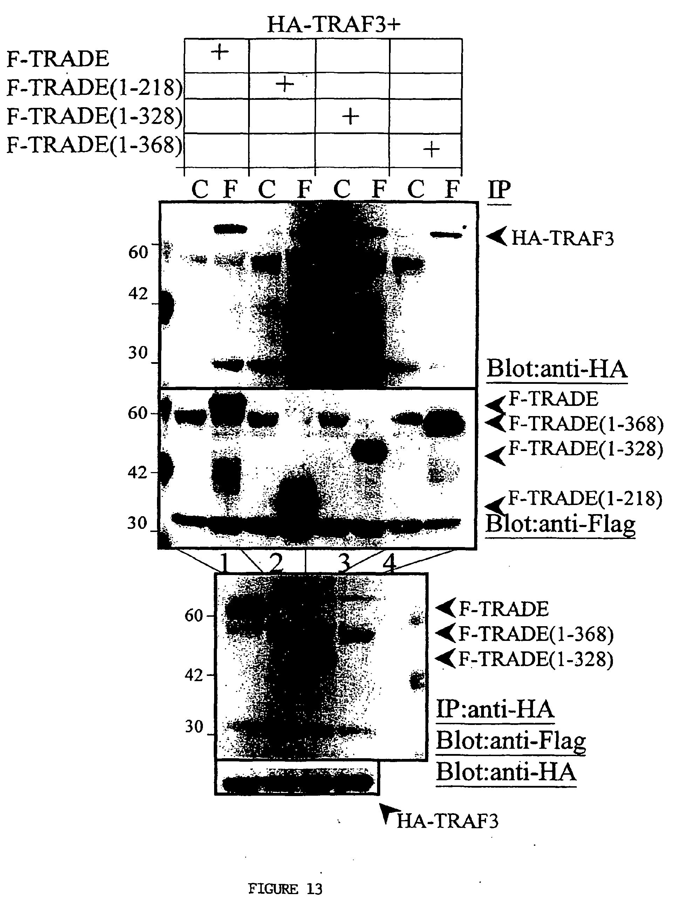

TRADEα and TRADEβ also bind TRAF3. Cell lysates from 293T cells expressing cDNAs of the designated Flag tagged TRADE constructs and HA-TRAF3 were split equally for three immunoprecipitations - control (C) and anti-Flag (F). Both TRADE isoforms bound TRAF3, with the upper panels showing the anti-HA and anti-Flag bolts, respectively (Figure 13, upper panel).

Analysis of TRADE binding with deletion mutants demonstrates that the HA-TRAF3 construct fails to coprecipitate with deletion construct TRADE¹⁻²¹⁸, while successfully associating with full length TRADE, deletion construct TRADE¹⁻³⁶⁸ and deletion construct TRADE¹⁻³²⁸ (Figure 13, lower panel). These results suggest that the TRAF3 binding site on TRADE requires the intracellular domain to amino acid residue 328, as evidenced by the failure of deletion construct TRADE¹⁻²¹⁸ to bind TRAF3 (Figure 13, lower panel). The lower two panels show the anti-Flag and anti-HA blots, respectively.

### Example 11. Deletion analysis of TRADE and the NFkB activation signal.

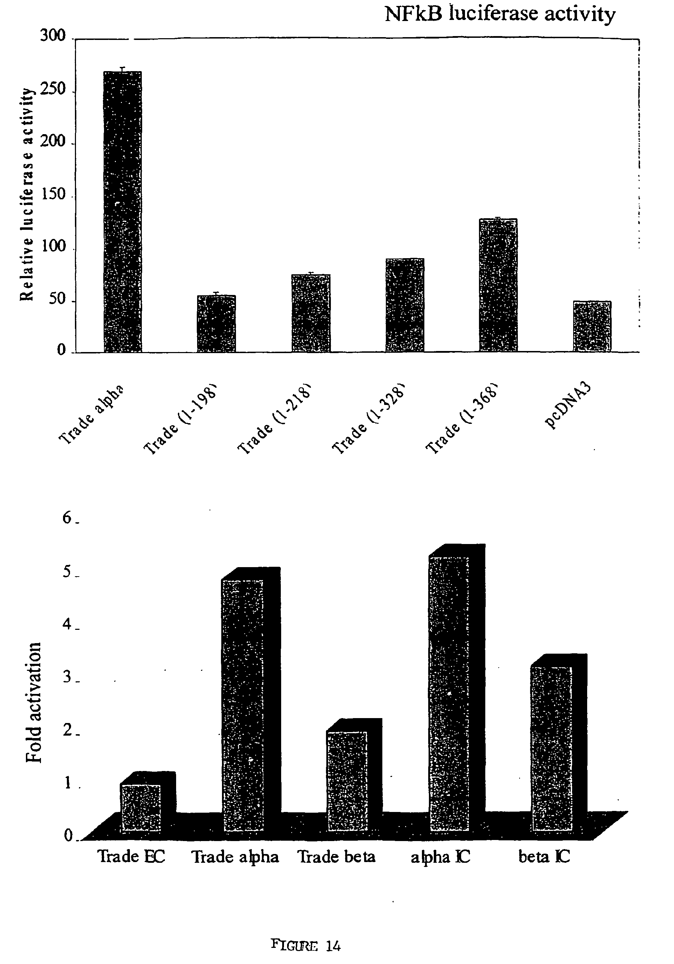

The importance of the intracellular domain on TRADE for signaling is defined by functional analysis using the various deletion constructs in the NFkB promoter driven luciferase assay. The designated TRADE constructs were coexpressed with the NFkB promoter driven luciferase reporter construct (and CMV driven β-galactosidase construct) in 293T cells and assayed for luciferase activity 36 hours post-transfection. The relative luciferase activity was calculated with respect to β-galactosidase activity to control for transfection efficiency. Deleting the C terminus to amino acid residue 368 (regions associated with kinase function and the TRAF3 binding domain) significantly attenuated the NFkB activating signal (Figure 14A). Further deleting the intracellular amino acid residues lead to complete loss of NFkB activity. The NFkB activation signal from the TRADEα and TRADEβ intracellular domains (α IC and β IC) relative to the TRADE EC are shown in Figure 14B. NFkB promoter driven luciferase activity was assayed in response to the designated expression constructs and plotted as fold activation in comparison with TRADE extracellular domain (TRADE EC). Therefore, the NFkB signaling function resides entirely in the intracellular domain of TRADEα and TRADEβ. These results suggest a potential signaling role for the kinase activity and TRAF binding within the intracellular TRADE sequence.

## Claims

1. The use of an agent in the manufacture of a medicament for treating neoplasia, wherein the agent is a soluble form of a TRADE polypeptide comprising the extracellular domain of a protein according to SEQ ID NO:2 or SEQ ID NO:4.

2. The use of claim 1, wherein the neoplasia is neoplasia of a cell selected from the group consisting of an epithelial cell, a ductal epithelial cell, or a bronchial epithelial cell.

3. The use of claim 1, wherein the neoplasia is neoplasia of a cell which is a carcinoma or an adenocarcinoma cell.

4. The use of claim 1, wherein the neoplasia is neoplasia of a cell selected from the group consisting of: a lung cell, a liver cell, a brain cell, and a prostate cell.

5. The use of claim 1, wherein the soluble form of the TRADE polypeptide is a TRADE-Fc fusion protein.

6. The use of claim 1, wherein the agent consists essentially of a TRADE polypeptide extracellular domain of SEQ ID NO:2 or SEQ ID NO:4.

7. The use of claim 1, wherein the neoplasia is a carcinoma.

8. The use of claim 1, wherein the neoplasia is present in lung or prostate tissue.

9. The use of claim 1, wherein the neoplasia is an adenocarcinoma.

## Patentansprüche

1. Verwendung eines Agens bei der Herstellung eines Medikaments zum Behandeln von Neoplasie, wobei es sich bei dem Agens um eine lösliche Form eines TRADE-Polypeptids handelt, das die extrazelluläre Domäne eines Proteins gemäß SEQ ID NO: 2 oder SEQ ID NO: 4 umfasst.

2. Verwendung nach Anspruch 1, wobei es sich bei der Neoplasie um Neoplasie einer Zelle handelt, die aus der Gruppe bestehend aus einer Epithelzelle, einer Gangepithelzelle oder einer Bronchialepithelzelle ausgewählt ist.

3. Verwendung nach Anspruch 1, wobei es sich bei der Neoplasie um Neoplasie einer Zelle handelt, bei der es sich um eine Karzinom- oder eine Adenokarzinomzelle handelt.

4. Verwendung nach Anspruch 1, wobei es sich bei der Neoplasie um Neoplasie einer Zelle handelt, die aus der Gruppe bestehend aus: einer Lungenzelle, einer Leberzelle, einer Hirnzelle und einer Prostatazelle ausgewählt ist.

5. Verwendung nach Anspruch 1, wobei es sich bei der löslichen Form des TRADE-Polypeptids um ein TRADE-Fc-Fusionsprotein handelt.

6. Verwendung nach Anspruch 1, wobei das Agens im Wesentlichen aus der extrazellulären Domäne von SEQ ID NO: 2 oder SEQ ID NO: 4 eines TRADE-Polypeptids besteht.

7. Verwendung nach Anspruch 1, wobei es sich bei der Neoplasie um ein Karzinom handelt.

8. Verwendung nach Anspruch 1, wobei die Neoplasie in Lungen- oder Prostatagewebe vorliegt.

9. Verwendung nach Anspruch 1, wobei es sich bei der Neoplasie um ein Adenokarzinom handelt.

## Revendications

1. utilisation d'un agent dans la fabrication d'un médicament destiné au traitement de la néoplasie, dans laquelle l'agent est une forme soluble d'un polypeptide TRADE comprenant le domaine extracellulaire d'une protéine selon SEQ ID N° 2 ou SEQ ID N° 4.

2. Utilisation selon la revendication 1, dans laquelle la néoplasie est une néoplasie d'une cellule choisie dans le groupe formé par une cellule épithéliale, une cellule épithéliale ductale ou une cellule épithéliale bronchique.

3. Utilisation selon la revendication 1, dans laquelle la néoplasie est une néoplasie d'une cellule qui est une cellule de carcinome ou d'adénocarcinome.

4. Utilisation selon la revendication 1, dans laquelle la néoplasie est une néoplasie d'une cellule choisie dans le groupe formé par : une cellule du poumon, une cellule du foie, une cellule du cerveau et une cellule de la prostate.

5. Utilisation selon la revendication 1, dans laquelle la forme soluble du polypeptide TRADE est une protéine de fusion TRADE-Fc.

6. Utilisation selon la revendication 1, dans laquelle l'agent est constitué essentiellement d'un domaine extracellulaire de polypeptide TRADE de SEQ ID N° 2 ou SEQ ID N° 4.

7. Utilisation selon la revendication 1, dans laquelle la néoplasie est un carcinome.

8. Utilisation selon la revendication 1, dans laquelle la néoplasie est présente dans un tissu pulmonaire ou prostatique.

9. Utilisation selon la revendication 1, dans laquelle la néoplasie est un adénocarcinome.


### SEQUENCE LISTING

<110> Wood, Clive
   Chaudhary, Divya
   Long, Andrew
<120> TRADE MOLECULES, AND USES RELATED THERETO
<130> GNN-012CP
<140>
   <141>
<150> 60/181,922
   <151> 2000-02-11
<150> 60/182, 148
   <151> 2000-02-14
<160> 10
<170> PatentIn Ver. 2.0
<210> 1
   <211> 1660
   <212> DNA
   <213> Homo sapiens
<220>
   <221> CDS
   <222> (1)..(1251)
<400> 1
   atg gct tta aaa gtg cta cta gaa caa gag aaa acg ttt ttc act ctt    48
   tta gta tta cta ggc tat ttg tca tgt aaa gtg act tgt gaa tca gga    96
   gac tgt aga cag caa gaa ttc agg gat cgg tct gga aac tgt gtt ccc   144
   tgc aac cag tgt ggg cca ggc atg gag ttg tct aag gaa tgt ggc ttc   192
   ggc tat ggg gag gat gca cag tgt gtg acg tgc cgg ctg cac agg ttc   240
   aag gag gac tgg ggc ttc cag aaa tgc aag ccc tgt ctg gac tgc gca   288
   gtg gtg aac cgc ttt cag aag gca aat tgt tca gcc acc agt gat gcc   336
   atc tgc ggg gac tgc ttg cca gga ttt tat agg aag acg aaa ctt gtc     384
   ggc ttt caa gac atg gag tgt gtg cct tgt gga gac cct cct cct cct     432
   tac gaa ccg cac tgt gcc agc aag gtc aac ctc gtg aag atc gcg tcc     480
   acg gcc tcc agc cca cgg gac acg gcg ctg gct gcc gtt atc tgc agc     528
   gct ctg gcc acc gtc ctg ctg gcc ctg ctc atc ctc tgt gtc atc tat     576
   tgt aag aga cag ttt atg gag aag aaa ccc agc tgg tct ctg cgg tca     624
   cag gac att cag tac aac ggc tct gag ctg tcg tgt ttt gac aga cct     672
   cag ctc cac gaa tat gcc cac aga gcc tgc tgc cag tgc cgc cgt gac     720
   tca gtg cag acc tgc ggg ccg gtg cgc ttg ctc cca tcc atg tgc tgt     768
   gag gag gcc tgc agc ccc aac ccg gcg act ctt ggt tgt ggg gtg cat     816
   tct gca gcc agt ctt cag gca aga aac gca ggc cca gcc ggg gag atg     864
   gtg ccg act ttc ttc gga tcc ctc acg cag tcc atc tgt ggc gag ttt     912
   tca gat gcc tgg cct ctg atg cag aat ccc atg ggt ggt gac aac atc     960
   tct ttt tgt gac tct tat cct gaa ctc act gga gaa gac att cat tct    1008
   ctc aat cca gaa ctt gaa agc tca acg tct ttg gat tca aat agc agt    1056
   caa gat ttg gtt ggt ggg gct gtt cca gtc cag tct cat tct gaa aac     1104
   ttt aca gca gct act gat tta tct aga tat aac aac aca ctg gta gaa     1152
   tca gca tca act cag gat gca cta act atg aga agc cag cta gat cag     1200
   gag agt ggc gct atc atc cac cca gcc act cag acg tcc ctc cag gaa     1248
   gct taaagaacct gcttctttct gcagtagaag cgtgtgctgg aacccaaaga         1301
   gtactccttt gttaggctta tggactgagc agtctggacc ttgcatggct tctggggcaa 1361
   aaatgaatct gaaccaaact gacggcattt gaagcctttc agccagttgc ttctgagcca 1421
   gaccagctgt aagctgaaac ctcaatgaat aacaagaaaa gactccaggc cgactcatga 1481
   tactctgcat ttttcctaca tgagaagctt ctctgccaca aaagtgactt caaagacgga 1541
   tgggttgagc tggcagccta tgagattgtg gacatataac aagaaacaga aatgccctca 1601
   tgcttatttt catggtgatt gtggttttac aagactgaag acccagagta tactttttc  1660
<210> 2
   <211> 417
   <212> PRT
   <213> Homo sapiens
<400> 2
<210> 3
   <211> 1325
   <212> DNA
   <213> Homo sapiens
<220>
   <221> CDS
   <222> (1)..(1269)
<400> 3
   atg gct tta aaa gtg cta cta gaa caa gag aaa acg ttt ttc act ctt      48
   tta gta tta cta ggc tat ttg tca tgt aaa gtg act tgt gaa aca gga      96
   gac tgt aga cag caa gaa ttc agg gat cgg tct gga aac tgt gtt ccc     144
   tgc aac cag tgt ggg cca ggc atg gag ttg tct aag gaa tgt ggc ttc     192
   ggc tat ggg gag gat gca cag tgt gtg acg tgc cgg ctg cac agg ttc     240
   aag gag gac tgg ggc ttc cag aaa tgc aag ccc tgt ctg gac tgc gca     288
   gtg gtg aac cgc ttt cag aag gca aat tgt tca gcc acc agt gat gcc     336
   atc tgc ggg gac tgc ttg cca gga ttt tat agg aag acg aaa ctt gtc     384
   ggc ttt caa gac atg gag tgt gtg cct tgt gga gac cct cct cct cct     432
   tac gaa ccg cac tgt gcc agc aag gtc aac ctc gtg aag atc gcg tcc     480
   acg gcc tcc agc cca cgg gac acg gcg ctg gct gcc gtt atc tgc agc     528
   gct ctg gcc acc gtc ctg ctg gcc ctg ctc atc ctc tgt gtc atc tat    576
   tgt aag aga cag ttt atg gag aag aaa ccc agc tgg tct ctg cgg tca    624
   cag gac att cag tac aac ggc tct gag ctg tcg tgt ctt gac aga cct    672
   cag ctc cac gaa tat gcc cac aga gcc tgc tgc cag tgc cgc cgt gac    720
   tca gtg cag acc tgc ggg ccg gtg cgc ttg ctc cca tcc atg tgc tgt    768
   gag gag gcc tgc agc ccc aac ccg gcg act ctt ggt tgt ggg gtg cat    816
   tct gca gcc agt ctt cag gca aga aac gca ggc cca gcc ggg gag atg    864
   gtg ccg act ttc ttc gga tcc ctc acg cag tcc atc tgt ggc gag ttt    912
   tca gat gcc tgg cct ctg atg cag aat ccc atg ggt ggt gac aac atc    960
   tct ttt tgt gac tct tat cct gaa ctc gct gga gaa gac att cat tct    1008
   ctc aat cca gaa ctt gaa agc tca acg tct ttg gat tca aat agc agt    1056
   caa gat ttg gtt ggt ggg gct gtt cca gtc cag tct cat tct gaa aac    1104
   ttt aca gca gct act gat tta tct aga tat aac aac aca ctg gta gaa    1152
   tca gca tca act cag gat gca cta act atg aga agc cag cta gat cag    1200
   gag agt ggc gct atc atc cac cca gcc act cag acg tcc ctc cag gta    1248
   agg cag cga ctg ggt tcc ctg tgaacacagc actgacttac agtagatcag      1299
   aactctgttc ccagcataag atttgg                                      1325
<210> 4
   <211> 423
   <212> PRT
   <213> Homo sapiens
<400> 4
<210> 5
   <211> 1914
   <212> DNA
   <213> Mus musculus
<220>
   <221> CDS
   <222> (1)..(1248)
<400> 5
   atg gca ctc aag gtc cta cct cta cac agg acg gtg ctc ttc gct gcc    48
   Met Ala Leu Lys Val Leu Pro Leu His Arg Thr Val Leu Phe Ala Ala
     1               5                  10                  15
   att ctc ttc cta ctc cac ctg gca tgt aaa gtg agt tgc gaa acc gga    96
   Ile Leu Phe Leu Leu His Leu Ala Cys Lys Val Ser Cys Glu Thr Gly
                    20                  25                  30
   gat tgc agg cag cag gaa ttc aag gat cga tct gga aac tgt gtc ctc   144
   Asp Cys Arg Gln Gln Glu Phe Lys Asp Arg Ser Gly Asn Cys Val Leu
                35                  40                  45
   tgc aaa cag tgc gga cct ggc atg gag ttg tcc aag gaa tgt ggc ttc    192
   Cys Lys Gln Cys Gly Pro Gly Met Glu Leu Ser Lys Glu Cys Gly Phe
        50                  55                  60
   ggc tat ggg gag gat gca cag tgt gtg ccc tgc agg ccg cac cgg ttc    240
   Gly Tyr Gly Glu Asp Ala Gln Cys Val Pro Cys Arg Pro His Arg Phe
    65                  70                  75                  80
   aag gaa gac tgg ggt ttc cag aag tgt aag cca tgt gcg gac tgt gcg    288
   Lys Glu Asp Trp Gly Phe Gln Lys Cys Lys Pro Cys Ala Asp Cys Ala
                    85                  90                  95
   ctg gtg aac cgc ttt cag agg gcc aac tgc tca cac acc agt gat gct    336
   Leu Val Asn Arg Phe Gln Arg Ala Asn Cys Ser His Thr Ser Asp Ala
               100                 105                 110
   gtc tgc ggg gac tgc ctg cca gga ttt tac cgg aag acc aaa ctg gtt    384
   Val Cys Gly Asp Cys Leu Pro Gly Phe Tyr Arg Lys Thr Lys Leu Val
           115                 120                 125
   ggt ttt caa gac atg gag tgt gtg ccc tgc gga gac cca cct cct ccc    432
   Gly Phe Gln Asp Met Glu Cys Val Pro Cys Gly Asp Pro Pro Pro Pro
       130                 135                 140
   tac gaa cca cac tgt acc agc aag gtg aac ctt gtg aag atc tcc tcc    480
   Tyr Glu Pro His Cys Thr Ser Lys Val Asn Leu Val Lys Ile Ser Ser
   145                 150                 155                 160
   acc gtc tcc agc cct cgg gac acg gcg ctg gct gcc gtc atc tgc agt    528
   Thr Val Ser Ser Pro Arg Asp Thr Ala Leu Ala Ala Val Ile Cys Ser
                   165                 170                 175
   gct ctg gcc acg gtg ctg ctc gcc ctg ctc atc ctg tgt gtc atc tac    576
   Ala Leu Ala Thr Val Leu Leu Ala Leu Leu Ile Leu Cys Val Ile Tyr
               180                 185                 190
   tgc aag agg cag ttc atg gag aag aaa ccc agc tgg tct ctg cgg tca    624
   Cys Lys Arg Gln Phe Met Glu Lys Lys Pro Ser Trp Ser Leu Arg Ser
           195                 200                 205
   cag gac att cag tac aat ggc tct gag ctg tca tgc ttt gac cag cct    672
   Gln Asp Ile Gln Tyr Asn Gly Ser Glu Leu Ser Cys Phe Asp Gln Pro
       210                 215                 220
   cgg ctc cgc cac tgt gcc cat aga gca tgc tgt cag tat cac cgg gac    720
   Arg Leu Arg His Cys Ala His Arg Ala Cys Cys Gln Tyr His Arg Asp
   225                 230                 235                 240
   tca gcc cca atg tat ggg cct gtt cac ctg att ccg tcc ttg tgc tgt    768
   Ser Ala Pro Met Tyr Gly Pro Val His Leu Ile Pro Ser Leu Cys Cys
                   245                 250                 255
   gaa gag gcc cgc agc tct gcc cga gct gtg ctt ggc tgt ggg ctg cgt    816
   Glu Glu Ala Arg Ser Ser Ala Arg Ala Val Leu Gly Cys Gly Leu Arg
               260                 265                 270
   tct ccc act acc ctc cag gag aga aac ccg gct tct gtg ggg gac acg    864
   Ser Pro Thr Thr Leu Gln Glu Arg Asn Pro Ala Ser Val Gly Asp Thr
           275                 280                 285
   atg cca gcc ttc ttc ggg tct gtt tcc cgt tcc atc tgc gct gaa ttt    912
   Met Pro Ala Phe Phe Gly Ser Val Ser Arg Ser Ile Cys Ala Glu Phe
       290                 295                 300
   tct gat gcc tgg cct ctg atg cag aat cct ctg ggt ggt gac agc tct    960
   Ser Asp Ala Trp Pro Leu Met Gln Asn Pro Leu Gly Gly Asp Ser Ser
   305                 310                 315                 320
   ctc tgt gac tct tat cct gaa ctc act gga gaa gat acc aat tcc ctc    1008
   Leu Cys Asp Ser Tyr Pro Glu Leu Thr Gly Glu Asp Thr Asn Ser Leu
                   325                 330                 335
   aat ccc gaa aac gaa agc gca gca tct ctg gat tcc agt ggc ggc cag    1056
   Asn Pro Glu Asn Glu Ser Ala Ala Ser Leu Asp Ser Ser Gly Gly Gln
               340                 345                 350
   gat ctg gct ggg aca gct gct cta gag tct tct ggg aat gtt tca gaa    1104
   Asp Leu Ala Gly Thr Ala Ala Leu Glu Ser Ser Gly Asn Val Ser Glu
           355                 360                 365
   tct act gac tca cct aga cat ggt gac act ggt aca gtc tgg gag cag    1152
   Ser Thr Asp Ser Pro Arg His Gly Asp Thr Gly Thr Val Trp Glu Gln
       370                 375                 380
   acg cta gct cag gat gct caa agg act cca agc caa gga ggc tgg gaa    1200
   Thr Leu Ala Gln Asp Ala Gln Arg Thr Pro Ser Gln Gly Gly Trp Glu
   385                 390                 395                 400
   gac agg gaa aac ctg aat cta gcc atg ccc aca gcc ttc cag gat gcc    1248
   Asp Arg Glu Asn Leu Asn Leu Ala Met Pro Thr Ala Phe Gln Asp Ala
                   405                 410                 415
   tgaaggccat cttcctgacg tggaggtgtg ggtctggaca agcctgtgat gaggcctaca 1308
   gactgagcag tcttggtgtc tggaagcaaa aataaatctg aaccaaactg acaacatttc 1368
   catcctttca gccactagct tctgagccag accagctgta agctgaaacc ccagcaagaa 1428
   gcaaggagag actgactgta ggcggccttg ggacatgtgc ttcttcccta agcgagaacc 1488
   ttagctgggg ccaatttgaa ggacccatgg gtggaatgtg ctgcctgtga gcttgtgggc 1548
   acagcaggac ccagcctggc tccttcttat gtccacggtg aatgtggttt cacaagaccc 1608
   agagtataaa ctttcataga cattctcttt tagaaataat ccattaccct gtcttcaaaa 1668
   accaaaaaaa aaaaaaagtg gtgttaaggt tttgaacatc acctagccaa gttagtaaaa 1728
   tctttatttg tatttcatct caattttttt aactattcat tttccttgta tgaattcttg 1788
   tgtgttttat gtgtaaatat attcattatt ttgacactat caatattctt tgtggttttg 1848
   taatttttac ttttattaat gactcaagct gtaaaaataa actaatttca acgtcgacgc 1908
   ggccgc                                                            1914
<210> 6
   <211> 416
   <212> PRT
   <213> Mus musculus
<400> 6
   Met Ala Leu Lys Val Leu Pro Leu His Arg Thr Val Leu Phe Ala Ala
    1               5                  10                  15
   Ile Leu Phe Leu Leu His Leu Ala Cys Lys Val Ser Cys Glu Thr Gly
               20                  25                  30
   Asp Cys Arg Gln Gln Glu Phe Lys Asp Arg Ser Gly Asn Cys Val Leu
           35                  40                  45
   Cys Lys Gln Cys Gly Pro Gly Met Glu Leu Ser Lys Glu Cys Gly Phe
       50                  55                  60
   Gly Tyr Gly Glu Asp Ala Gln Cys Val Pro Cys Arg Pro His Arg Phe
    65                 70                  75                  80
   Lys Glu Asp Trp Gly Phe Gln Lys Cys Lys Pro Cys Ala Asp Cys Ala
                   85                  90                  95
   Leu Val Asn Arg Phe Gln Arg Ala Asn Cys Ser His Thr Ser Asp Ala
               100                 105                 110
   Val Cys Gly Asp Cys Leu Pro Gly Phe Tyr Arg Lys Thr Lys Leu Val
           115                 120                 125
   Gly Phe Gln Asp Met Glu Cys Val Pro Cys Gly Asp Pro Pro Pro Pro
       130                 135                 140
   Tyr Glu Pro His Cys Thr Ser Lys Val Asn Leu Val Lys Ile Ser Ser
   145                 150                 155                 160
   Thr Val Ser Ser Pro Arg Asp Thr Ala Leu Ala Ala Val Ile Cys Ser
                   165                 170                 175
   Ala Leu Ala Thr Val Leu Leu Ala Leu Leu Ile Leu Cys Val Ile Tyr
               180                 185                 190
   Cys Lys Arg Gln Phe Met Glu Lys Lys Pro Ser Trp Ser Leu Arg Ser
           195                 200                 205
   Gln Asp Ile Gln Tyr Asn Gly Ser Glu Leu Ser Cys Phe Asp Gln Pro
       210                 215                 220
   Arg Leu Arg His Cys Ala His Arg Ala Cys Cys Gln Tyr His Arg Asp
   225                 230                 235                 240
   Ser Ala Pro Met Tyr Gly Pro Val His Leu Ile Pro Ser Leu Cys Cys
                   245                 250                 255
   Glu Glu Ala Arg Ser Ser Ala Arg Ala Val Leu Gly Cys Gly Leu Arg
               260                 265                 270
   Ser Pro Thr Thr Leu Gln Glu Arg Asn Pro Ala Ser Val Gly Asp Thr
           275                 280                 285
   Met Pro Ala Phe Phe Gly Ser Val Ser Arg Ser Ile Cys Ala Glu Phe
   290 295 300
   Ser Asp Ala Trp Pro Leu Met Gln Asn Pro Leu Gly Gly Asp Ser Ser
   305 310 315 320
   Leu Cys Asp Ser Tyr Pro Glu Leu Thr Gly Glu Asp Thr Asn Ser Leu
   325 330 335
   Asn Pro Glu Asn Glu Ser Ala Ala Ser Leu Asp Ser Ser Gly Gly Gln
   340 345 350
   Asp Leu Ala Gly Thr Ala Ala Leu Glu Ser Ser Gly Asn Val Ser Glu
   355 360 365
   Ser Thr Asp Ser Pro Arg His Gly Asp Thr Gly Thr Val Trp Glu Gln
   370 375 380
   Thr Leu Ala Gln Asp Ala Gln Arg Thr Pro Ser Gln Gly Gly Trp Glu
   385 390 395 400
   Asp Arg Glu Asn Leu Asn Leu Ala Met Pro Thr Ala Phe Gln Asp Ala
   405 410 415
<210> 7
   <211> 27
   <212> DNA
   <213> Mus musculus
<400> 7 27
   aggccatctt cctgacgtgg aggtgtg 27
<210> 8
   <211> 35
   <212> DNA
   <213> Mus musculus
<400> 8
   cggaattcgt ttcagctcag cacattccaa ggccg 35
<210> 9
   <211> 9
   <212> PRT
   <213> Homo sapiens
<400> 9
   Ser Thr Ala Ser Ser Pro Arg Asp Thr
   1 5
<210> 10
   <211> 7
   <212> PRT
   <213> Homo sapiens
<400> 10
   Asp Lys Thr His Thr Cys Pro
    1              5